(19) Europäisches Patentamt
European Patent Office
Office européen des brevets

(11) Veröffentlichungsnummer: **0 561 845 B1**

# (12) EUROPÄISCHE PATENTSCHRIFT

(45) Veröffentlichungstag der Patentschrift: **08.03.95**

(51) Int. Cl.[6]: **C07D 491/052**, A01N 25/32, //(C07D491/052,311:00,221:00)

(21) Anmeldenummer: **91920844.7**

(22) Anmeldetag: **29.11.91**

(86) Internationale Anmeldenummer:
**PCT/EP91/02267**

(87) Internationale Veröffentlichungsnummer:
**WO 92/10500 (25.06.92 92/14)**

## (54) PYRIDO-ANELLIERTE 4-OXO-4H-BENZOPYRANE, VERFAHREN ZU IHRER HERSTELLUNG UND IHRE VERWENDUNG ALS ANTIDOTS.

(30) Priorität: **08.12.90 DE 4039272**

(43) Veröffentlichungstag der Anmeldung:
**29.09.93 Patentblatt 93/39**

(45) Bekanntmachung des Hinweises auf die Patenterteilung:
**08.03.95 Patentblatt 95/10**

(84) Benannte Vertragsstaaten:
**AT BE CH DE FR GB IT LI**

(56) Entgegenhaltungen:
**EP-A- 0 387 582**
**DE-A- 2 809 720**

(73) Patentinhaber: **BASF Aktiengesellschaft**
**Carl-Bosch-Strasse 38**
**D-67063 Ludwigshafen (DE)**

(72) Erfinder: **HAGEN, Helmut**
**Max-Slevogt-Stra e 17 e**
**D-6710 Frankenthal (DE)**
Erfinder: **NILZ, Gerhard**
**Haardtstrasse 13**
**D-6701 Dannstadt-Schauernheim (DE)**
Erfinder: **WALTER, Helmut**
**Gruenstadter Strasse 82**
**D-6719 Obrigheim (DE)**
Erfinder: **LANDES, Andreas**
**Untere Hart 12**
**D-6703 Limburgerhof (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 561 845 B1

JOURNAL OF THE CHEMICAL SOCIETY, PERKIN TRANSACTIONS 1. Nr. 8, 1979,LETCHWORTH GB Seiten 1964 - 1968; C. GHOSH ET AL.: 'Heterocyclic systems. part 6.Reactions of 4-oxo-4H-(1)benzopyran-3-carbonitriles with hydrazine, phenylhydrazine, hydroxylamine and some reactive methylene compounds.'siehe Seite 1966, Schema 2

CHEMICAL ABSTRACTS, vol. 98, no. 3, 17. Januar 1983, Columbus, Ohio, US; abstract no. 16602G, V. M. PETRICHENKO ET AL.: 'Synthesis and acid-base transformations of 2-substituted 3-methylpyrido(2,3-d)cromones' Seite 503; & Khim. Geterotsikl. Soedin. 1982, (9), 1229-31 siehe Zusammenfassung

CHEMICAL ABSTRACTS, vol. 75, no. 9, 30. August 1971, Columbus, Ohio, US; abstract no. 63555D, S. M. GLOZMAN ET AL.: 'Synthesis and reactions of 2-aminochromone derivatives' Seite 433; & Khim. Farm. Zh. 1971, 5(5), 17-21 siehe Zusammenfassung

JOURNAL OF MEDICINAL CHEMISTRY. Bd. 28, Nr. 5, 1985, WASHINGTON US Seiten 559-568; A. NOHARA ET AL.: 'Studies on antianaphylactic agents. Synthesis of antiallergic 5-oxo-5H-(1)benzopyrano(2,3-b)pyridines.' in der Anmeldung erwähnt siehe Seite 563 - Seite 564; Tabellen III, IV

ARCHIV DER PHARMAZIE. Bd. 318, Nr. 11, November 1985, WEINHEIM DE Seiten 1048 -1050; G. SEITZ ET AL.: 'Inverse intramolekulare(4+2)-Cycloaddition mit substituierten 1,2,4-Triazinen' siehe Seite 1049, Verbindung 5

ARCHIV DER PHARMAZIE. Bd. 320, Nr. 6, Juni 1987, WEINHEIM DE Seiten 500 - 506; U. ASHAUER-HOLZGRABE: '5-Oxo-1H-3,4,5,6-tetrahyd ro-2,6-methano-2-benzazocin und seine Reduktionsprodukte' siehe Seite 500, Verbindung 4,6

JOURNAL OF HETEROCYCLIC CHEMISTRY. Bd. 26, Nr. 6, November 1989, PROVO US Seiten 1589 - 1594; F. MARSAIS ET AL.: 'Directed ortho-lithiation of chloroquinolines. Application to synthesis of 2,3-disubstituted quinolines' siehe Seite 1590, Verbindung 31

## Beschreibung

Die vorliegende Erfindung betrifft pyrido-anellierte 4-Oxo-4H-benzopyrane der allgemeinen Formel I

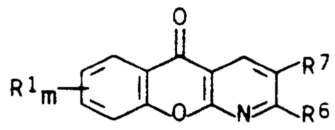

I

in der die Substituenten und der Index folgende Bedeutung haben:

- m — 0, 1 oder 2, wobei die Reste $R^1$ verschieden sein können, wenn m 2 bedeutet;
- $R^1$ — Wasserstoff; Hydroxy; Halogen; Nitro; $C_1$-$C_6$-Alkyl ; $C_1$-$C_6$- Alkoxy; $C_1$-$C_6$-Halogenalkyl; $C_1$-$C_4$-Halogenalkoxy; $C_1$-$C_4$-Alkylthio oder eine Gruppe $NR^2R^3$, wobei
- $R^2$ — Wasserstoff oder $C_1$-$C_4$-Alkyl und
- $R^3$ — Wasserstoff, $C_1$-$C_4$-Alkyl, eine Gruppe -CO-$R^4$, -CS-$R^4$ oder -$SO_2$-$R^5$ bedeuten;
- $R^4$ — $C_1$-$C_{20}$-Alkyl; $C_1$-$C_6$-Halogenalkyl; $C_3$-$C_8$-Cycloalkyl; $C_1$-$C_3$-Aralkyl; Amino; $C_1$-$C_4$-Alkylamino; Di-$C_1$-$C_4$-alkylamino; eine Phenyl- oder Phenylaminogruppe, wobei der aromatische Ring eine bis drei der für $R^1$ genannten Gruppen tragen kann;
- $R^5$ — $C_1$-$C_4$-Alkyl; Phenyl, das eine bis drei der bei $R^1$ genannten Gruppen tragen kann;
- $R^6$ — Hydroxy; $C_1$-$C_6$-Alkoxy; $C_1$-$C_6$-Alkyl; eine Gruppe -NH-$R^8$ oder -N($C_1$-$C_4$-Alkyl)-$R^8$, wobei $R^8$ für eine der Bedeutungen von $R^3$ steht; ein Phenylrest, der eine bis drei der für $R^1$ genannten Gruppen tragen kann, eine $C_1$-$C_4$-alkylsubstituierte 4-Methyl-5-oxo-imidazolin-2-yl-Gruppe oder eine Gruppe -CO-$R^9$ oder -CS-$R^9$;
- $R^7$ — Cyano, $C_1$-$C_6$-Alkyl; ein Phenylrest, der eine bis drei der für $R^1$ genannten Gruppen tragen kann; eine Gruppe -CH = N-$R^{10}$, -C($C_1$-$C_4$-Alkyl) = N-$R^{10}$, -CO-$R^{11}$ oder -CS-$R^{11}$;
- $R^9$, $R^{11}$ — $C_1$-$C_6$-Alkyl; $C_1$-$C_6$-Alkoxy; Hydroxy; Amino; $C_1$-$C_4$-Alkylamino; durch eine Amidgruppe $CONH_2$ substituiertes, verzweigtes $C_2$-$C_6$-Alkyl; Di-$C_1$-$C_4$-alkylamino; ein Aminophenylrest, wobei der aromatische Ring ein bis drei der für $R^1$ genannten Gruppen tragen kann;
- $R^{10}$ — $C_1$-$C_4$-Alkyl; $C_3$-$C_8$-Cycloalkyl; Phenyl, welches eine bis drei der für $R^1$ genannten Gruppen tragen kann; Hydroxy, $C_1$-$C_8$-Alkoxy; eine Gruppe -O-CO-$R^{12}$ oder -O-CS-$R^{12}$, wobei $R^{12}$ für $C_1$-$C_{20}$-Alkyl; $C_1$-$C_6$-Halogenalkyl; $C_3$-$C_8$-Cycloalkyl; Phenyl-$C_1$-$C_3$-alkyl oder Phenyl, wobei die Phenylringe gewünschtenfalls noch einen bis drei der bei $R^1$ genannten Reste tragen können, steht;

oder für den Fall, daß $R^6$ und $R^7$ Alkyl oder $R^6$ Alkyl und $R^7$ eine Gruppe -CO-H, -CO-($C_1$-$C_4$-Alkyl), -CH = N-$R^{10}$ oder -C($C_1$-$C_4$-Alkyl) = N-$R^{10}$ bedeuten:

- $R^6$ und $R^7$ — gemeinsam einen 5 - 7-gliedrigen Ring, der mit einer bis vier $C_1$-$C_4$-Alkylgruppen substituiert oder mit einem Phenylring, der eine bis drei der für $R^1$ genannten Gruppen tragen kann, anelliert ist;

sowie die landwirtschaftlich brauchbaren Salze der Verbindungen I, sofern diese Verbindungen einen basischen Stickstoffsubstituenten oder einen sauren Hydroxysubstituenten enthalten,

ausgenommen 2,3-Diphenyl-[1]benzopyran[2,3-b]pyridin-5-on,
2,3-Dimethyl-[1]benzopyran[2,3-b]pyridin-5-on,
3-Ethoxycarbonyl-2-methyl-[1]benzopyran[2,3-b]pyridin-5-on,
3-Ethoxycarbonyl-7-ethyl-2-methoxy-[1]benzopyran[2,3-b]pyridin-5-on,
3-Ethoxycarbonyl-7-ethyl-2-methyl-[1]benzopyran[2,3-b]pyridin-5-on,
2-Hydroxy-3-methyl-[1]benzopyran[2,3-b]pyridin-5-on,
2,3-Diethoxycarbonyl-7-ethyl-[1]benzopyran[2,3-b]pyridin-5-on,

sowie diejenigen Verbindungen I, bei denen entweder $R^6$ für $C_1$-$C_6$-Alkyl, Phenyl, Carboxyl, Hydroxyl, $C_1$-$C_6$-Alkoxy, Amino oder $C_1$-$C_4$-Alkylamino und $R^7$ für Cyano, Carboxyl oder Ethoxycarbonyl stehen, oder

$R^6$ für Hydroxy und $R^7$ für Ethoxycarbonyl stehen oder

$R^6$ für Methyl und $R^7$ für Methylcarbonyl stehen.

Außerdem betrifft die Erfindung Verfahren zur Herstellung der Verbindungen I sowie herbizide Mittel, die 2-(4-Heteroaryloxy) oder 2-(4-Aryloxy)-phenoxyessig- oder -propionsäurederivate und/oder Cyclohexenonderivate als herbizide Wirkstoffe und pyridin-anellierte 4-Oxo-4H-benzopyrane I' (wobei I' die Bedeutung von I ohne die Maßgaben hat) als Antidots enthalten, sowie Verfahren zur selektiven Bekämpfung von

unerwünschtem Pflanzenwuchs mit diesen herbiziden Mitteln.

Die erfindungsgemäßen Verbindungen I sind auf verschiedenen Wegen zugänglich. 2-Amino-4-oxo-4H-benzopyran-3-carbaldehyde erhält man z. B. durch Formylierung von gegebenenfalls substituierten o-Hydroxyacetophenonen mit Phosphoroxytrichlorid und Dimethylformamid (H. Harnisch, Liebigs Ann. Chem. 765 (1972) 8; A. Nohara et al, Tetrahedron Lett. 1973, 1995), Umsetzung des Aldehyds zum Oxim, dessen alkalikatalysierte Umlagerung sowie anschließende Kondensation mit Malonsäurederivaten (U. Petersen et al., Liebigs Ann. Chem. 1976, 1659). Eine pflanzenschützende Wirkung von pyrido-anellierten 4-Oxo-4H-benzopyranen ist dem Stand der Technik nicht zu entnehmen.

Aus Journal of Medical Chemistry, Bd. 28, Nr. 5, 1985, Seiten 559 - 568, ist die Verbindung 2,3-Diethoxycarbonyl-7-ethyl-[1]benzopyran[2,3b]pyridin-5-on als Zwischenprodukt für antiallergische Mittel bekannt. DE-A 2 809 720 beschreibt [1]-Benzopyran[2,3b]pyridin-5-one mit einer Amino- oder Methylgruppe in 4-Position ($R^6$) und Ethoxycarbonyl- bzw. Cyanosubstitution in 3-Position ($R^7$) als Ausgangsstoffe für pharmakologisch interessante 4-Carbonsäurederivate. J. Chem. Soc. Nr. 8, 1979, Seiten 1964 - 1968, offenbart entsprechende Benzopyronpyridinderivate mit $R^6$ = Methyl, Hydroxy und Amino und $R^7$ = Ethylcarbonyl. Chem. Abstracts, Vol. 98, Nr. 16602g, beschreibt die Herstellung von 2,3-Dimethyl[1]-benzopyran[2,3]-pyridin-5-on. Die Herstellung weiterer Benzopyranpyridin-5-on-derivate ist in Chem. Abstracts, Vol. 75, Nr. 63555d, beschrieben.

Der Erfindung lag nun die Aufgabe zugrunde, Verbindungen zu finden, welche die Nachteile, die bei der Verwendung der obengenannten Herbizide der Formeln V und VI bestehen, zumindest so stark zu verringern, daß die Herbizide für die Kulturpflanzen aus der Familie der Gräser verträglich werden.

Entsprechend der Aufgabe wurden die eingangs definierten pyridoanellierten 4-Oxo-4H-benzopyrane I gefunden. Ferner wurden Verfahren zur Herstellung dieser Verbindungen I sowie zur gemeinsamen Anwendung dieser Verbindungen und Verbindungen I' mit den Herbiziden V und VI zur Beeinflussung unerwünschten Pflanzenwachstums gefunden. Des weiteren betrifft die Erfindung herbizide Mittel, enthaltend mindestens ein pyridoanelliertes 4-Oxo-4H-benzopyran der allgemeinen Formel I'

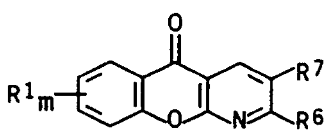

I',

- m 0, 1 oder 2, wobei die Reste $R^1$ verschieden sein können, wenn m 2 bedeutet;
- $R^1$ Wasserstoff; Hydroxy; Halogen; Nitro; $C_1$-$C_6$-Alkyl; $C_1$-$C_6$-Alkoxy; $C_1$-$C_6$-Halogenalkyl; $C_1$-$C_4$-Halogenalkoxy; $C_1$-$C_4$-Alkylthio oder eine Gruppe $NR^2R^3$, wobei
- $R^2$ Wasserstoff oder $C_1$-$C_4$-Alkyl und
- $R^3$ Wasserstoff, $C_1$-$C_4$-Alkyl, eine Gruppe -CO-$R^4$, -CS-$R^4$ oder -$SO_2$-$R^5$ bedeuten;
- $R^4$ $C_1$-$C_{20}$-Alkyl; $C_1$-$C_6$-Halogenalkyl; $C_3$-$C_8$-Cycloalkyl; $C_1$-$C_3$-Aralkyl; Amino; $C_1$-$C_4$-Alkylamino; Di-$C_1$-$C_4$-alkylamino; eine Phenyl- oder Phenylaminogruppe, wobei der aromatische Ring eine bis drei der für $R^1$ genannten Gruppen tragen kann;
- $R^5$ $C_1$-$C_4$-Alkyl; Phenyl, das eine bis drei der bei $R^1$ genannten Gruppen tragen kann;
- $R^6$ Hydroxy; $C_1$-$C_6$-Alkoxy; $C_1$-$C_6$-Alkyl; eine Gruppe -NH-$R^8$ oder -N($C_1$-$C_4$-Alkyl)-$R^8$, wobei $R^8$ für eine der Bedeutungen von $R^3$ steht; ein Phenylrest, der eine bis drei der für $R^1$ genannten Gruppen tragen kann, eine $C_1$-$C_4$-alkylsubstituierte 4-Methyl-5-oxo-imidazolin-2-yl-Gruppe oder eine Gruppe -CO-$R^9$ oder -CS-$R^9$;
- $R^7$ Cyano, $C_1$-$C_6$-Alkyl; ein Phenylrest, der eine bis drei der für $R^1$ genannten Gruppen tragen kann; eine Gruppe -CH=N-$R^{10}$, -C($C_1$-$C_4$-Alkyl)=N-$R^{10}$, -CO-$R^{11}$ oder -CS-$R^{11}$;
- $R^9$, $R^{11}$ $C_1$-$C_6$-Alkyl; $C_1$-$C_6$-Alkoxy; Hydroxy; Amino; $C_1$-$C_4$-Alkylamino; durch eine Amidgruppe $CONH_2$ substituiertes, verzweigtes $C_2$-$C_6$-Alkyl; Di-$C_1$-$C_4$-alkylamino; ein Aminophenylrest, wobei der aromatische Ring ein bis drei der für $R^1$ genannten Gruppen tragen kann;
- $R^{10}$ $C_1$-$C_4$-Alkyl; $C_3$-$C_8$-Cycloalkyl; Phenyl, welches eine bis drei der für $R^1$ genannten Gruppen tragen kann; Hydroxy, $C_1$-$C_8$-Alkoxy; eine Gruppe -O-CO-$R^{12}$ oder -O-CS-$R^{12}$, wobei $R^{12}$ für $C_1$-$C_{20}$-Alkyl; $C_1$-$C_6$-Halogenalkyl; $C_3$-$C_8$-Cycloalkyl; Phenyl-$C_1$-$C_3$-alkyl oder Phenyl, wobei die Phenylringe gewünschtenfalls noch einen bis drei der für $R^1$ genannten Reste tragen können, steht;

oder für den Fall, daß $R^6$ und $R^7$ Alkyl oder $R^6$ Alkyl und $R^7$ eine Gruppe -CO-H, -CO-($C_1$-$C_4$-Alkyl), -CH=N-$R^{10}$ oder -C($C_1$-$C_4$-Alkyl)=N-$R^{10}$ bedeuten:

$R^6$ und $R^7$ gemeinsam einen 5 - 7-gliedrigen Ring, der mit einer bis vier $C_1$-$C_4$-Alkylgruppen

substituiert oder mit einem Phenylring, der eine bis drei der für $R^1$ genannten Gruppen tragen kann, anelliert ist;

sowie die landwirtschaftlich brauchbaren Salze der Verbindungen I', sofern diese Verbindungen einen basischen Stickstoffsubstituenten oder einen sauren Hydroxysubstituenten enthalten,

und mindestens einen herbiziden Wirkstoff aus der Gruppe

A) der 2-(4-Heteroaryloxy)- oder 2-(4-Aryloxy)-phenoxyessigsäurederivate der Formel V

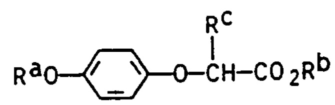

V

$R^a$ ein Phenylring, ein Pyridylring, ein Benzoxazylrest, ein Benzthiazylrest oder ein Benzpyrazinylrest, wobei diese aromatischen Ringsysteme bis zu zwei der folgenden Reste tragen können: Halogen, Nitro, $C_1$-$C_4$-Alkyl, $C_1$-$C_4$-Halogenalkyl und/oder $C_1$-$C_4$-Halogenalkoxy,

$R^b$ Wasserstoff oder eine $C_1$-$C_4$-Alkylgruppe und

$R^c$ Wasserstoff oder eine Methylgruppe und

B) der Cyclohexenonoximether der Formel VI

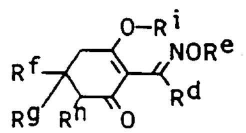

VI

in welcher die Substituenten folgenden Bedeutung haben:

$R^d$ eine $C_1$-$C_4$-Alkylgruppe;

$R^e$ eine $C_1$-$C_4$-Alkylgruppe, eine $C_3$-$C_4$-Alkenylgruppe, eine $C_3$-$C_4$-Alkinylgruppe oder eine partiell oder vollständig halogenierte $C_3$-$C_4$-Alkenylgruppe;

eine $C_1$-$C_4$-Alkylen- oder $C_2$-$C_4$-Alkenylkette, die beide noch ein bis 3 $C_1$-$C_3$-Alkylreste und/oder Halogenatome tragen können, oder eine gewünschtenfalls durch $C_1$-$C_3$-Alkyl substituierte 3- bis 6-gliedrige Alkylen- oder 4- bis 6-gliedrige Alkenylenkette, die jeweils ein dem Oximetherteil nicht direkt benachbartes Sauerstoff- oder Schwefelatom als Kettenglied enthält, wobei alle vorstehend genannten Ketten endständig den Phenylring tragen, der seinerseits durch ein bis drei Reste substituiert sein kann, ausgewählt aus einer Gruppe bestehend aus einem Benzyloxycarbonyl- oder Phenylrest und jeweils einem bis drei der folgenden Reste: Nitro, Cyano, Halogen, $C_1$-$C_4$-Alkyl, $C_1$-$C_4$-Alkoxy, $C_1$-$C_4$-Alkylthio, partiell oder vollständig halogeniertes $C_1$-$C_4$-Alkyl, partiell oder vollständig halogeniertes $C_1$-$C_4$-Alkoxy, Carboxyl und $C_1$-$C_4$-Alkoxycarbonyl, und wobei der Phenylring noch zusätzlich so viele Halogenatome tragen kann, daß die Gesamtzahl der Reste 4 oder 5 beträgt;

eine Thienylmethylgruppe, die noch ein Halogenatom tragen kann;

$R^f$ eine $C_1$-$C_4$-Alkylgruppe, welche einfach durch $C_1$-$C_4$-Alkylthio oder $C_1$-$C_4$-Alkoxy substituiert sein kann;

ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, wobei dieser Ring bis zu drei der folgenden Reste tragen kann: Hydroxy, Halogen, $C_1$-$C_4$-Alkyl, partiell oder vollständig halogeniertes $C_1$-$C_4$-Alkyl, $C_1$-$C_4$-Alkoxy und/oder $C_1$-$C_4$-Alkylthio;

einen 10-gliedrigen gesättigten oder einfach ungesättigten Heterocyclus, welcher zwei Sauerstoffatome oder Schwefelatome enthält und durch bis zu drei $C_1$-$C_4$-Alkylgruppen und/oder Methoxygruppen substituiert sein kann;

die Phenylgruppe, die Pyridylgruppe, die Thiazolylgruppe, die Pyrazolylgruppe, die Pyrrolylgruppe oder die Isoxazolylgruppe, wobei diese Gruppen jeweils ein bis drei Reste tragen können, ausgewählt aus einer Gruppe bestehend aus $C_1$-$C_4$-Alkyl, partiell oder vollständig halogeniertes $C_1$-$C_4$-Alkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-Alkyl, $C_1$-$C_4$-Alkylthio-$C_1$-$C_3$-Alkyl, $C_1$-$C_4$-Dialkoxy-$C_1$-$C_3$-alkyl, Formyl, Halogen und Benzoylamino;

$R^g$ Wasserstoff, Hydroxy oder, wenn $R^f$ die Bedeutung $C_1$-$C_6$-Alkylgruppe hat, eine $C_1$-$C_6$-

Alkylgruppe;

$R^h$ Wasserstoff, eine Cyanogruppe, ein Halogenatom, eine $C_1$-$C_4$-Alkoxycarbonylgruppe oder eine $C_1$-$C_4$-Alkylketoximgruppe und

$R^i$ Wasserstoff.

Dabei ist es unerheblich, ob der herbizide Wirkstoff und die antidotische Verbindung I' gemeinsam oder getrennt formuliert und ausgebracht werden bzw. bei getrennter Ausbringung, in welcher Reihenfolge herbizider Wirkstoff und Antidot appliziert werden.

Die erfindungsgemäßen Verbindungen I sind auf mehreren Wegen zugänglich. So erhält man pyrido-anellierte 4-Oxo-4H-benzopyrane beispielsweise gemäß der eingangs zitierten Literatur dadurch, daß man 2-Hydroxyacetophenone VII in einem inerten, aprotischen, polaren Lösungsmittel mit Dimethylformamid/Phosphoroxytrichlorid formyliert, den Aldehyd ins Oxim überführt und dieses unter alkalischer Katalyse in den o-Aminoaldehyd II umlagert und diesen in Gegenwart einer Base in einem inerten organischen Lösungsmittel mit dem Keton III anelliert.

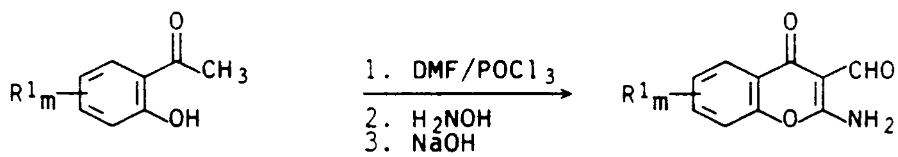


VII II

II +

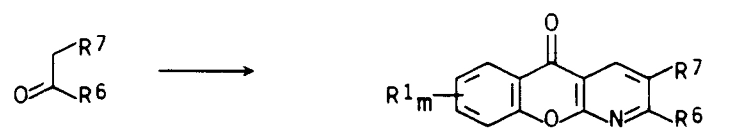

III I

Üblicherweise setzt man die Ausgangsstoffe VII und $POCl_3$ in stöchiometrischem Verhältnis ein. Ein Überschuß des einen oder anderen kann in Einzelfällen aber durchaus vorteilhaft sein.

Die Umsetzung kann kontinuierlich oder diskontinuierlich bei Normal-, Über- oder Unterdruck nach den dafür üblichen Techniken durchgeführt werden. Die Reaktionstemperatur liegt im allgemeinen bei (-10) bis 100 °C, insbesondere bei 0 bis 40 °C.

Als Lösungsmittel dienen beispielsweise aliphatische und aromatische chlorierte Kohlenwasserstoffe wie Dichlormethan, Chloroform und Chlorbenzol oder im Überschuß vorhandenes Dimethylformamid.

Als Lösungsmittel für die Anellierung mit III kommen beispielsweise aliphatische und aromatische, gegebenenfalls chlorierte Kohlenwasserstoffe wie Petrolether, Benzol, Toluol, Xylol, Benzin, Dichlormethan, Chloroform, Tetrachlormethan, 1,2-Dichlorethan, Chlobenzol, höhersiedende Ether wie Tetrahydrofuran oder Dioxan, aliphatische Alkohole wie Methanol, Ethanol, Propanol oder Isopropanol; Nitrile wie Acetonitril und Propionitril in Betracht.

Als Basen dienen insbesondere aliphatische, aromatische oder heterocyclische Amine, beispielsweise Triethylamin, Dimethylamin, Diisopropylamin, Piperidin, Dimethylanilin, Dimethylbenzylamin, Pyridin und 4-Dimethylaminopyridin, Hydroxide von Alkali- und Erdalkalimetallen wie z. B. Natriumhydroxid, Kaliumhydroxid, Calciumhydroxid; Alkoholate von Alkali-und Erdalkalimetallen, wie beispielsweise Natriummethylat, Natriumethylat, Calciummethylat, Kalium-tert.-butylat; Alkali- oder Erdalkalimetallhydride wie beispielsweise Natriumhydrid, Kaliumhydrid oder Calciumhydrid.

Unter Umständen kann es vorteilhaft sein, die Kondensation in Gegenwart eines üblichen Phasentransferkatalysators vorzunehmen.

Verbindungen I mit $R^6$ = Hydroxy oder Amino sind auch in der Weise zugänglich,
daß man den 2-Amino-4-oxo-4H-chromen-3-carbaldehyd II in an sich bekannter Weise in einem inerten Lösungsmittel in Gegenwart einer Base mit einem Essigsäurederivat der Formel IVa oder IVb anelliert, wobei R in der Formel IVa für eine $C_1$-$C_4$-Alkylgruppe steht.

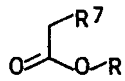

IVa

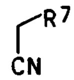

IVb

In Hinblick auf die bestimmungsgemäße Verwendung der Verbindungen I und I' als pflanzenschützende Mittel kommen als Substituenten folgende Reste in Betracht:

m — 0, 1 oder 2, wobei die Reste $R^1$ verschieden sein können, wenn m 2 bedeutet;

$R^1$ — Wasserstoff; Hydroxy; Nitro;
Halogen wie Fluor, Chlor, Brom und Jod, insbesondere Chlor und Brom; $C_1$-$C_6$-Alkyl wie Methyl, Ethyl, Propyl, 1-Methylethyl, Butyl, 1-Methylpropyl und 1,1-Dimethylethyl, besonders bevorzugt Methyl und Ethyl;
$C_1$-$C_6$-Alkoxy wie Methoxy, Ethoxy, Propoxy, 1-Methylethoxy, Butoxy, 1-Methylpropoxy, 2-Methylpropoxy, Hexoxy, insbesondere Methoxy und Ethoxy;
$C_1$-$C_6$-Halogenalkyl, insbesondere $C_1$- und $C_2$-Halogenalkyl, wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluormethyl, 2-Chlor-2,2-difluorethyl, 2,2-Dichlor-2-fluorethyl, 2,2,2-Trichlorethyl und Pentafluorethyl, besonders bevorzugt Trichlormethyl und Trifluormethyl;
$C_1$-$C_4$-Halogenalkoxy, insbesondere $C_1$- und $C_2$-Halogenalkoxy, wie Difluormethoxy, Trifluormethoxy, Chlor-difluormethoxy, Dichlorfluormethoxy, 1-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, besonders bevorzugt 2,2,2-Trifluorethoxy;
$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio;
eine Gruppe $NR^2R^3$, wobei

$R^2$ — Wasserstoff oder
$C_1$-$C_4$-Alkyl wie Methyl, Ethyl, n-Propyl, Isopropyl, n-Butyl und tert.-Butyl, insbesondere Methyl und Ethyl, und

$R^3$ — Wasserstoff oder
$C_1$-$C_4$-Alkyl wie Methyl, Ethyl, n-Propyl, Isopropyl, n-Butyl und tert.-Butyl, insbesondere Methyl und Ethyl;
eine Gruppe -CO-$R^4$ oder -CS-$R^4$, insbesondere Acetyl- und Propionyl;
eine Gruppe -$SO_2$-$R^5$, insbesondere 4-Methylsulfonyl ; bedeuten,

$R^4$ — $C_1$-$C_{20}$-Alkyl wie Methyl, Ethyl, n-Propyl, Isopropyl, n-Butyl, tert.-Butyl, n-Pentyl, n-Hexyl, Octyl, Dodecyl, Hexadecyl, Octadecyl, bevorzugt $C_1$-$C_6$-Alkyl wie bei $R^1$ genannt, insbesondere Methyl und Ethyl;
$C_1$-$C_6$-Halogenalkyl wie bei $R^1$ genannt;
$C_3$-$C_8$-Cycloalkyl wie Cyclopropyl, Cyclobutyl, Cyclopentyl, Cyclohexyl, Cycloheptyl und Cyclooctyl, insbesondere Cyclohexyl;
Phenyl-$C_1$-$C_3$-alkyl wie Benzyl, 2-Phenylethyl, 3-Phenylpropyl, insbesondere Benzyl;
Amino, welches ein oder zwei der bei $R^1$ genannten Alkylreste, insbesondere Methyl, tragen kann, wobei diese Reste bei zweifach substituiertem Amino verschieden sein können, insbesondere Methylbenzylamino;
Aminophenyl, wobei der Phenylring noch eine bis drei der bei $R^1$ genannten Gruppen tragen kann;
Phenyl, das eine bis drei der bei $R^1$ genannten Gruppen tragen kann;

$R^5$ — $C_1$-$C_4$-Alkyl wie Methyl, Ethyl, n-Propyl, Isopropyl, n-Butyl und tert.-Butyl, insbesondere Methyl und Ethyl;
Phenyl, das eine bis drei der bei $R^1$ genannten Gruppen tragen kann;

$R^6$ — Hydroxy;
$C_1$-$C_6$-Alkoxy wie bei $R^1$ genannt, insbesondere Methoxy und Ethoxy; $C_1$-$C_6$-Alkyl wie bei $R^1$ genannt, insbesondere Methyl und Ethyl ; Phenyl, das eine bis drei der bei $R^1$ genannten Gruppen tragen kann, eine Gruppe -CO-$R^9$ oder -CS-$R^9$, insbesondere CO-$R^9$, z.B. -COOH, -CO-O($C_1$-$C_4$-Alkyl),

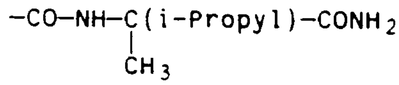

oder $C_1$-$C_4$-alkylsubstituiertes 4-Methyl-5-oxo-imidazolin-2-yl, z.B. die Gruppe

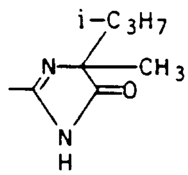

$R^7$ Cyano oder $C_1$-$C_6$-Alkyl wie bei $R^1$ genannt, insbesondere Methyl; Phenyl, das eine bis drei der bei $R^1$ genannten Gruppen tragen kann; eine Gruppe -CH = N-$R^{10}$, -C($C_1$-$C_4$-Alkyl) = N-$R^{10}$, -CO-$R^{11}$ oder -CS-$R^{11}$;

$R^{10}$ $C_1$-$C_4$-Alkyl wie Methyl, Ethyl, n-Propyl, Isopropyl, n-Butyl und tert.-Butyl;
$C_3$-$C_8$-Cycloalkyl wie Cyclopropyl, Cyclopentyl, Cyclohexyl, Cycloheptyl und Cyclooctyl, insbesondere Cyclohexyl;
Phenyl, das eine bis drei der bei $R^1$ genannten Gruppen tragen kann;
Hydroxy;
$C_1$-$C_8$-Alkoxy wie bei $R^1$ genannt;
eine Gruppe -O-CO-$R^{12}$ oder -O-CS-$R^{12}$, wobei $R^{12}$
$C_1$-$C_{20}$-Alkyl wie bei $R^4$ genannt;
$C_1$-$C_6$-Halogenalkyl wie bei $R^1$ genannt, insbesondere Chlorethyl;
$C_3$-$C_8$-Cycloalkyl wie Cyclopropyl, Cyclopentyl, Cyclohexyl, Cycloheptyl und Cyclooctyl, insbesondere Cyclohexyl;
Phenyl-$C_1$-$C_3$-alkyl wie Benzyl, 2-Phenylethyl, 3-Phenylpropyl und 2-Phenylpropyl, insbesondere Benzyl;
Phenyl, das einen bis drei der bei $R^1$ genannten Reste tragen kann;

$R^9$, $R^{11}$ $C_1$-$C_6$-Alkyl wie bei $R^1$ genannt; insbesondere Methyl;
$C_1$-$C_6$-Alkoxy wie bei $R^1$ genannt; insbesondere Methoxy und Ethoxy;
Hydroxy;
unsubstituiertes oder alkylsubstituiertes Amino wie bei $R^4$ genannt;
durch eine Amidgruppe $CONH_2$ substituiertes verzweigtes $C_2$-$C_6$-Alkyl, z.B.

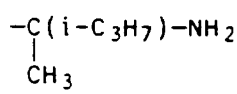 ;

Aminophenyl, wobei der Phenylring noch ein bis drei der bei $R^1$ genannten Gruppen tragen kann;

oder für den Fall, daß $R^6$ und $R^7$ Alkyl oder $R^6$ Alkyl und $R^7$ eine Gruppe -CO-H, -CO-($C_1$-$C_4$-Alkyl), -CH = N-$R^{10}$ oder -C($C_1$-$C_4$-Alkyl) = N-$R^{10}$ bedeuten:

$R^6$ und $R^7$ gemeinsam einen 5 - 7-gliedrigen Ring, der mit einer bis vier $C_1$-$C_4$-Alkylgruppen substituiert oder mit einem Phenylring, der eine bis drei der bei $R^1$ genannten Gruppen tragen kann, anelliert ist.

Derivate I und I' mit sauren Endgruppen oder mit basischen Stickstoffatomen können in Form ihrer landwirtschaftlich brauchbaren Salze vorliegen.

Als Säureadditionssalze eignen sich die Salze von solchen Säuren, welche die antidotische Wirkung von I nicht beeinträchtigen, also z.B. die Hydrochloride und -bromide, Sulfate, Nitrate, Phosphate, Oxalate oder die Dodecylbenzolsulfonate.

Als basische Salze eignen sich die Salze von solchen Basen, welche die antidotische Wirkung von I nicht beeinträchtigen, also beispielsweise die Alkalimetallsalze, insbesondere Natrium- und Kaliumsalze, die Erdalkalimetallsalze, insbesondere Calcium-, Magnesium- und Bariumsalze, übergangsmetallsalze, insbesondere Mangan-, Kupfer-, Zink- und Eisensalze, Ammoniumsalze, die ein bis drei $C_1$-$C_4$-Alkyl- oder

Hydroxy-$C_1$-$C_4$-alkylsubstituenten und/oder einen Phenyl- oder Benzylsubstituenten tragen können, insbesondere Diisopropylammonium-, Tetramethylammonium-, Tetrabutylammonium-, Trimethylbenzylammonium- und Trimethyl-(2-hydroxyethyl)-ammoniumsalze, die Phosphoniumsalze, die Sulfoniumsalze, insbesondere Tri-($C_1$-$C_4$)-alkylsulfoniumsalze sowie die Sulfoxoniumsalze, insbesondere Tri-($C_1$-$C_4$)-alkylsulfoxoniumsalze.

Die pyrido-anellierten 4-Oxo-4H-benzopyrane I und I' eignen sich als Antidots, um herbizide Wirkstoffe für Kulturpflanzen wie Kulturhirse (= Kultursorghum), Reis, Mais, Getreidearten (Weizen, Roggen, Gerste, Hafer), Baumwolle, Zuckerrüben, Zuckerrohr und Soja verträglicher zu machen. Sie wirken antagonistisch auf Herbizide verschiedenster Stoffklassen wie Triazine, Phenylharnstoffderivate, Carbamate, Thiolcarbamate, Halogenacetanilide, Benzoesäurederivate sowie insbesondere Halogenphenoxyessigsäureester, substituierte Phenoxyphenoxyessigsäureester, Phenoxyphenoxypropionsäureester und Cyclohexenonderivate.

Herbizide Wirkstoffe aus der Gruppe der 2-(4-Heteroaryloxy)- oder 2-(4-Aryloxy)-phenoxyessigsäurederivate der Formel V,

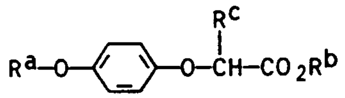

V

in der die Substituenten folgende Bedeutung haben:

- $R^a$ die Phenylgruppe, die Pyridylgruppe, eine Benzoxazylgruppe,
- $R^b$ Wasserstoff, eine $C_1$- $C_4$-Alkylgruppe
- $R^c$ Wasserstoff oder eine Methylgruppe,

sind aus der Literatur, z.B. aus DE-A-22 23 894, DE-A-24 33 067, DE-A-25 76 251, DE-A-30 04 770, BE-A-868 875 und BE-A-858 618 bekannt.

Sie dienen der Bekämpfung von unerwünschten Pflanzen aus der Familie der Gramineen. Die Verträglichkeit dieser Substanzen für Kulturpflanzen variiert jedoch zwischen kommerziell acceptabel und unverträglich, je nach Substituenten und Aufwandmenge.

Ebenso verhält es sich mit Cyclohexenonderivaten der Formel VI,

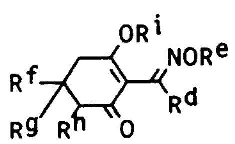

VI

in welcher die Substituenten folgende Bedeutung haben:

- $R^d$ eine $C_1$-$C_4$-Alkylgruppe wie Methyl, Ethyl, n-Propyl, Isopropyl, n-Butyl und tert.-Butyl, vorzugsweise Ethyl und n-Propyl;
- $R^e$ eine $C_1$-$C_4$-Alkylgruppe wie Methyl, Ethyl, n-Propyl, Isopropyl, n-Butyl und tert.-Butyl, vorzugsweise Ethyl und n-Propyl, eine $C_3$-$C_4$-Alkenylgruppe, vorzugsweise Prop-2-enyl, eine $C_3$-$C_4$-Alkinylgruppe oder eine $C_3$-$C_4$-Halogenalkenylgruppe, vorzugsweise 3-Chlor-prop-2-en-1-yl,
  eine $C_2$-$C_4$-Alkylen- oder $C_3$-$C_4$-Alkenylenkette, die beide noch ein bis drei $C_1$-$C_3$-Alkylreste und/oder Halogenatome tragen können, oder eine gewünschtenfalls durch $C_1$-$C_3$-Alkyl substituierte 3- bis 6-gliedrige Alkylen- oder 4- bis 6-gliedrige Alkenylenkette, die jeweils ein dem Oximetherteil nicht direkt benachbartes Sauerstoff- oder Schwefelatom als Kettenglied enthält, wobei alle vorstehend genannten Ketten endständig den Phenylring tragen, der seinerseits durch ein bis drei Reste substituiert sein kann, ausgewählt aus einer Gruppe bestehend aus einem Benzyloxycarbonyl- oder Phenylrest und jeweils einem bis drei der folgenden Reste: Nitro, Cyano, Halogen, $C_1$-$C_4$-Alkyl, $C_1$-$C_4$-Alkoxy, $C_1$-$C_4$-Alkylthio, partiell oder vollständig halogeniertes $C_1$-$C_4$-Alkyl, partiell oder vollständig halogeniertes $C_1$-$C_4$-Alkoxy, Carboxyl und $C_1$-$C_4$-Alkoxycarbonyl, und wobei der Phenylring noch zusätzlich so viele Halogenatome tragen kann, daß die Gesamtzahl der Reste 4 oder 5 beträgt; besonders bevorzugt sind 4-(p-Fluorphenyl)-but-3-enyl, 4-(p-Chlorphenyl)-but-3-enyl und 2-(p-Chlorphenoxy)-propyl;
  die Thienylgruppe, die noch ein Halogenatom tragen kann;
- $R^f$ eine $C_1$-$C_4$-Alkylgruppe wie bei $R^d$ genannt, welche ein- oder zweifach durch $C_1$-$C_4$-Alkylthio oder $C_1$-$C_4$-Alkoxy substituiert sein kann;
  ein 5- oder 6-gliedriges gesättigtes oder einfach ungesättigtes Ringsystem, welches neben

Kohlenstoffgliedern ein Sauerstoff-, ein Schwefelatom oder eine Sulfoxid- oder Sulfongruppe enthalten kann, vorzugsweise Tetrahydropyranyl, Dihydropyranyl und Tetrahydrothiopyranyl, wobei das Ringsystem noch einen bis drei Reste tragen kann, ausgewählt aus einer Gruppe bestehend aus Hydroxy, Halogen, $C_1$-$C_4$-Alkyl, $C_1$-$C_4$-Halogenalkyl, $C_1$-$C_4$-Alkoxy und $C_1$-$C_4$-Alkylthio;

einen 10-gliedrigen gesättigten oder einfach ungesättigten Heterocyclus, welcher zwei nicht benachbarte Sauerstoffatome oder Schwefelatome enthält und der durch bis zu drei $C_1$-$C_4$-Alkylgruppen und/oder Methoxygruppen substituiert sein kann;

die Phenylgruppe, eine Pyridylgruppe, eine Thiazolylgruppe, eine Pyrazolylgruppe, eine Pyrrolylgruppe oder eine Isoxazolylgruppe, wobei diese Gruppen jeweils ein bis drei Reste tragen können, ausgewählt aus einer Gruppe bestehend aus $C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und Benzoylamino;

$R^g$ Wasserstoff, Hydroxy oder, wenn $R^f$ die Bedeutung $C_1$-$C_6$-Alkylgruppe hat, eine $C_1$-$C_6$-Alkylgruppe, vorzugsweise Wasserstoff;

$R^h$ Wasserstoff, die Cyanogruppe, ein Halogenatom, eine $C_1$-$C_4$-Alkoxycarbonylgruppe oder eine $C_1$-$C_4$-Alkylketoximgruppe, vorzugsweise Wasserstoff;

$R^i$ Wasserstoff.

Sie sind in der Literatur (z.B. EP-A 228 598, EP-A 230 235, EP-A 238 021, EP-A 368 227, US-A 4 432 786, DE-A 24 39 104, DE-A 40 14 986 und DE-A 40 33 423) ebenfalls als Herbizide beschrieben und dienen vorwiegend zur Bekämpfung unerwünschter Gräser in dicotylen Kulturen und in Gräsern, die nicht zur Familie der Gramineen zählen. Je nach Struktur der Substituenten und Dosis der Anwendung können Verbindungen aus dieser Gruppe auch zur selektiven Bekämpfung von unerwünschten Gräsern in Gramineen Kulturen wie Weizen und Reis eingesetzt werden.

Cyclohexenonderivate der Formel VI, in denen $R^e$ für einen unsubstituierten oder substituierten Alkyl- oder Alkenyl-, z.B. Butyl- oder Butenylphenylrest steht, können in an sich bekannter Weise aus schon bekannten Derivaten der Formel VII (EP-A-80 301, EP-A-125 094, EP-A 142 741, US-A 4 249 937, EP-A 137 174 und EP-A 177 913) und den entsprechenden Hydroxylaminen der Formel VIII (Houben-Weyl, 10/1, S. 1181 ff) hergestellt werden (EP-A 169 521).

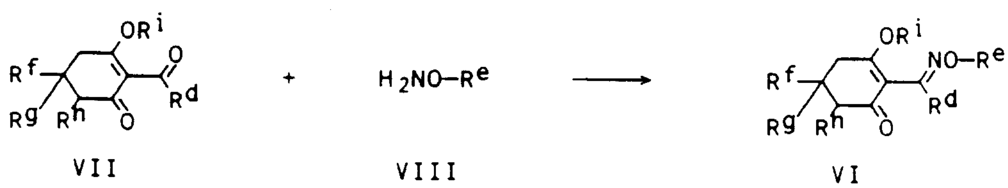

Zweckmäßig führt man die Umsetzung in heterogener Phase in einem Lösungsmittel, bei einer ausreichenden Temperatur unterhalb von etwa 80 °C, in Gegenwart einer Base durch und verwendet das Hydroxylamin VIII in Form seines Ammoniumsalzes.

Geeignete Basen sind z. B. Carbonate` Hydrogencarbonate, Acetate, Alkoholate oder Oxide von Alkali- oder Erdalkalimetallen, insbesondere Natriumhydroxid, Kaliumhydroxid, Magnesiumoxid, Calciumoxid. Außerdem können organische Basen wie Pyridin oder tertiäre Amine Verwendung finden. Die Base wird beispielsweise in einer Menge von 0,5 bis 2 Mol-Äquivalent bezogen auf die Ammoniumverbindung zugesetzt.

Als Lösungsmittel eignen sich beispielsweise Dimethylsulfoxid, Alkohole wie Methanol, Ethanol und Isopropanol, aromatische Kohlenwasserstoffe wie Benzol und Toluol, chlorierte Kohlenwasserstoffe wie Chloroform und Dichlorethan, aliphatische Kohlenwasserstoffe wie Hexan und Cyclohexan, Ester wie Essigsäureethylester sowie Ether wie Diethylether, Dioxan und Tetrahydrofuran. Vorzugsweise führt man die Umsetzung in Methanol mit Natriumhydrogencarbonat als Base durch.

Die Reaktion ist nach wenigen Stunden beendet. Das Produkt kann z.B. durch Einengen der Mischung, Verteilung des Rückstandes in Methylenchlorid/ Wasser und Abdestillieren des Lösungsmittels unter vermindertem Druck isoliert werden.

Man kann für diese Umsetzung aber auch unmittelbar die freie Hydroxylaminbase, z.B. in Form einer wäßrigen Lösung, verwenden; je nach verwendetem Lösungsmittel für die Verbindung VII erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol, aliphatische und aromatische, gegebenenfalls chlorierte Kohlenwasserstoffe wie Hexan, Cyclohexan, Methylenchlorid, Toluol und Dichlorethan, Ester wie Essigsäureethylester, Nitrile wie Acetonitril sowie cyclische Ether wie Dioxan und Tetrahydrofuran.

Alkalimetallsalze der Verbindungen VI können durch Behandeln der 3-Hydroxyverbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Ammonium-, Phosphonium-, Sulfonium- oder Sulfoxoniumhydroxiden.

Die Verbindungen des Typs VII können z.B. aus den entsprechenden Cyclohexan-1,3-dionen der Formel IX

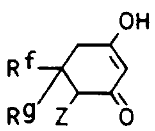

IX,

in der

Z Wasserstoff oder Methoxycarbonyl bedeutet und

$R^g$ Wasserstoff bedeutet

nach bekannten Methoden (Tetrahedron Lett., 2491 (1975)) hergestellt werden.

Es ist auch möglich, die Verbindungen der Formel VII über die Zwischenstufe der Enolester herzustellen, die bei der Umsetzung von Verbindungen der Formel IX mit Säurechloriden in Gegenwart von Basen anfallen und anschließend mit bestimmten Imidazol-oder Pyridinderivaten umgelagert werden (JP-OS 79/063 052).

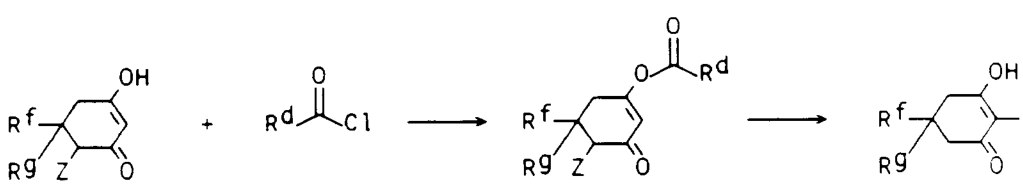

IX VII

Zu den Verbindungen der Formel IX gelangt man über eine Reihe bekannter Verfahrensschritte ausgehend von bekannten Vorprodukten.

Die Synthese der Hydroxylamine VIII, in denen $R^e$ für einen ggf. substituierten Phenylbutylrest steht, erfolgt gemäß dem nachstehenden Reaktionsschema beispielsweise über

α) die Alkylierung von cyclischen Hydroxyimiden X mit geeigneten Phenylbutylhalogeniden und anschließende Schutzgruppenabspaltung beispielsweise mit Hydrazin oder Ethanolamin analog Beispielen aus EP-A-244 786 bzw. Houben-Weyl, Methoden der organischen Chemie, Band X/1, Seite 1152ff.

β) Hydrierung von N-4-Phenylbutenyloxyphthalimiden, deren Herstellung in DE-A 38 38 310 beschrieben ist, mittels geeigneten Katalysatoren wie z.B. Palladium auf Aktivkohle, in geeigneten inerten Lösungsmitteln wie z.B. Methanol, Tetrahydrofuran, Dioxan und anschließende Schutzgruppenabspaltung wie voranstehend beschrieben.

Vorteilhaft wird die Hydrierung bei Temperaturen von 20 °C bis zum Siedepunkt des Lösungsmittels, insbesondere bei Raumtemperatur nach den dafür üblichen Techniken bei Atmosphärendruck, über- oder Unterdruck durchgeführt. Bevorzugt ist ein Druckbereich von 1 bis 10, insbesondere 1 bis 2 bar.

Reaktionsschema:

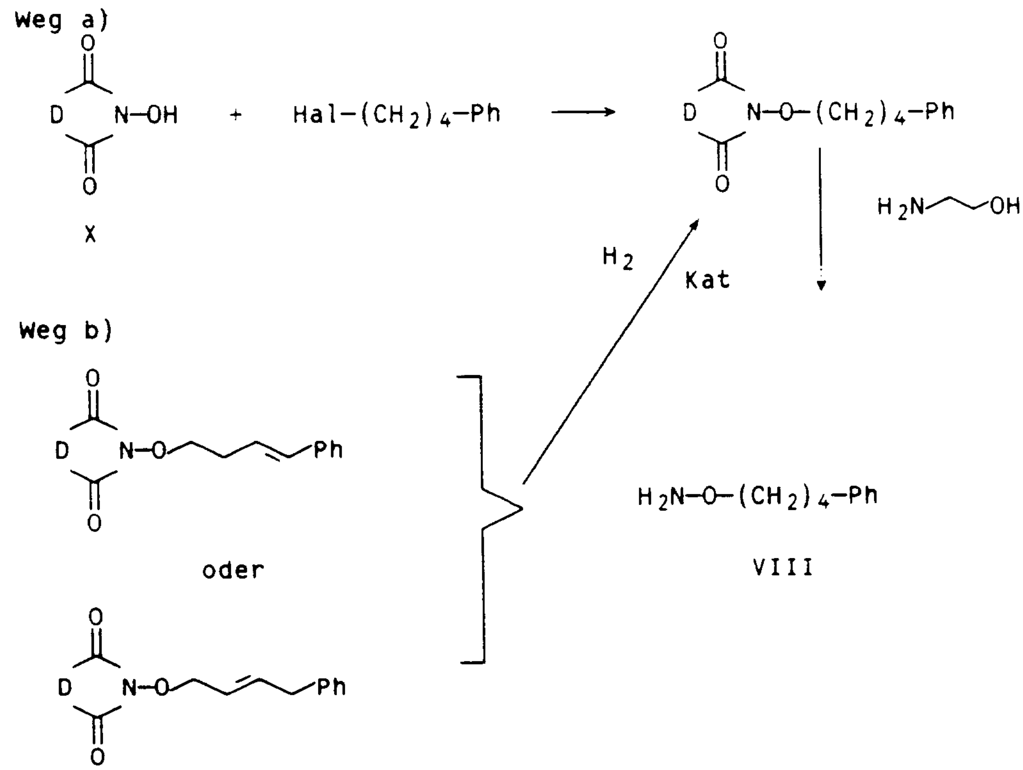


Ph = ggf. substituierter Phenylrest

Als cyclische Hydroxyimide X kommen beispielsweise folgende Substanzen in Betracht:

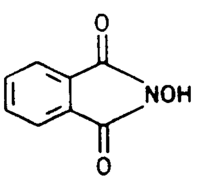 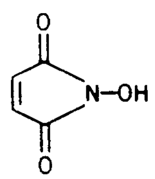 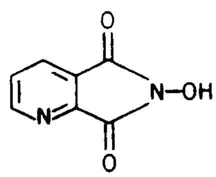 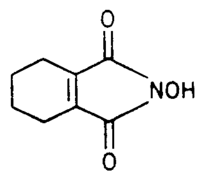

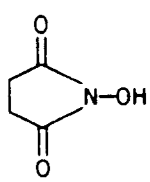 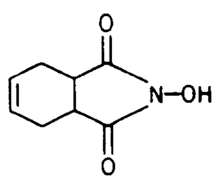 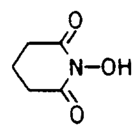

Die Synthese der Hydroxylamine VIII, in denen $R^e$ für einen unsubstituierten oder substituierten Butenylphenylrest steht, wobei der nachstehend mit Ph abgekürzte Phenylrest seinerseits substituiert oder unsubstituiert sein kann, erfolgt gemäß dem nachstehenden Reaktionsschema ausgehend von Anilinderivaten durch Diazotierung und anschließende Kupplung des Diazoniumsalzes mit einem entsprechend substituierten Butadien XI. Das so erhaltene Gemisch aus XIIa und XIIb wird mit einem cyclischen Hydroxyimid XIV gekoppelt und das erhaltene geschützte Hydroxylaminderivat XIII mit 2-Aminoethanol zum freien Hydroxylamin VIII gespalten:

Weg c)

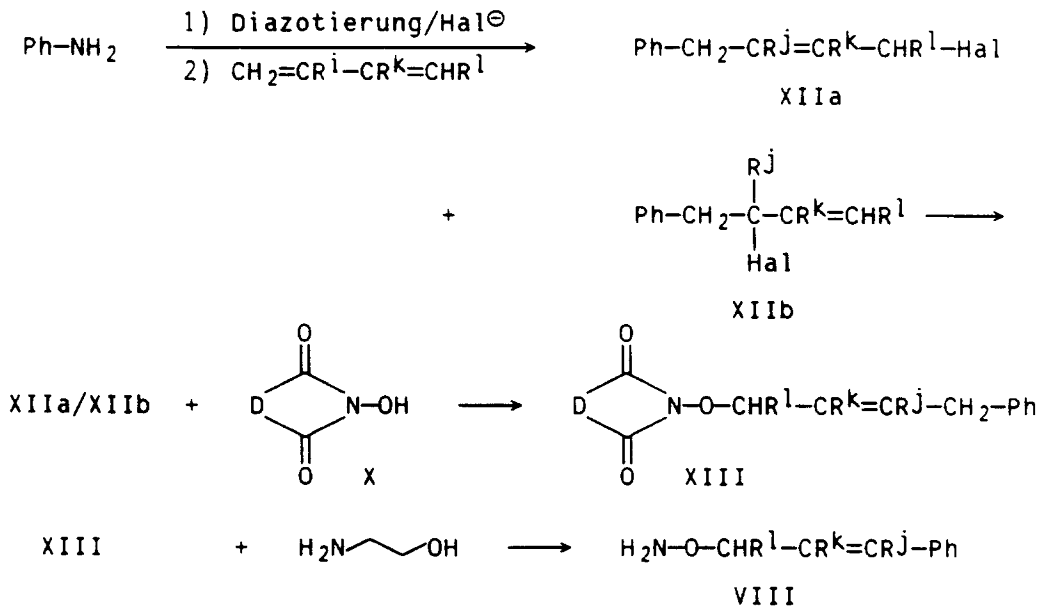


XIIa, XIIb, X, XIII, VIII

Die Reste $R^j$, $R^k$ und $R^l$ bedeuten hierbei unabhängig voneinander Wasserstoff, $C_1$-$C_3$-Alkylgruppen und/oder Halogenatome. Hal bedeutet Halogenatom, vorzugsweise Chloratom.

Die zur obigen Synthese der Hydroxylamine der Formel VIII benötigten Halogenide XIIa lassen sich nach literaturbekannten Verfahren im Gemisch mit XIIb herstellen, beispielsweise durch die Umsetzung von Diazoniumsalzen aromatischer und heteroaromatischer Aniline mit Dienen. Die Anwendungsbreite der Reaktion ist in Organic Reactions 11 (1960) 189 bzw. 24 (1976) 225 abgehandelt.

Bei der Kopplung der isomeren Halogenide XIIa und XIIb an ein cyclisches Hydroxyimid der Formel X entstehen ausschließlich die cyclischen Imidether der Formel XIII, die nach Abspaltung der Schutzgruppe am Stickstoff die Hydroxylamine VIII liefern.

Die Umsetzung mit einem Hydroxyimid X (weg a und c) erfolgt in Gegenwart eines säurebindenden Mittels und eines Lösungsmittels. Aus Kostengründen kommt als Hydroxyimid X bevorzugt Hydroxyphthalimid zum Einsatz.

Als säurebindende Mittel eignen sich Alkalimetallcarbonate wie Kalium- oder Natriumcarbonat, Alkalimetallhydrogencarbonate wie Kalium- und Natriumhydrogencarbonat, tertiäre Amine wie Trimethyl- und Triethylamin und basische Heterocyclen wie Pyridin. Aus Kostengründen kommen bevorzugt Kalium- und Natriumcarbonat in Betracht.

Als Lösungsmittel eignen sich aprotisch dipolare organische Lösungsmittel wie z.B. Dimethylformamid, Dimethylsulfoxid und/oder Sulfolan.

Ferner ist auch eine Alkylierung unter Phasentransferbedingungen möglich. Als organische Lösungsmittel werden hierbei mit Wasser nicht mischbare Verbindungen wie Kohlenwasserstoffe oder Chlorkohlenwasserstoffe eingesetzt. Als Phasentransferkatalysatoren eignen sich quartäre Ammonium- und Phosphoniumsalze.

Die Spaltung der cyclischen Imidether XIII gelingt analog einem in EP-A 244 786 beschriebenen Verfahren mit Alkanolaminen. Die Hydroxylamine VIII können nach diesem Verfahren als freie Basen oder nach Fällung mit Säuren als Salze isoliert werden. Gut kristallisierende Salze erhält man durch Umsetzung der Basen mit Oxalsäure.

Spezielle Beispiele für herbizide (Heteroaryloxy)- bzw. Aryloxyphenoxyessigsäurederivate der Formel V, deren Kulturpflanzenverträglichkeit durch pyrido-anellierte 4-Oxo-4H-benzopyrane der Formel I und I' verbessert werden kann, sind in der folgenden Tabelle 1 aufgeführt.

Tabelle 1

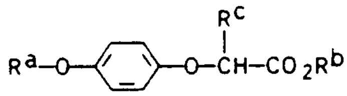

V

| Nr. | $R^a$ | $R^b$ | $R^c$ | Literatur |
|---|---|---|---|---|
| 1.01 | 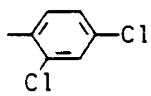 | $CH_3$ | $CH_3$ | DE-A 22 23 894 |
| 1.02 | 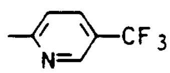 | n-$C_4H_9$ | $CH_3$ | BE-A 868 875 |
| 1.03 | 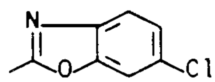 | $C_2H_5$ | $CH_3$ | BE-A 858 618 |
| 1.04 | 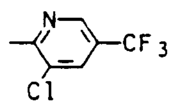 | $CH_3$ | $CH_3$ | BE-A 868 875 |
| 1.05 | 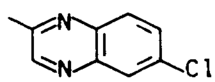 | $C_2H_5$ | $CH_3$ | DE-A 30 04 770 |

Spezielle Beispiele für Cyclohexenone der Formel VI, deren Kulturpflanzenverträglichkeit durch pyridoanellierte 4-Oxo-4H-benzopyrane I und I' verbessert werden kann, sind den folgenden Tabellen 2 bis 13 zu entnehmen.

Tabelle 2

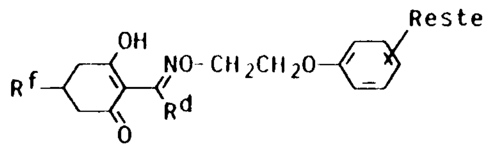 VI ($R^e$ = $-CH_2CH_2-O-$ 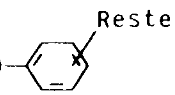 ; $R^g$, $R^h$, $R^i$ = H)

| Nr. | $R^d$ | $R^f$ | Reste am Phenylring | phys. Daten / ¹H-NMR [δ in ppm], Fp. [°C] |
|---|---|---|---|---|
| 2.01 | Ethyl | Tetrahydropyran-3-yl | - | 42- 45 |
| 2.02 | Propyl | Tetrahydropyran-3-yl | - | 3,90 (m,2H), 4,20 (t,2H), 4,40 (m, 2H), 6,80-7,00 (m,3H), 7,13-7,37 (m,2H) |
| 2.03 | Ethyl | Tetrahydropyran-4-yl | - | 106-107 |
| 2.04 | Propyl | Tetrahydropyran-4-yl | - | 72- 73 |
| 2.05 | Ethyl | Tetrahydrothiopyran-3-yl | - | 52- 55 |
| 2.06 | Propyl | Tetrahydrothiopyran-3-yl | - | 92 |
| 2.07 | Ethyl | Tetrahydropyran-3-yl | 2-F | 76- 78 |
| 2.08 | Propyl | Tetrahydropyran-3-yl | 2-F | 72- 77 |
| 2.09 | Ethyl | Tetrahydropyran-4-yl | 2-F | 121-125 |
| 2.10 | Propyl | Tetrahydropyran-4-yl | 2-F | 103-107 |
| 2.11 | Ethyl | Tetrahydrothiopyran-3-yl | 2-F | 82- 86 |
| 2.12 | Propyl | Tetrahydrothiopyran-3-yl | 2-F | 81- 85 |
| 2.13 | Ethyl | Tetrahydropyran-3-yl | 3-F | 62- 68 |
| 2.14 | Propyl | Tetrahydropyran-3-yl | 3-F | 3,90 (m,2H), 4,20 (t,2H), 4,40 (m,2H) 6,70 (m,3H), 7,25 (m,1H), |
| 2.15 | Ethyl | Tetrahydropyran-4-yl | 3-F | 103-109 |

Tabelle 2 (Fortsetzung)

| Nr. | $R^d$ | $R^f$ | Reste am Phenylring | phys. Daten / $^1$H-NMR [δ in ppm], Fp. [°C] |
|---|---|---|---|---|
| 2.16 | Propyl | Tetrahydropyran-4-yl | 3-F | 73- 79 |
| 2.17 | Ethyl | Tetrahydrothiopyran-3-yl | 3-F | 4,20 (t, 2H), 4,40 (m, 2H) 6,70 (m, 3H), 7,25 (m, 1H) |
| 2.18 | Propyl | Tetrahydrothiopyran-3-yl | 3-F | |
| 2.19 | Ethyl | Tetrahydropyran-3-yl | 4-F | 64- 67 |
| 2.20 | Propyl | Tetrahydropyran-3-yl | 4-F | 70- 72 |
| 2.21 | Ethyl | Tetrahydropyran-4-yl | 4-F | 101-103 |
| 2.22 | Propyl | Tetrahydropyran-4-yl | 4-F | 107-109 |
| 2.23 | Ethyl | Tetrahydrothiopyran-3-yl | 4-F | 105-108 |
| 2.24 | Propyl | Tetrahydrothiopyran-3-yl | 4-F | 82- 84 |
| 2.25 | Ethyl | Tetrahydropyran-3-yl | 2-Cl | 74- 80 |
| 2.26 | Propyl | Tetrahydropyran-3-yl | 2-Cl | 67- 71 |
| 2.27 | Ethyl | Tetrahydropyran-4-yl | 2-Cl | 4,00 (m, 2H), 4,27 (t, 2H), 4,47 (m, 2H), 7,20 (t, 1H), 7,37 (d, 1H) |
| 2.28 | Propyl | Tetrahydropyran-4-yl | 2-Cl | 68- 72 |
| 2.29 | Ethyl | Tetrahydrothiopyran-3-yl | 2-Cl | 74- 78 |
| 2.30 | Propyl | Tetrahydrothiopyran-3-yl | 2-Cl | 72-78 |
| 2.31 | Ethyl | Tetrahydropyran-3-yl | 3-Cl | |
| 2.32 | Propyl | Tetrahydropyran-3-yl | 3-Cl | |
| 2.33 | Ethyl | Tetrahydropyran-4-yl | 3-Cl | |
| 2.34 | Propyl | Tetrahydropyran-4-yl | 3-Cl | |

Tabelle 2 (Fortsetzung)

| Nr. | $R^d$ | $R^f$ | Reste am Phenylring | phys. Daten / $^1H$-NMR [$\delta$ in ppm], Fp. [°C] |
|---|---|---|---|---|
| 2.35 | Ethyl | Tetrahydrothiopyran-3-yl | 3-Cl | |
| 2.36 | Propyl | Tetrahydrothiopyran-3-yl | 3-Cl | |
| 2.37 | Ethyl | Tetrahydropyran-3-yl | 4-Cl | 3,93 (m,2H), 4,20 (t,2H), 4,43 (m,2H), 6,90 (m, 2H), 7,25 (m,2H) |
| 2.38 | Propyl | Tetrahydropyran-3-yl | 4-Cl | 3,93 (m,2H), 4,20 (t,2H), 4,43 (m,2H), 6,90 (m, 2H), 7,25 (m,2H) |
| 2.39 | Ethyl | Tetrahydropyran-4-yl | 4-Cl | 116-118 |
| 2.40 | Propyl | Tetrahydropyran-4-yl | 4-Cl | 104-106 |
| 2.41 | Ethyl | Tetrahydrothiopyran-3-yl | 4-Cl | 74- 77 |
| 2.42 | Propyl | Tetrahydrothiopyran-3-yl | 4-Cl | 86- 88 |
| 2.43 | Ethyl | Tetrahydropyran-3-yl | 2-$CF_3$ | |
| 2.44 | Propyl | Tetrahydropyran-3-yl | 2-$CF_3$ | |
| 2.45 | Ethyl | Tetrahydropyran-4-yl | 2-$CF_3$ | |
| 2.46 | Propyl | Tetrahydropyran-4-yl | 2-$CF_3$ | |
| 2.47 | Ethyl | Tetrahydrothiopyran-3-yl | 2-$CF_3$ | |
| 2.48 | Propyl | Tetrahydrothiopyran-3-yl | 2-$CF_3$ | |
| 2.49 | Ethyl | Tetrahydropyran-3-yl | 3-$CF_3$ | |
| 2.50 | Propyl | Tetrahydropyran-3-yl | 3-$CF_3$ | |
| 2.51 | Ethyl | Tetrahydropyran-4-yl | 3-$CF_3$ | |
| 2.52 | Propyl | Tetrahydropyran-4-yl | 3-$CF_3$ | |
| 2.53 | Ethyl | Tetrahydrothiopyran-3-yl | 3-$CF_3$ | |

Tabelle 2 (Fortsetzung)

| Nr. | $R^d$ | $R^f$ | Reste am Phenylring | phys. Daten / $^1H$-NMR [δ in ppm], Fp. [°C] |
|---|---|---|---|---|
| 2.54 | Propyl | Tetrahydrothiopyran-3-yl | $3-CF_3$ | |
| 2.55 | Ethyl | Tetrahydropyran-3-yl | $4-CF_3$ | 72- 77 |
| 2.56 | Propyl | Tetrahydropyran-3-yl | $4-CF_3$ | 3,90 (m, 2H), 4,27 (t, 2H), 4,47 (m, 2H) 7,00 (d, 2H), 7,55 (d, 2H) |
| 2.57 | Ethyl | Tetrahydropyran-4-yl | $4-CF_3$ | |
| 2.58 | Propyl | Tetrahydropyran-4-yl | $4-CF_3$ | 90- 94 |
| 2.59 | Ethyl | Tetrahydrothiopyran-3-yl | $4-CF_3$ | 73- 79 |
| 2.60 | Propyl | Tetrahydrothiopyran-3-yl | $4-CF_3$ | 4,27 (t, 2H), 4,47 (m, 2H), 7,00 (d, 2H) 7,55 (d, 2H) |
| 2.61 | Ethyl | Tetrahydropyran-3-yl | $2,4-Cl_2$ | 73- 75 |
| 2.62 | Propyl | Tetrahydropyran-3-yl | $2,4-Cl_2$ | 69- 73 |
| 2.63 | Ethyl | Tetrahydropyran-4-yl | $2,4-Cl_2$ | 4,00 (m, 2H), 4,25 (t, 2H), 4,45 (t, 2H) 6,87 (d, 1H), 7,17 (d, 1H), 7,37 (d, 1H) |
| 2.64 | Propyl | Tetrahydropyran-4-yl | $2,4-Cl_2$ | 4,00 (m, 2H), 4,25 (t, 2H), 4,45 (t, 2H) 6,87 (d, 1H), 7,17 (d, 1H), 7,37 (d, 1H) |
| 2.65 | Ethyl | Tetrahydrothiopyran-3-yl | $2,4-Cl_2$ | 4,25 (t, 2H), 4,45 (t, 2H), 6,87 (d, 1H) 7,17 (d, 1H), 7,37 (d, 1H) |
| 2.66 | Propyl | Tetrahydrothiopyran-3-yl | $2,4-Cl_2$ | 4,25 (t, 2H), 4,45 (t, 2H), 6,87 (d, 1H) 7,17 (d, 1H), 7,37 (d, 1H) |
| 2.67 | Ethyl | Tetrahydropyran-3-yl | $2,4,6-Cl_3$ | 90- 93 |
| 2.68 | Propyl | Tetrahydropyran-3-yl | $2,4,6-Cl_3$ | 83- 87 |

Tabelle 2 (Fortsetzung)

| Nr. | $R^d$ | $R^f$ | Reste am Phenylring | phys. Daten / $^1$H-NMR [δ in ppm], Fp. [°C] |
|---|---|---|---|---|
| 2.69 | Ethyl | Tetrahydropyran-4-yl | 2,4,6-$Cl_3$ | 79- 82 |
| 2.70 | Propyl | Tetrahydropyran-4-yl | 2,4,6-$Cl_3$ | 4,00 (m,2H), 4,27 (t,2H), 4,45 (m,2H), 7,32 (s,2H) |
| 2.71 | Ethyl | Tetrahydrothiopyran-3-yl | 2,4,6-$Cl_3$ | 105-108 |
| 2.72 | Propyl | Tetrahydrothiopyran-3-yl | 2,4,6-$Cl_3$ | 4,27 (t,2H), 4,45 (m,2H), 7,82 (s,2H) |
| 2.73 | Ethyl | Tetrahydropyran-3-yl | 4-$NO_2$ | 3,90 (m,2H), 4,32 (m,2H), 4,50 (m,2H), 7,00 (d,2H), 8,20 (d,2H) |
| 2.74 | Propyl | Tetrahydropyran-3-yl | 4-$NO_2$ | 3,90 (m,2H), 4,32 (m,2H), 4,50 (m,2H) 7,00 (d,2H), 8,20 (d,2H) |
| 2.75 | Ethyl | Tetrahydropyran-4-yl | 4-$NO_2$ | 126-129 |
| 2.76 | Propyl | Tetrahydropyran-4-yl | 4-$NO_2$ | 138-141 |
| 2.77 | Ethyl | Tetrahydrothiopyran-3-yl | 4-$NO_2$ | 4,32 (m,2H), 4,50 (m,2H), 7,00 (d,2H), 8,20 (d,2H) |
| 2.78 | Propyl | Tetrahydrothiopyran-3-yl | 4-$NO_2$ | 4,32 (m,2H), 4,50 (m,2H), 7,00 (d,2H) 8,20 (d,2H) |

Tabelle 3

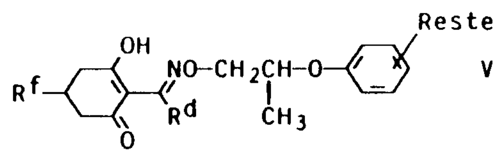 VI ($R^e$ = $-CH_2CH(CH_3)-O-$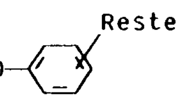 ; $R^g$, $R^h$, $R^i$ = H)

| Nr. | $R^d$ | $R^f$ | Reste am Phenylring | phys. Daten / $^1$H-NMR [δ in ppm], Fp. [°C] |
|---|---|---|---|---|
| 3.01 | Ethyl | Tetrahydropyran-3-yl | - | |
| 3.02 | Propyl | Tetrahydropyran-3-yl | - | |
| 3.03 | Ethyl | Tetrahydropyran-4-yl | - | |
| 3.04 | Propyl | Tetrahydropyran-4-yl | - | |
| 3.05 | Ethyl | Tetrahydrothiopyran-3-yl | - | |
| 3.06 | Propyl | Tetrahydrothiopyran-3-yl | - | |
| 3.07 | Ethyl | Tetrahydropyran-3-yl | 4-F | |
| 3.08 | Propyl | Tetrahydropyran-3-yl | 4-F | |
| 3.09 | Ethyl | Tetrahydropyran-4-yl | 4-F | |
| 3.10 | Propyl | Tetrahydropyran-4-yl | 4-F | |
| 3.11 | Ethyl | Tetrahydrothiopyran-3-yl | 4-F | |
| 3.12 | Propyl | Tetrahydrothiopyran-3-yl | 4-F | |
| 3.13 | Ethyl | Tetrahydropyran-3-yl | 4-Cl | |
| 3.14 | Propyl | Tetrahydropyran-3-yl | 4-Cl | |
| 3.15 | Ethyl | Tetrahydropyran-4-yl | 4-Cl | 1,35 (m, 3H), 4,05-4,30 (m, 2H), 4,60 (m, 1H), 6,80-7,40 (m, 4H) |

Tabelle 3 (Fortsetzung)

| Nr. | $R^d$ | $R^f$ | Reste am Phenylring | phys. Daten / $^1$H-NMR [$\delta$ in ppm], Fp. [°C] |
|---|---|---|---|---|
| 3.16 | Propyl | Tetrahydropyran-4-yl | 4-Cl | 1,35 (m, 3H), 4,05-4,30 (m, 2H), 4,60 (m, 1H), 6,80-7,40 (m, 4H) |
| 3.17 | Ethyl | Tetrahydrothiopyran-3-yl | 4-Cl | 1,35 (m, 3H), 4,05-4,25 (m, 2H), 4,60 (m, 1H), 6,80-7,40 (m, 4H) |
| 3.18 | Propyl | Tetrahydrothiopyran-3-yl | 4-Cl | 1,35 (m, 3H), 4,05-4,30 (m, 2H), 4,60 (m, 1H), 6,80-7,40 (m, 4H) |

Tabelle 4

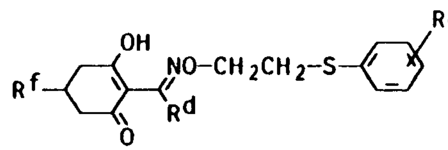 VI ($R^e$ = $-CH_2CH_2-S-$ 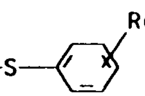 ; $R^g$, $R^h$, $R^i$ = H)

| Nr. | $R^d$ | $R^f$ | Reste am Phenylring | phys. Daten / $^1$H-NMR [δ in ppm], Fp. [°C] |
|---|---|---|---|---|
| 4.01 | Ethyl | Tetrahydropyran-3-yl | - | 3,90 (m, 2H), 4,23 (t, 2H), 7,17-7,43 (m, 5H) |
| 4.02 | Propyl | Tetrahydropyran-3-yl | - | 65 |
| 4.03 | Ethyl | Tetrahydropyran-4-yl | - | 3,97 (m, 2H), 4,23 (t, 2H), 7,17-7,43 (m, 5H) |
| 4.04 | Propyl | Tetrahydropyran-4-yl | - | 3,97 (m, 2H), 4,23 (t, 2H), 7,17-7,43 (m, 5H) |
| 4.05 | Ethyl | Tetrahydrothiopyran-3-yl | - | 4,23 (t, 2H), 7,17-7,43 (m, 5H) |
| 4.06 | Propyl | Tetrahydrothiopyran-3-yl | - | 4,23 (t, 2H), 7,17-7,43 (m, 5H) |
| 4.07 | Ethyl | Tetrahydropyran-3-yl | 4-F | 3,90 (m, 2H), 4,17 (t, 2H), 7,00 (m, 2H), 7,40 (m, 2H) |
| 4.08 | Propyl | Tetrahydropyran-3-yl | 4-F | 3,90 (m, 2H), 4,17 (t, 2H), 7,00 (m, 2H), 7,40 (m, 2H) |
| 4.09 | Ethyl | Tetrahydropyran-4-yl | 4-F | 4,00 (m, 2H), 4,17 (t, 2H), 7,00 (m, 2H), 7,40 (m, 2H) |
| 4.10 | Propyl | Tetrahydropyran-4-yl | 4-F | 4,00 (m, 2H), 4,17 (t, 2H), 7,00 (m, 2H), 7,40 (m, 2H) |
| 4.11 | Ethyl | Tetrahydrothiopyran-3-yl | 4-F | 4,17 (t, 2H), 7,00 (m, 2H), 7,40 (m, 2H), |
| 4.12 | Propyl | Tetrahydrothiopyran-3-yl | 4-F | 4,17 (t, 2H), 7,00 (m, 2H), 7,40 (m, 2H), |
| 4.13 | Ethyl | Tetrahydropyran-3-yl | 4-Cl | 71- 75 |

Tabelle 4 (Fortsetzung)

| Nr. | $R^d$ | $R^f$ | Reste am Phenylring | phys. Daten / $^1H$-NMR [δ in ppm], Fp. [°C] |
|---|---|---|---|---|
| 4.14 | Propyl | Tetrahydropyran-3-yl | 4-Cl | 63- 65 |
| 4.15 | Ethyl | Tetrahydropyran-4-yl | 4-Cl | 4,00 (m,2H), 4,20 (t,2H), 7,30 (m,4H) |
| 4.16 | Propyl | Tetrahydropyran-4-yl | 4-Cl | 4,00 (m,2H), 4,20 (t,2H), 7,30 (m,4H) |
| 4.17 | Ethyl | Tetrahydrothiopyran-3-yl | 4-Cl | 4,20 (t,2H), 7,30 (m,4H) |
| 4.18 | Propyl | Tetrahydrothiopyran-3-yl | 4-Cl | 4,20 (t,2H), 7,30 (m,4H) |
| 4.19 | Ethyl | Tetrahydropyran-3-yl | 2-Cl | 3,90 (m,2H), 4,25 (t,2H), 7,10-7,50 (m,4H) |
| 4.20 | Propyl | Tetrahydropyran-3-yl | 2-Cl | 3,90 (m,2H), 4,25 (t,2H), 7,10-7,50 (m,4H) |
| 4.21 | Ethyl | Tetrahydropyran-4-yl | 2-Cl | 4,00 (m,2H), 4,25 (t,2H), 7,10-7,50 (m,4H) |
| 4.22 | Propyl | Tetrahydropyran-4-yl | 2-Cl | 4,00 (m,2H), 4,25 (t,2H), 7,10-7,50 (m,4H) |
| 4.23 | Ethyl | Tetrahydrothiopyran-3-yl | 2-Cl | 4,25 (t,2H) 7,10-7,50 (m,4H) |
| 4.24 | Propyl | Tetrahydrothiopyran-3-yl | 2-Cl | 4,25 (t,2H) 7,10-7,50 (m,4H) |
| 4.25 | Ethyl | Tetrahydropyran-3-yl | 2,6-$Cl_2$ | 3,90 (m,2H) 4,20 (t,2H), 7,20 (t,1H) 7,40 (d,2H) |
| 4.26 | Propyl | Tetrahydropyran-3-yl | 2,6-$Cl_2$ | 3,90 (m,2H) 4,20 (t,2H), 7,20 (t,1H) 7,40 (d,2H) |
| 4.27 | Ethyl | Tetrahydropyran-4-yl | 2,6-$Cl_2$ | 61- 64 |

Tabelle 4 (Fortsetzung)

| Nr. | $R^d$ | $R^f$ | Reste am Phenylring | phys. Daten / $^1H$-NMR [δ in ppm], Fp. [°C] |
|---|---|---|---|---|
| 4.28 | Propyl | Tetrahydropyran-4-yl | 2,6-$Cl_2$ | 4,00 (m,2H) 4,20 (t,2H), 7,20 (t,1H) 7,40 (d,2H) |
| 4.29 | Ethyl | Tetrahydrothiopyran-3-yl | 2,6-$Cl_2$ | 4,20 (t,2H) 7,20 (t,2H), 7,40 (d,2H) |
| 4.30 | Propyl | Tetrahydrothiopyran-3-yl | 2,6-$Cl_2$ | 4,20 (t,2H) 7,20 (t,2H), 7,40 (d,2H) |

Tabelle 5

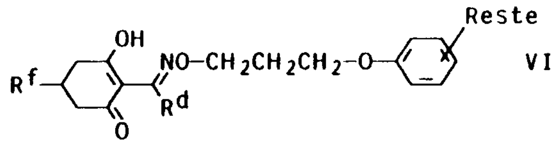 VI ($R^e$ = $-CH_2CH_2-CH_2-O-$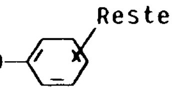 ; $R^g$, $R^h$, $R^i$ = H)

| Nr. | $R^d$ | $R^f$ | Reste am Phenylring | phys. Daten / $^1$H-NMR [$\delta$ in ppm], Fp. [°C] |
|---|---|---|---|---|
| 5.01 | Ethyl | Tetrahydropyran-3-yl | - | 3,90 (m, 2H), 4,03 (t, 2H), 4,23 (t, 2H), 6,90 (m, 3H), 7,27 (m, 2H) |
| 5.02 | Propyl | Tetrahydropyran-3-yl | - | 3,90 (m, 2H), 4,03 (t, 2H), 4,23 (t, 2H), 6,90 (m, 3H), 7,27 (m, 2H) |
| 5.03 | Ethyl | Tetrahydropyran-4-yl | - | 3,97 (m, 2H), 4,03 (t, 2H), 4,23 (t, 2H), 6,90 (m, 3H), 7,27 (m, 2H) |
| 5.04 | Propyl | Tetrahydropyran-4-yl | - | 3,97 (m, 2H), 4,03 (t, 2H), 4,23 (t, 2H), 6,90 (m, 3H), 7,27 (m, 2H) |
| 5.05 | Ethyl | Tetrahydrothiopyran-3- | - | 4,03 (t, 2H), 4,23 (t, 2H), 6,90 (m, 3H), 7,27 (m, 2H) |
| 5.06 | Propyl | Tetrahydrothiopyran-3- | - | 4,03 (t, 2H), 4,23 (t, 2H), 6,90 (m, 3H), 7,27 (m, 2H) |
| 5.07 | Ethyl | Tetrahydropyran-3-yl | 2-F | 3,90 (m, 2H), 4,10 (t, 2H), 4,27 (t, 2H), 6,80-7,15 (m, 4H) |
| 5.08 | Propyl | Tetrahydropyran-3-yl | 2-F | 3,90 (m, 2H), 4,10 (t, 2H), 4,27 (t, 2H), 6,80-7,15 (m, 4H) |

Tabelle 5 (Fortsetzung)

| Nr. | $R^d$ | $R^f$ | Reste am Phenylring | phys. Daten / $^1H$-NMR [δ in ppm], Fp. [°C] |
|---|---|---|---|---|
| 5.09 | Ethyl | Tetrahydropyran-4-yl | 2-F | 4,00 (m,2H), 4,10 (t,2H), 4,27 (t,2H), 6,80-7,15 (m,4H) |
| 5.10 | Propyl | Tetrahydropyran-4-yl | 2-F | 76- 80 |
| 5.11 | Ethyl | Tetrahydrothiopyran-3-yl | 2-F | 4,10 (t,2H), 4,27 (t,2H), 6,80-7,15(m,4H), |
| 5.12 | Propyl | Tetrahydrothiopyran-3-yl | 2-F | 4,10 (t,2H), 4,27 (t,2H), 6,80-7,15(m,4H), |
| 5.13 | Ethyl | Tetrahydropyran-3-yl | 3-F | 3,90 (m,2H), 4,05 (t,2H), 4,27 (t,2H), 6,67 (m,3H), 7,23 (m,1H) |
| 5.14 | Propyl | Tetrahydropyran-3-yl | 3-F | 3,90 (m,2H), 4,05 (t,2H), 4,27 (t,2H), 6,67 (m,3H), 7,23 (m,1H) |
| 5.15 | Ethyl | Tetrahydropyran-4-yl | 3-F | 3,90-4,10 (m,4H), 4,27 (t,2H), 6,67 (m,3H), 7,23 (m,1H) |
| 5.16 | Propyl | Tetrahydropyran-4-yl | 3-F | 3,90-4,10 (m,4H), 4,27 (t,2H), 6,67 (m,3H), 7,23 (m,1H) |
| 5.17 | Ethyl | Tetrahydrothiopyran-3-yl | 3-F | 4,05 (t,2H), 4,27 (t,2H), 6,67 (m,3H) 7,23 (m,1H) |
| 5.18 | Propyl | Tetrahydrothiopyran-3-yl | 3-F | 4,05 (t,2H), 4,27 (t,2H), 6,67 (m,3H) 7,23 (m,1H) |
| 5.19 | Ethyl | Tetrahydropyran-3-yl | 4-F | 3,90 (m,2H), 4,03 (t,2H), 4,27 (t,2H), 6,90 (m,2H), 7,00 (m,2H) |
| 5.20 | Propyl | Tetrahydropyran-3-yl | 4-F | 3,90 (m,2H), 4,03 (t,2H), 4,27 (t,2H), 6,90 (m,2H), 7,00 (m,2H) |

Tabelle 5 (Fortsetzung)

| Nr. | $R^d$ | $R^f$ | Reste am Phenylring | phys. Daten / $^1$H-NMR [δ in ppm], Fp. [°C] |
|---|---|---|---|---|
| 5.21 | Ethyl | Tetrahydropyran-4-yl | 4-F | 3,90-4,06 (m,4H), 4,23 (t,2H), 6,90 (m,2H), 7,00 (m,2H) |
| 5.22 | Propyl | Tetrahydropyran-4-yl | 4-F | 3,90-4,06 (m,4H), 4,28 (t,2H), 6,90 (m,2H), 7,00 (m,2H) |
| 5.23 | Ethyl | Tetrahydrothiopyran-3-yl | 4-F | 4,03 (t,2H), 4,27 (t,2H), 6,90 (m,2H), 7,00 (m,2H) |
| 5.24 | Propyl | Tetrahydrothiopyran-3-yl | 4-F | 4,03 (t,2H), 4,27 (t,2H), 6,90 (m,2H), 7,00 (m,2H) |
| 5.25 | Ethyl | Tetrahydropyran-3-yl | 2-Cl | |
| 5.26 | Propyl | Tetrahydropyran-3-yl | 2-Cl | |
| 5.27 | Ethyl | Tetrahydropyran-4-yl | 2-Cl | |
| 5.28 | Propyl | Tetrahydropyran-4-yl | 2-Cl | |
| 5.29 | Ethyl | Tetrahydrothiopyran-3-yl | 2-Cl | |
| 5.30 | Propyl | Tetrahydrothiopyran-3-yl | 2-Cl | |
| 5.31 | Ethyl | Tetrahydropyran-3-yl | 3-Cl | 3,90 (m,2H), 4,06 (t,2H), 4,27 (t,2H), 6,77 (m,1H), 6,90 (m,2H), 7,17 (m,1H) |
| 5.32 | Propyl | Tetrahydropyran-3-yl | 3-Cl | 3,90 (m,2H), 4,06 (t,2H), 4,27 (t,2H), 6,77 (m,1H), 6,90 (m,2H), 7,17 (m,1H) |
| 5.33 | Ethyl | Tetrahydropyran-4-yl | 3-Cl | 3,90-4,10 (m,4H), 4,27 (t,2H), 6,77 (m,1H), 6,90 (m,2H), 7,17 (m,1H) |

Tabelle 5 (Fortsetzung)

| Nr. | $R^d$ | $R^f$ | Reste am Phenylring | phys. Daten / $^1$H-NMR [δ in ppm], Fp. [°C] |
|---|---|---|---|---|
| 5.34 | Propyl | Tetrahydropyran-4-yl | 3-Cl | 3,90-4,10 (m,4H), 4,27 (t,2H), 6,77 (m,1H), 6,90 (m,2H), 7,17 (m,1H) |
| 5.35 | Ethyl | Tetrahydrothiopyran-3-yl | 3-Cl | 4,06 (t,2H), 4,27 (t,2H), 6,77 (m,1H), 6,90 (m,2H), 7,17 (m,1H) |
| 5.36 | Propyl | Tetrahydrothiopyran-3-yl | 3-Cl | 4,06 (t,2H), 4,27 (t,2H), 6,77 (m,1H), 6,90 (m,2H), 7,17 (m,1H) |
| 5.37 | Ethyl | Tetrahydropyran-3-yl | 4-Cl | 3,90 (m,2H), 4,03 (t,2H), 4,23 (t,2H), 6,80 (m,2H), 7,20 (m,2H) |
| 5.38 | Propyl | Tetrahydropyran-3-yl | 4-Cl | 3,90 (m,2H), 4,03 (t,2H), 4,23 (t,2H), 6,80 (m,2H), 7,20 (m,2H) |
| 5.39 | Ethyl | Tetrahydropyran-4-yl | 4-Cl | 3,90-4,09 (m,4H), 4,23 (t,2H), 6,80 (m,2H), 7,20 (m,2H) |
| 5.40 | Propyl | Tetrahydropyran-4-yl | 4-Cl | 3,90-4,09 (m,4H), 4,23 (t,2H), 6,80 (m,2H), 7,20 (m,2H) |
| 5.41 | Ethyl | Tetrahydrothiopyran-3-yl | 4-Cl | 4,03 (t,2H), 4,23 (t,2H), 6,80 (m,2H), 7,20 (m,2H) |
| 5.42 | Propyl | Tetrahydrothiopyran-3-yl | 4-Cl | 4,03 (t,2H), 4,23 (t,2H), 6,80 (m,2H), 7,20 (m,2H) |
| 5.43 | Ethyl | Tetrahydropyran-3-yl | 4-$NO_2$ | 3,90 (m,2H), 4,20 (t,2H), 4,28 (t,2H), 6,93 (d,2H), 8,20 (d,H) |

Tabelle 5 (Fortsetzung)

| Nr. | $R^d$ | $R^f$ | Reste am Phenylring | phys. Daten / $^1H$-NMR [δ in ppm], Fp. [°C] |
|---|---|---|---|---|
| 5.44 | Propyl | Tetrahydropyran-3-yl | 4-$NO_2$ | 3,90 (m,2H), 4,20 (t,2H), 4,28 (t,2H), 6,93 (d,2H), 8,20 (d,2H) |
| 5.45 | Ethyl | Tetrahydropyran-4-yl | 4-$NO_2$ | 4,00 (m,2H), 4,20 (t,2H), 4,28 (t,2H), 6,93 (d,2H), 8,20 (d,2H) |
| 5.46 | Propyl | Tetrahydropyran-4-yl | 4-$NO_2$ | 4,00 (m,2H), 4,20 (t,2H), 4,28 (t,2H), 6,93 (d,2H), 8,20 (d,2H) |
| 5.47 | Ethyl | Tetrahydrothiopyran-3-yl | 4-$NO_2$ | 4,20 (t,2H), 4,28 (t,2H), 6,93 (d,2H), 8,20 (d,2H) |
| 5.48 | Propyl | Tetrahydrothiopyran-3-yl | 4-$NO_2$ | 4,20 (t,2H), 4,28 (t,2H), 6,93 (d,2H), 8,20 (d,2H) |
| 5.49 | Ethyl | Tetrahydropyran-3-yl | 4-Br | 3,90 (m,2H), 4,00 (t,2H), 4,27 (t,2H), 6,80 (d,2H), 7,37 (d,2H) |
| 5.50 | Propyl | Tetrahydropyran-3-yl | 4-Br | 3,90 (m,2H), 4,00 (t,2H), 4,27 (t,2H), 6,80 (d,2H), 7,37 (d,2H) |
| 5.51 | Ethyl | Tetrahydropyran-4-yl | 4-Br | 3,90-4,10 (m,4H), 4,27 (t,2H), 6,80 (d,2H), 7,37 (d,2H) |
| 5.52 | Propyl | Tetrahydropyran-4-yl | 4-Br | 3,90-4,10 (m,4H), 4,27 (t,2H), 6,80 (d,2H), 7,37 (d,2H) |
| 5.53 | Ethyl | Tetrahydrothiopyran-4-yl | 4-Br | 4,00 (t,2H), 4,27 (t,2H), 6,80 (d,2H), 7,37 (d,2H) |
| 5.54 | Propyl | Tetrahydrothiopyran-4-yl | 4-Br | 4,00 (t,2H), 4,27 (t,2H), 6,80 (d,2H), 7,37 (d,2H) |

Tabelle 6

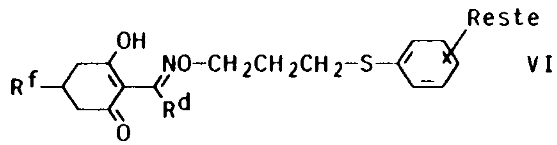 VI (R^e = 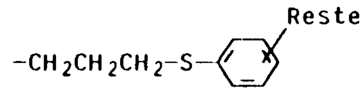 ; $R^g$, $R^h$, $R^i$ = H)

| Nr. | $R^d$ | $R^f$ | Reste am Phenylring | phys. Daten / $^1$H-NMR [δ in ppm], Fp. [°C] |
|---|---|---|---|---|
| 6.01 | Ethyl | Tetrahydropyran-3-yl | - | 3,90 (m, 2H), 4,17 (t, 2H), 7,10-7,40 (m, 5H) |
| 6.02 | Propyl | Tetrahydropyran-3-yl | - | 3,90 (m, 2H), 4,17 (t, 2H), 7,10-7,40 (m, 5H) |
| 6.03 | Ethyl | Tetrahydropyran-4-yl | - | 4,00 (m, 2H), 4,17 (t, 2H), 7,10-7,40 (m, 5H) |
| 6.04 | Propyl | Tetrahydropyran-4-yl | - | 4,00 (m, 2H), 4,17 (t, 2H), 7,10-7,40 (m, 5H) |
| 6.05 | Ethyl | Tetrahydrothiopyran-3-yl | - | 4,17 (t, 2H), 7,10-7,40 (m, 5H) |
| 6.06 | Propyl | Tetrahydrothiopyran-3-yl | - | 4,17 (t, 2H), 7,10-7,40 (m, 5H) |
| 6.07 | Ethyl | Tetrahydropyran-3-yl | 4-F | 3,90 (m, 2H), 4,17 (t, 2H), 7,00 (t, 2H), 7,33 (m, 2H) |
| 6.08 | Propyl | Tetrahydropyran-3-yl | 4-F | 3,90 (m, 2H), 4,17 (t, 2H), 7,00 (t, 2H), 7,33 (m, 2H) |
| 6.09 | Ethyl | Tetrahydropyran-4-yl | 4-F | 4,00 (m, 2H), 4,17 (t, 2H), 7,00 (t, 2H), 7,33 (m, 2H) |
| 6.10 | Propyl | Tetrahydropyran-4-yl | 4-F | 4,00 (m, 2H), 4,17 (t, 2H), 7,00 (t, 2H), 7,33 (m, 2H) |
| 6.11 | Ethyl | Tetrahydrothiopyran-3-yl | 4-F | 4,17 (t, 2H), 7,00 (t, 2H), 7,33 (m, 2H) |
| 6.12 | Propyl | Tetrahydrothiopyran-3-yl | 4-F | 4,17 (t, 2H), 7,00 (t, 2H), 7,33 (m, 2H) |
| 6.13 | Ethyl | Tetrahydropyran-3-yl | 4-Cl | 3,90 (m, 2H), 4,17 (t, 2H), 7,27 (s, 4H) |

Tabelle 6 (Fortsetzung)

| Nr. | $R^d$ | $R^f$ | Reste am Phenylring | phys. Daten / $^1$H-NMR [δ in ppm], Fp. [°C] |
|---|---|---|---|---|
| 6.14 | Propyl | Tetrahydropyran-3-yl | 4-Cl | 3,90 (m, 2H), 4,17 (t, 2H), 7,27 (s, 4H) |
| 6.15 | Ethyl | Tetrahydropyran-4-yl | 4-Cl | 4,00 (m, 2H), 4,17 (t, 2H), 7,27 (s, 4H) |
| 6.16 | Propyl | Tetrahydropyran-4-yl | 4-Cl | 4,00 (m, 2H), 4,17 (t, 2H), 7,27 (s, 4H) |
| 6.17 | Ethyl | Tetrahydrothiopyran-3-yl | 4-Cl | 4,17 (t, 2H), 7,27 (s, 4H) |
| 6.18 | Propyl | Tetrahydrothiopyran-3-yl | 4-Cl | 4,17 (t, 2H), 7,27 (s, 4H) |
| 6.19 | Ethyl | Tetrahydropyran-3-yl | 2-Cl | 3,90 (m, 2H), 4,20 (t, 2H), 7,07-7,40 (m, 4H) |
| 6.20 | Propyl | Tetrahydropyran-3-yl | 2-Cl | 3,90 (m, 2H), 4,20 (t, 2H), 7,07-7,40 (m, 4H) |
| 6.21 | Ethyl | Tetrahydropyran-4-yl | 2-Cl | 4,00 (m, 2H), 4,20 (t, 2H), 7,07-7,40 (m, 4H) |
| 6.22 | Propyl | Tetrahydropyran-4-yl | 2-Cl | 4,00 (m, 2H), 4,20 (t, 2H), 7,07-7,40 (m, 4H) |
| 6.23 | Ethyl | Tetrahydrothiopyran-3-yl | 2-Cl | 4,20 (t, 2H), 7,07-7,40 (m, 4H) |
| 6.24 | Propyl | Tetrahydrothiopyran-3-yl | 2-Cl | 4,20 (t, 2H), 7,07-7,40 (m, 4H) |
| 6.25 | Ethyl | Tetrahydropyran-3-yl | 3-Cl | 3,90 (m, 2H), 4,20 (t, 2H), 7,17 (m, 3H) 7,30 (m, 1H) |
| 6.26 | Propyl | Tetrahydropyran-3-yl | 3-Cl | 3,90 (m, 2H), 4,20 (t, 2H), 7,17 (m, 3H) 7,30 (m, 1H) |
| 6.27 | Ethyl | Tetrahydropyran-4-yl | 3-Cl | 4,00 (m, 2H), 4,20 (t, 2H), 7,17 (m, 3H) 7,30 (m, 1H) |
| 6.28 | Propyl | Tetrahydropyran-4-yl | 3-Cl | 4,00 (m, 2H), 4,20 (t, 2H), 7,17 (m, 3H) 7,30 (m, 1H) |
| 6.29 | Ethyl | Tetrahydrothiopyran-3-yl | 3-Cl | 4,20 (t, 2H), 7,17 (m, 3H), 7,30 (m, 1H) |
| 6.30 | Propyl | Tetrahydrothiopyran-3-yl | 3-Cl | 4,20 (t, 2H), 7,17 (m, 3H), 7,30 (m, 1H) |

Tabelle 6 (Fortsetzung)

| Nr. | $R^d$ | $R^f$ | Reste am Phenylring | phys. Daten / $^1H$-NMR [δ in ppm], Fp. [°C] |
|---|---|---|---|---|
| 6.31 | Ethyl | Tetrahydropyran-3-yl | 2,5-$Cl_2$ | 3,90 (m,2H), 4,20 (t,2H), 7,07 (dd,1H), 7,20 (d,1H) 7,30 (d,1H) |
| 6.32 | Propyl | Tetrahydropyran-3-yl | 2,5-$Cl_2$ | 3,90 (m,2H), 4,20 (t,2H), 7,07 (dd,1H), 7,20 (d,1H) 7,30 (d,1H) |
| 6.33 | Ethyl | Tetrahydropyran-4-yl | 2,5-$Cl_2$ | 4,00 (m,2H), 4,20 (t,2H), 7,07 (dd,1H), 7,20 (d,1H) 7,30 (d,1H) |
| 6.34 | Propyl | Tetrahydropyran-4-yl | 2,5-$Cl_2$ | 4,00 (m,2H), 4,20 (t,2H), 7,07 (dd,1H), 7,20 (d,1H) 7,30 (d,1H) |
| 6.35 | Ethyl | Tetrahydrothiopyran-3-yl | 2,5-$Cl_2$ | 4,20 (t,2H), 7,07 (dd,1H), 7,20 (d,1H) 7,30 (d,1H) |
| 6.36 | Propyl | Tetrahydrothiopyran-3-yl | 2,5-$Cl_2$ | 4,20 (t,2H), 7,07 (dd,1H), 7,20 (d,1H) 7,30 (d,1H) |
| 6.37 | Ethyl | Tetrahydropyran-3-yl | 2,6-$Cl_2$ | 3,90 (m,2H), 4,20 (t,2H), 7,20 (t,1H) 7,40 (d,2H) |
| 6.38 | Propyl | Tetrahydropyran-3-yl | 2,6-$Cl_2$ | 3,90 (m,2H), 4,20 (t,2H), 7,20 (t,1H) 7,40 (d,2H) |
| 6.39 | Ethyl | Tetrahydropyran-4-yl | 2,6-$Cl_2$ | 4,00 (m,2H), 4,20 (t,2H), 7,20 (t,1H) 7,40 (d,2H) |
| 6.40 | Propyl | Tetrahydropyran-4-yl | 2,6-$Cl_2$ | 4,00 (m,2H), 4,20 (t,2H), 7,20 (t,1H) 7,40 (d,2H) |
| 6.41 | Ethyl | Tetrahydrothiopyran-3-yl | 2,6-$Cl_2$ | 4,20 (t,2H), 7,20 (t,1H), 7,40 (d,2H) |
| 6.42 | Propyl | Tetrahydrothiopyran-3-yl | 2,6-$Cl_2$ | 4,20 (t,2H), 7,20 (t,1H), 7,40 (d,2H) |

Tabelle 7

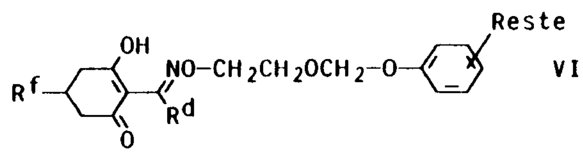 VI ($R^e$ = $-CH_2CH_2-O-CH_2-$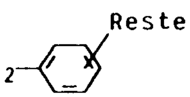 ; $R^g$, $R^h$, $R^i$ = H)

| Nr. | $R^d$ | $R^f$ | Reste am Phenylring | phys. Daten / $^1$H-NMR [δ in ppm], Fp. [°C] |
|---|---|---|---|---|
| 7.01 | Ethyl | Tetrahydropyran-3-yl | - | 3,90 (m, 2H), 4,25 (t, 2H), 4,58 (s, 2H), 7,38 (s, 5H) |
| 7.02 | Propyl | Tetrahydropyran-3-yl | - | 3,90 (m, 2H), 4,25 (t, 2H), 4,58 (s, 2H), 7,38 (s, 5H) |
| 7.03 | Ethyl | Tetrahydropyran-4-yl | - | 4,03 (m, 2H), 4,33 (m, 2H), 4,60 (s, 2H), 7,40 (s, 5H) |
| 7.04 | Propyl | Tetrahydropyran-4-yl | - | 4,03 (m, 2H), 4,33 (m, 2H), 4,60 (s, 2H), 7,40 (s, 5H) |
| 7.05 | Ethyl | Tetrahydrothiopyran-3-yl | - | 4,27 (m, 2H), 4,57 (s, 2H), 7,35 (s, 5H) |
| 7.06 | Propyl | Tetrahydrothiopyran-3-yl | - | 4,27 (m, 2H), 4,57 (s, 2H), 7,35 (s, 5H) |
| 7.07 | Ethyl | Tetrahydropyran-3-yl | 2-F | 3,93 (m, 2H), 4,27 (m, 2H), 4,67 (s, 2H), 6,93-7,50 (m, 4H) |

Tabelle 7 (Fortsetzung)

| Nr. | $R^d$ | $R^f$ | Reste am Phenylring | phys. Daten / $^1$H-NMR [δ in ppm], Fp. [°C] |
|---|---|---|---|---|
| 7.08 | Propyl | Tetrahydropyran-3-yl | 2-F | 3,93 (m, 2H), 4,27 (m, 2H), 4,67 (s, 2H), 6,93-7,50 (m, 4H) |
| 7.09 | Ethyl | Tetrahydropyran-4-yl | 2-F | 4,03 (m, 2H), 4,27 (m, 2H), 4,63 (s, 2H), 6,97-7,50 (m, 4H) |
| 7.10 | Propyl | Tetrahydropyran-4-yl | 2-F | 4,03 (m, 2H), 4,27 (m, 2H), 4,63 (s, 2H), 6,97-7,50 (m, 4H) |
| 7.11 | Ethyl | Tetrahydrothiopyran-3-yl | 2-F | 4,27 (m, 2H), 4,67 (s, 2H), 6,97-7,50 (m, 4H) |
| 7.12 | Propyl | Tetrahydrothiopyran-3-yl | 2-F | 4,27 (m, 2H), 4,67 (s, 2H), 6,97-7,50 (m, 4H) |
| 7.13 | Ethyl | Tetrahydropyran-3-yl | 3-F | 3,93 (m, 2H), 4,27 (m, 2H), 4,57 (s, 2H), 6,90-7,15 (m, 3H), 7,23-7,40 (m, 1H) |
| 7.14 | Propyl | Tetrahydropyran-3-yl | 3-F | 3,93 (m, 2H), 4,27 (m, 2H), 4,57 (s, 2H), 6,90-7,15 (m, 3H), 7,23-7,40 (m, 1H) |
| 7.15 | Ethyl | Tetrahydropyran-4-yl | 3-F | 4,03 (m, 2H), 4,25 (m, 2H), 4,60 (s, 2H), 6,90-7,18 (m, 3H), 7,26-7,40 (m, 1H) |
| 7.16 | Propyl | Tetrahydropyran-4-yl | 3-F | 4,03 (m, 2H), 4,25 (m, 2H), 4,60 (s, 2H), 6,90-7,18 (m, 3H), 7,26-7,40 (m, 1H) |
| 7.17 | Ethyl | Tetrahydrothiopyran-3-yl | 3-F | 4,27 (m, 2H), 4,60 (s, 2H), 6,90-7,15 (m, 3H), 7,23-7,40 (m, 1H) |

Tabelle 7 (Fortsetzung)

| Nr. | $R^d$ | $R^f$ | Reste am Phenylring | phys. Daten / $^1H$-NMR [δ in ppm], Fp. [°C] |
|---|---|---|---|---|
| 7.18 | Propyl | Tetrahydrothiopyran-3-yl | 3-F | 4,27 (m, 2H), 4,60 (s, 2H), 6,90-7,15 (m, 3H), 7,23-7,40 (m, 1H) |
| 7.19 | Ethyl | Tetrahydropyran-3-yl | 4-F | 3,93 (m, 2H), 4,23 (m, 2H), 4,53 (s, 2H), 7,00 (m, 2H), 7,30 (m, 2H) |
| 7.20 | Propyl | Tetrahydropyran-3-yl | 4-F | 3,93 (m, 2H), 4,23 (m, 2H), 4,53 (s, 2H), 7,00 (m, 2H), 7,30 (m, 2H) |
| 7.21 | Ethyl | Tetrahydropyran-4-yl | 4-F | 92 |
| 7.22 | Propyl | Tetrahydropyran-4-yl | 4-F | 4,00 (m, 2H), 4,23 (m, 2H), 4,53 (s, 2H), 7,03 (m, 2H), 7,30 (m, 2H) |
| 7.23 | Ethyl | Tetrahydrothiopyran-3-yl | 4-F | 4,27 (m, 2H), 4,53 (s, 2H), 7,03 (m, 2H), 7,30 (m, 2H) |
| 7.24 | Propyl | Tetrahydrothiopyran-3-yl | 4-F | 4,27 (m, 2H), 4,53 (s, 2H), 7,03 (m, 2H), 7,30 (m, 2H) |
| 7.25 | Ethyl | Tetrahydropyran-3-yl | 2-Cl | |
| 7.26 | Propyl | Tetrahydropyran-3-yl | 2-Cl | |
| 7.27 | Ethyl | Tetrahydropyran-4-yl | 2-Cl | |
| 7.28 | Propyl | Tetrahydropyran-4-yl | 2-Cl | |
| 7.29 | Ethyl | Tetrahydrothiopyran-3-yl | 2-Cl | |
| 7.30 | Propyl | Tetrahydrothiopyran-3-yl | 2-Cl | |
| 7.31 | Ethyl | Tetrahydropyran-3-yl | 3-Cl | |
| 7.32 | Propyl | Tetrahydropyran-3-yl | 3-Cl | |

Tabelle 7 (Fortsetzung)

| Nr. | $R^d$ | $R^f$ | Reste am Phenylring | phys. Daten / $^1H$-NMR [δ in ppm], Fp. [°C] |
|---|---|---|---|---|
| 7.33 | Ethyl | Tetrahydropyran-4-yl | 3-Cl | |
| 7.34 | Propyl | Tetrahydropyran-4-yl | 3-Cl | |
| 7.35 | Ethyl | Tetrahydrothiopyran-3-yl | 3-Cl | |
| 7.36 | Propyl | Tetrahydrothiopyran-3-yl | 3-Cl | |
| 7.37 | Ethyl | Tetrahydropyran-3-yl | 4-Cl | 3,93 (m, 2H), 4,27 (m, 2H), 4,53 (s, 2H), 7,28 (m, 4H) |
| 7.38 | Propyl | Tetrahydropyran-3-yl | 4-Cl | 3,93 (m, 2H), 4,27 (m, 2H), 4,53 (s, 2H), 7,28 (m, 4H) |
| 7.39 | Ethyl | Tetrahydropyran-4-yl | 4-Cl | 67- 72 |
| 7.40 | Propyl | Tetrahydropyran-4-yl | 4-Cl | 4,00 (m, 2H), 4,23 (m, 2H), 4,53 (s, 2H), 7,28 (m, 4H) |
| 7.41 | Ethyl | Tetrahydrothiopyran-3-yl | 4-Cl | 4,27 (m, 2H), 4,53 (s, 2H), 7,28 (m, 4H) |
| 7.42 | Propyl | Tetrahydrothiopyran-3-yl | 4-Cl | 4,27 (m, 2H), 4,53 (s, 2H), 7,28 (m, 4H) |
| 7.43 | Ethyl | Tetrahydropyran-3-yl | 2-$CH_3$ | 3,93 (m, 2H), 4,23 (m, 2H), 4,57 (s, 2H), 7,09-7,33 (m, 4H) |
| 7.44 | Propyl | Tetrahydropyran-3-yl | 2-$CH_3$ | 3,93 (m, 2H), 4,23 (m, 2H), 4,57 (s, 2H), 7,09-7,33 (m, 4H) |
| 7.45 | Ethyl | Tetrahydropyran-4-yl | 2-$CH_3$ | 4,00 (m, 2H), 4,23 (m, 2H), 4,57 (s, 2H), 7,09-7,33 (m, 4H) |
| 7.46 | Propyl | Tetrahydropyran-4-yl | 2-$CH_3$ | 4,00 (m, 2H), 4,23 (m, 2H), 4,57 (s, 2H), 7,09-7,33 (m, 4H) |

Tabelle 7 (Fortsetzung)

| Nr. | $R^d$ | $R^f$ | Reste am Phenylring | phys. Daten / $^1H$-NMR [$\delta$ in ppm], Fp. [°C] |
|---|---|---|---|---|
| 7.47 | Ethyl | Tetrahydrothiopyran-3-yl | 2-$CH_3$ | 4,23 (m, 2H), 4,57 (s, 2H), 7,09-7,33 (m, 4H) |
| 7.48 | Propyl | Tetrahydrothiopyran-3-yl | 2-$CH_3$ | 4,23 (m, 2H), 4,57 (s, 2H), 7,09-7,33 (m, 4H) |
| 7.49 | Ethyl | Tetrahydropyran-3-yl | 3-$CH_3$ | 3,93 (m, 2H), 4,25 (m, 2H), 4,57 (s, 2H), 7,00-7,32 (m, 4H) |
| 7.50 | Propyl | Tetrahydropyran-3-yl | 3-$CH_3$ | 3,93 (m, 2H), 4,25 (m, 2H), 4,57 (s, 2H), 7,00-7,32 (m, 4H) |
| 7.51 | Ethyl | Tetrahydropyran-4-yl | 3-$CH_3$ | 4,00 (m, 2H), 4,27 (m, 2H), 4,57 (s, 2H), 7,00-7,32 (m, 4H) |
| 7.52 | Propyl | Tetrahydropyran-4-yl | 3-$CH_3$ | 4,00 (m, 2H), 4,27 (m, 2H), 4,57 (s, 2H), 7,00-7,32 (m, 4H) |
| 7.53 | Ethyl | Tetrahydrothiopyran-3-yl | 3-$CH_3$ | 4,27 (m, 2H), 4,60 (s, 2H), 7,00-7,32 (m, 4H) |
| 7.54 | Propyl | Tetrahydrothiopyran-3-yl | 3-$CH_3$ | 4,27 (m, 2H), 4,60 (s, 2H), 7,00-7,32 (m, 4H) |
| 7.55 | Ethyl | Tetrahydropyran-3-yl | 4-$CH_3$ | 3,93 (m, 2H), 4,20 (m, 2H), 4,53 (s, 2H), 7,07-7,30 (m, 4H) |
| 7.56 | Propyl | Tetrahydropyran-3-yl | 4-$CH_3$ | 3,93 (m, 2H), 4,20 (m, 2H), 4,53 (s, 2H), 7,07-7,30 (m, 4H) |

Tabelle 7 (Fortsetzung)

| Nr. | $R^d$ | $R^f$ | Reste am Phenylring | phys. Daten / $^1H$-NMR [$\delta$ in ppm], Fp. [°C] |
|---|---|---|---|---|
| 7.57 | Ethyl | Tetrahydropyran-4-yl | 4-$CH_3$ | 4,00 (m,2H), 4,23 (m,2H), 4,57 (s,2H), 7,03-7,27 (m,4H) |
| 7.58 | Propyl | Tetrahydropyran-4-yl | 4-$CH_3$ | 4,00 (m,2H), 4,23 (m,2H), 4,57 (s,2H), 7,03-7,27 (m,4H) |
| 7.59 | Ethyl | Tetrahydrothiopyran-3-yl | 4-$CH_3$ | 4,23 (m,2H), 4,57 (s,2H), 7,07-7,30 (m,4H) |
| 7.60 | Propyl | Tetrahydrothiopyran-3-yl | 4-$CH_3$ | 4,28 (m,2H), 4,57 (s,2H), 7,07-7,30 (m,4H) |
| 7.61 | Ethyl | Tetrahydropyran-3-yl | 4-tert.-$C_4H_9$ | 3,93 (m,2H), 4,23 (m,2H), 4,53 (s,2H), 7,20-7,40 (m,4H) |
| 7.62 | Propyl | Tetrahydropyran-3-yl | 4-tert.-$C_4H_9$ | 3,93 (m,2H), 4,23 (m,2H), 4,53 (s,2H), 7,20-7,40 (m,4H) |
| 7.63 | Ethyl | Tetrahydropyran-4-yl | 4-tert.-$C_4H_9$ | 4,00 (m,2H), 4,23 (m,2H), 4,53 (s,2H), 7,20-7,40 (m,4H) |
| 7.64 | Propyl | Tetrahydropyran-4-yl | 4-tert.-$C_4H_9$ | 4,00 (m,2H), 4,23 (m,2H), 4,53 (s,2H), 7,20-7,40 (m,4H) |
| 7.65 | Ethyl | Tetrahydrothiopyran-3-yl | 4-tert.-$C_4H_9$ | 4,23 (m,2H), 4,53 (s,2H), 7,20-7,40 (m,4H) |
| 7.66 | Propyl | Tetrahydrothiopyran-3-yl | 4-tert.-$C_4H_9$ | 4,23 (m,2H), 4,53 (s,2H), 7,20-7,40 (m,4H) |

Tabelle 8

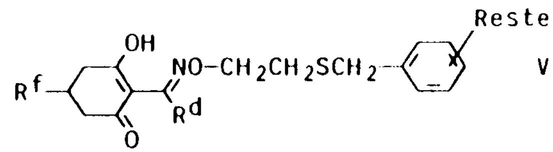 VI  ($R^e$ = $-CH_2CH_2-S-CH_2-$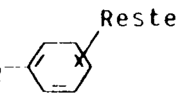 ; $R^g$, $R^h$, $R^i$ = H)

| Nr. | $R^d$ | $R^f$ | Reste am Phenylring | phys. Daten / $^1$H-NMR [δ in ppm], Fp. [°C] |
|---|---|---|---|---|
| 8.01 | Ethyl | Tetrahydropyran-3-yl | - | 3,73 (s,2H), 3,90 (m,2H), 4,17 (t,2H), 7,28 (s,5H) |
| 8.02 | Propyl | Tetrahydropyran-3-yl | - | 3,73 (s,2H), 3,90 (m,2H), 4,17 (t,2H), 7,28 (s,5H) |
| 8.03 | Ethyl | Tetrahydropyran-4-yl | - | 3,77 (s,2H), 4,00 (m,2H), 4,13 (t,2H), 7,28 (s,5H) |
| 8.04 | Propyl | Tetrahydropyran-4-yl | - | 3,77 (s,2H), 4,00 (m,2H), 4,13 (t,2H), 7,28 (s,5H) |
| 8.05 | Ethyl | Tetrahydrothiopyran-3-yl | - | 3,80 (s,2H), 4,13 (t,2H), 7,28 (s,5H) |
| 8.06 | Propyl | Tetrahydrothiopyran-3-yl | - | 3,80 (s,2H), 4,13 (t,2H), 7,28 (s,5H) |
| 8.07 | Ethyl | Tetrahydropyran-3-yl | 4-F | 3,72 (s,2H), 3,90 (m,2H), 4,13 (t,2H), 7,00 (m,2H), 7,30 (m,2H) |

Tabelle 8 (Fortsetzung)

| Nr. | $R^d$ | $R^f$ | Reste am Phenylring | phys. Daten / $^1$H-NMR [δ in ppm], Fp. [°C] |
|---|---|---|---|---|
| 8.08 | Propyl | Tetrahydropyran-3-yl | 4-F | 3,72 (s,2H), 3,90 (m,2H), 4,13 (t,2H), 7,00 (m,2H), 7,30 (m,2H) |
| 8.09 | Ethyl | Tetrahydropyran-4-yl | 4-F | 63- 65 |
| 8.10 | Propyl | Tetrahydropyran-4-yl | 4-F | 3,73 (s,2H), 4,00 (m,2H), 4,13 (t,2H), 7,00 (m,2H), 7,30 (m,2H) |
| 8.11 | Ethyl | Tetrahydrothiopyran-3-yl | 4-F | 3,75 (s,2H), 4,13 (t,2H), 7,00 (m,2H), 7,30 (m,2H) |
| 8.12 | Propyl | Tetrahydrothiopyran-3-yl | 4-F | 3,75 (s,2H), 4,13 (t,2H), 7,00 (m,2H), 7,30 (m,2H) |
| 8.13 | Ethyl | Tetrahydropyran-3-yl | 4-Cl | 3,77 (s,2H), 3,93 (m,2H), 4,13 (t,2H), 7,30 (s,4H) |
| 8.14 | Propyl | Tetrahydropyran-3-yl | 4-Cl | 3,77 (s,2H), 3,93 (m,2H), 4,13 (t,2H), 7,30 (s,4H) |
| 8.15 | Ethyl | Tetrahydropyran-4-yl | 4-Cl | 3,73 (s,2H), 4,00 (m,2H), 4,17 (t,2H), 7,30 (s,4H) |
| 8.16 | Propyl | Tetrahydropyran-4-yl | 4-Cl | 3,73 (s,2H), 4,00 (m,2H), 4,17 (t,2H), 7,30 (s,4H) |
| 8.17 | Ethyl | Tetrahydrothiopyran-3-yl | 4-Cl | 3,73 (s,2H), 4,13 (m,2H), 7,30 (s,4H) |
| 8.18 | Propyl | Tetrahydrothiopyran-3-yl | 4-Cl | 3,73 (s,2H), 4,13 (m,2H), 7,30 (s,4H) |

Tabelle 9

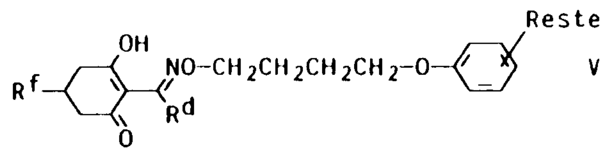 VI ($R^e$ = $-CH_2CH_2CH_2CH_2-O-$ 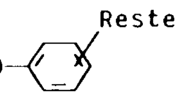 ; $R^g$, $R^h$, $R^i$ = H)

| Nr. | $R^d$ | $R^f$ | Reste am Phenylring | phys. Daten / $^1$H-NMR [δ in ppm], Fp. [°C] |
|---|---|---|---|---|
| 9.01 | Ethyl | Tetrahydropyran-3-yl | - | 3,70-4,20 (m,6H), 6,90 (m,3H), 7,30 (m,2H) |
| 9.02 | Propyl | Tetrahydropyran-3-yl | - | 3,70-4,20 (m,6H), 6,90 (m,3H), 7,30 (m,2H) |
| 9.03 | Ethyl | Tetrahydropyran-4-yl | - | 3,83-4,23 (m,6H), 6,90 (m,3H), 7,30 (m,2H) |
| 9.04 | Propyl | Tetrahydropyran-4-yl | - | 3,83-4,23 (m,6H), 6,90 (m,3H), 7,30 (m,2H) |
| 9.05 | Ethyl | Tetrahydrothiopyran-3-yl | - | 4,00 (bs,2H), 4,13 (bs,2H), 6,90 (m,3H) 7,30 (m,2H) |
| 9.06 | Propyl | Tetrahydrothiopyran-3-yl | - | 4,00 (bs,2H), 4,13 (bs,2H), 6,90 (m,3H) 7,30 (m,2H) |
| 9.07 | Ethyl | Tetrahydropyran-3-yl | 2-F | 3,93 (m,2H), 4,00-4,20 (m,4H), 6,80-7,15 (m,4H) |
| 9.08 | Propyl | Tetrahydropyran-3-yl | 2-F | 3,93 (m,2H), 4,00-4,20 (m,4H), 6,80-7,15 (m,4H) |
| 9.09 | Ethyl | Tetrahydropyran-4-yl | 2-F | 68- 72 |

Tabelle 9 (Fortsetzung)

| Nr. | $R^d$ | $R^f$ | Reste am Phenylring | phys. Daten / $^1H$-NMR [δ in ppm], Fp. [°C] |
|---|---|---|---|---|
| 9.10 | Propyl | Tetrahydropyran-4-yl | 2-F | 3,90-4,20 (m,6H), 6,80-7,15 (m,4H) |
| 9.11 | Ethyl | Tetrahydrothiopyran-3-yl | 2-F | 4,00-4,20 (m,4H), 6,80-7,15 (m,4H) |
| 9.12 | Propyl | Tetrahydrothiopyran-3-yl | 2-F | 4,00-4,20 (m,4H), 6,80-7,15 (m,4H) |
| 9.13 | Ethyl | Tetrahydropyran-3-yl | 3-F | |
| 9.14 | Propyl | Tetrahydropyran-3-yl | 3-F | |
| 9.15 | Ethyl | Tetrahydropyran-4-yl | 3-F | |
| 9.16 | Propyl | Tetrahydropyran-4-yl | 3-F | |
| 9.17 | Ethyl | Tetrahydrothiopyran-3-yl | 3-F | |
| 9.18 | Propyl | Tetrahydrothiopyran-3-yl | 3-F | |
| 9.19 | Ethyl | Tetrahydropyran-3-yl | 4-F | 3,80-4,20 (m,6H), 6,75-7,05 (m,4H) |
| 9.20 | Propyl | Tetrahydropyran-3-yl | 4-F | 3,80-4,20 (m,6H), 6,75-7,05 (m,4H) |
| 9.21 | Ethyl | Tetrahydropyran-4-yl | 4-F | 3,90-4,20 (m,6H), 6,75-7,05 (m,4H) |
| 9.22 | Propyl | Tetrahydropyran-4-yl | 4-F | 3,90-4,20 (m,6H), 6,75-7,05 (m,4H) |
| 9.23 | Ethyl | Tetrahydrothiopyran-3-yl | 4-F | 3,90-4,20 (m,4H), 6,75-7,05 (m,4H) |
| 9.24 | Propyl | Tetrahydrothiopyran-3-yl | 4-F | 3,90-4,20 (m,4H), 6,75-7,05 (m,4H) |
| 9.25 | Ethyl | Tetrahydropyran-3-yl | 4-Cl | 3,80-4,20 (m,6H), 6,80 (m,2H), 7,20 (m,2H) |
| 9.26 | Propyl | Tetrahydropyran-3-yl | 4-Cl | 3,80-4,20 (m,6H), 6,80 (m,2H), 7,20 (m,2H) |
| 9.27 | Ethyl | Tetrahydropyran-4-yl | 4-Cl | 3,90-4,20 (m,6H), 6,80 (m,2H), 7,20 (m,2H) |

Tabelle 9 (Fortsetzung)

| Nr. | $R^d$ | $R^f$ | Reste am Phenylring | phys. Daten / $^1H$-NMR [δ in ppm], Fp. [°C] |
|---|---|---|---|---|
| 9.28 | Propyl | Tetrahydropyran-4-yl | 4-Cl | 3,90-4,20 (m,6H), 6,80 (m,2H), 7,20 (m,2H) |
| 9.29 | Ethyl | Tetrahydrothiopyran-3-yl | 4-Cl | 3,90-4,20 (m,4H), 6,80 (m,2H), 7,20 (m,2H) |
| 9.30 | Propyl | Tetrahydrothiopyran-3-yl | 4-Cl | 3,90-4,20 (m,4H), 6,80 (m,2H), 7,20 (m,2H) |
| 9.31 | Ethyl | Tetrahydropyran-3-yl | 2,6-$Cl_2$ | 3,93 (m,2H), 4,00-4,25 (m,4H), 7,00 (t,1H), 7,30 (d,2H) |
| 9.32 | Propl | Tetrahydropyran-3-yl | 2,6-$Cl_2$ | 3,93 (m,2H), 4,00-4,25 (m,4H), 7,00 (t,1H), 7,30 (d,2H) |
| 9.33 | Ethyl | Tetrahydropyran-4-yl | 2,6-$Cl_2$ | 3,90-4,25 (m,6H), 7,00 (t,1H), 7,30 (d,2H) |
| 9.34 | Propyl | Tetrahydropyran-4-yl | 2,6-$Cl_2$ | 3,90-4,25 (m,6H), 7,00 (t,1H), 7,30 (d,2H) |
| 9.35 | Ethyl | Tetrahydrothiopyran-3-yl | 2,6-$Cl_2$ | 4,00-4,20 (m,4H), 7,00 (t,1H), 7,30 (d,2H) |
| 9.36 | Propyl | Tetrahydrothiopyran-3-yl | 2,6-$Cl_2$ | 4,00-4,20 (m,4H), 7,00 (t,1H), 7,30 (d,2H) |

Tabelle 10

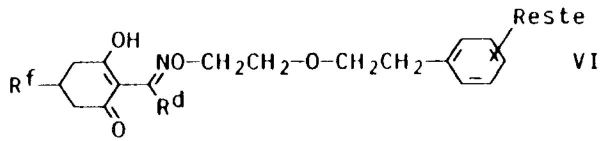 VI (R$^e$ = $-CH_2CH_2-O-CH_2CH_2-$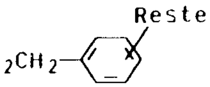 ; R$^g$, R$^h$, R$^i$ = H)

| Nr. | R$^d$ | R$^f$ | Reste am Phenylring | phys. Daten / $^1$H-NMR [δ in ppm], Fp. [°C] |
|---|---|---|---|---|
| 10.01 | Ethyl | Tetrahydropyran-3-yl | - | 3,90 (m, 2H), 4,20 (m, 2H), 7,25 (m, 5H) |
| 10.02 | Propyl | Tetrahydropyran-3-yl | - | 3,90 (m, 2H), 4,20 (m, 2H), 7,25 (m, 5H) |
| 10.03 | Ethyl | Tetrahydropyran-4-yl | - | |
| 10.04 | Propyl | Tetrahydropyran-4-yl | - | |
| 10.05 | Ethyl | Tetrahydrothiopyran-3-yl | - | 4,20 (m, 2H), 7,25 (m, 5H) |
| 10.06 | Propyl | Tetrahydrothiopyran-3-yl | - | 4,20 (m, 2H), 7,25 (m, 5H) |
| 10.07 | Ethyl | Tetrahydropyran-3-yl | 4-F | 3,90 (m, 2H), 4,17 (m, 2H), 6,93 (m, 2H), 7,13 (m, 2H) |
| 10.08 | Propyl | Tetrahydropyran-3-yl | 4-F | 3,90 (m, 2H), 4,17 (m, 2H), 6,93 (m, 2H), 7,13 (m, 2H) |
| 10.09 | Ethyl | Tetrahydropyran-4-yl | 4-F | |
| 10.10 | Propyl | Tetrahydropyran-4-yl | 4-F | |
| 10.11 | Ethyl | Tetrahydrothiopyran-3-yl | 4-F | 4,17 (m, 2H), 6,93 (m, 2H), 7,13 (m, 2H) |
| 10.12 | Propyl | Tetrahydrothiopyran-3-yl | 4-F | 4,17 (m, 2H), 6,93 (m, 2H), 7,13 (m, 2H) |
| 10.13 | Ethyl | Tetrahydropyran-3-yl | 4-Cl | 3,90 (m, 2H), 4,17 (m, 2H), 7,13 (m, 4H) |

Tabelle 10 (Fortsetzung)

| Nr. | $R^d$ | $R^f$ | Reste am Phenylring | phys. Daten / $^1H$-NMR [$\delta$ in ppm], Fp. [°C] |
|---|---|---|---|---|
| 10.14 | Propyl | Tetrahydropyran-3-yl | 4-Cl | 3,90 (m, 2H), 4,17 (m, 2H), 7,13 (m, 4H) |
| 10.15 | Ethyl | Tetrahydropyran-4-yl | 4-Cl | |
| 10.16 | Propyl | Tetrahydropyran-4-yl | 4-Cl | |
| 10.17 | Ethyl | Tetrahydrothiopyran-3-yl | 4-Cl | 4,17 (m, 2H), 7,13 (m, 4H) |
| 10.18 | Propyl | Tetrahydrothiopyran-3-yl | 4-Cl | 4,17 (m, 2H), 7,13 (m, 4H) |

Tabelle 11

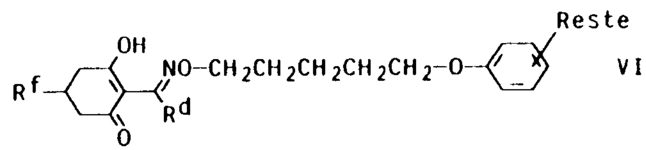 VI (R^e = 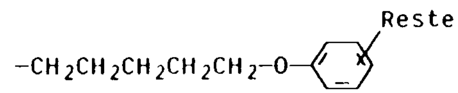 ; $R^g$, $R^h$, $R^i$ = H)

| Nr. | $R^d$ | $R^f$ | Reste am Phenylring | phys. Daten / $^1$H-NMR [δ in ppm], Fp. [°C] |
|---|---|---|---|---|
| 11.01 | Ethyl | Tetrahydropyran-3-yl | - | 3,80-4,17 (m,6H), 6,90 (m,3H), 7,27 (m,2H) |
| 11.02 | Propyl | Tetrahydropyran-3-yl | - | 3,80-4,17 (m,6H), 6,90 (m,3H), 7,27 (m,2H) |
| 11.03 | Ethyl | Tetrahydropyran-4-yl | - | 3,90-4,17 (m,6H), 6,90 (m,3H), 7,27 (m,2H) |
| 11.04 | Propyl | Tetrahydropyran-4-yl | - | 3,90-4,17 (m,6H), 6,90 (m,3H), 7,27 (m,2H) |
| 11.05 | Ethyl | Tetrahydrothiopyran-3-yl | - | 3,97 (t,2H), 4,07 (t,2H), 6,90 (m,3H), 7,27 (m,2H) |
| 11.06 | Propyl | Tetrahydrothiopyran-3-yl | - | 3,97 (t,2H), 4,07 (t,2H), 6,90 (m,3H), 7,27 (m,2H) |
| 11.07 | Ethyl | Tetrahydropyran-3-yl | 4-F | 3,90 (m,4H), 4,03 (t,2H), 6,70-7,03 (m,4H) |
| 11.08 | Propyl | Tetrahydropyran-3-yl | 4-F | 3,90 (m,4H), 4,03 (t,2H), 6,70-7,03 (m,4H) |
| 11.09 | Ethyl | Tetrahydropyran-4-yl | 4-F | 3,83-4,13 (m,6H), 6,70-7,03 (m,4H) |
| 11.10 | Propyl | Tetrahydropyran-4-yl | 4-F | 3,83-4,13 (m,6H), 6,70-7,03 (m,4H) |
| 11.11 | Ethyl | Tetrahydrothiopyran-3-yl | 4-F | 3,90 (t,2H), 4,03 (t,2H) 6,70-7,03 (m,4H) |
| 11.12 | Propyl | Tetrahydrothiopyran-3-yl | 4-F | 3,90 (t,2H), 4,03 (t,2H) 6,70-7,03 (m,4H) |
| 11.13 | Ethyl | Tetrahydropyran-3-yl | 4-Cl | 3,80-4,10 (m,6H), 6,80 (d,2H), 7,20 (d,2H) |
| 11.14 | Propyl | Tetrahydropyran-3-yl | 4-Cl | 3,80-4,10 (m,6H), 6,80 (d,2H), 7,20 (d,2H) |

Tabelle 11 (Fortsetzung)

| Nr. | $R^d$ | $R^f$ | Reste am Phenylring | phys. Daten / $^1H$-NMR [$\delta$ in ppm], Fp. [°C] |
|---|---|---|---|---|
| 11.15 | Ethyl | Tetrahydropyran-4-yl | 4-Cl | 3,87-4,10 (m,6H), 6,80 (d,2H), 7,20 (d,2H) |
| 11.16 | Propyl | Tetrahydropyran-4-yl | 4-Cl | 3,87-4,10 (m,6H), 6,80 (d,2H), 7,20 (d,2H) |
| 11.17 | Ethyl | Tetrahydrothiopyran-3-yl | 4-Cl | 54- 61 |
| 11.18 | Propyl | Tetrahydrothiopyran-3-yl | 4-Cl | 3,90 (t,2H), 4,07 (t,2H), 6,80 (d,2H) 7,20 (d,2H) |

Tabelle 12

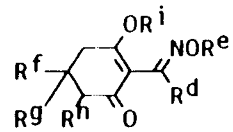

VI

| Nr. | $R^d$ | $R^e$ | $R^f$ | $R^g$ | $R^h$ | $R^i$ | Literatur |
|---|---|---|---|---|---|---|---|
| 12.1 | $C_3H_7$ | $CH_2CH=CH_2$ | $CH_3$ | $CH_3$ | $CO_2CH_3$ | Na | DE-A 2 439 104 |
| 12.2 | $C_3H_7$ | $CH_2CH_3$ | $CH_2CH(CH_3)SCH_2CH_3$ | H | H | H | DE-A 2 822 304 |
| 12.3 | $C_2H_5$ | $CH_2CH=CHCl$ | $CH_2CH(CH_3)SCH_2CH_3$ | H | H | H | US-A 4 440 566 |
| 12.4 | $C_3H_7$ | $CH_2CH=CHCl$ | $CH_2CH(CH_3)SCH_2CH_3$ | H | H | H | US-A 4 440 566 |
| 12.5 | $C_3H_7$ | $C_2H_5$ | 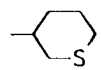 | H | H | H | EP-A 71 707 |
| 12.6 | $C_2H_5$ | $C_2H_5$ | 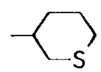 | H | H | H | EP-A 71 707 |
| 12.7 | $CH_3$ | $CH_2CH=CHCH_3$ | 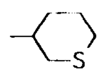 | H | H | H | EP-A 71 707 |
| 12.8 | $C_3H_7$ | $C_2H_5$ | 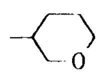 | H | H | H | EP-A 71 707 |
| 12.9 | $C_2H_5$ | $CH_2CH=CHCl$ | 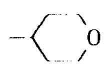 | H | H | H | EP-A 142 741 |

Tabelle 12 (Fortsetzung)

| Nr. | $R^d$ | $R^e$ | $R^f$ | $R^g$ | $R^h$ | $R^i$ | Literatur |
|---|---|---|---|---|---|---|---|
| 12.10 | $C_3H_7$ | $C_2H_5$ | 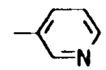 | H | H | H | EP-A 66 195 |
| 12.11 | $C_2H_5$ | $C_2H_5$ | 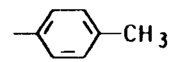 | H | H | H | DE-A 24 39 104 |
| 12.12 | $C_2H_5$ | $CH_2CH=CHCH_3$ | 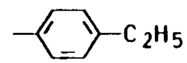 | H | H | H | DE-A 38 08 072 |
| 12.13 | $C_2H_5$ | $C_2H_5$ | 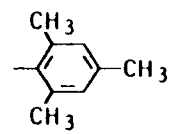 | H | H | H | EP-A 880 301 |
| 12.14 | $C_3H_7$ | $CH_2CH=CHCl$ | 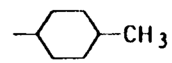 | H | H | H | EP-A 88 299 |
| 12.15 | $C_3H_7$ | $CH_2CH=CHCH_3$ | 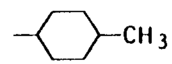 | H | H | H | EP-A 88 299 |
| 12.16 | $C_2H_5$ | $CH_2CH=CHCH_3$ | 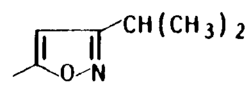 | H | H | H | EP-A 238 021 |
| 12.17 | $C_3H_7$ | $CH_2CH=CHCH_3$ | 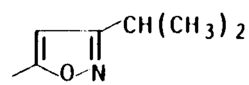 | H | H | H | EP-A 238 021 |
| 12.18 | $C_2H_5$ | $CH_2CH=CHCl$ | 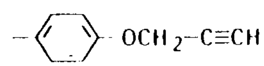 | H | H | H | EP-A 137 174 |
| 12.19 | $C_3H_7$ | $C_2H_5$ | 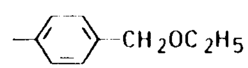 | H | H | H | EP-A 2 137 200 |

Tabelle 12 (Fortsetzung)

| Nr. | $R^d$ | $R^e$ | $R^f$ | $R^g$ | $R^h$ | $R^i$ | Literatur |
|---|---|---|---|---|---|---|---|
| 12.20 | $C_3H_7$ | $C_2H_5$ | 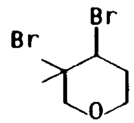 | H | H | H | EP-A 230 235 |
| 12.21 | $C_3H_7$ | $CH_2CH=CHCl$ | 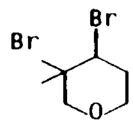 | H | H | H | EP-A 230 235 |
| 12.22 | $C_3H_7$ | $CH_2CH=CHCl$ | 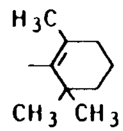 | H | H | H | EP-A 46 860 |
| 12.23 | $C_3H_7$ | $C_2H_5$ | 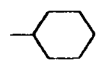 | H | H | H | JP-A 540 191 945 |
| 12.24 | $C_3H_7$ | $C_2H_5$ | 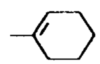 | H | H | H | EP-A 46 860 |
| 12.25 | $CH_3$ | $CH_2CH=CHCl$ | 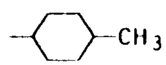 | H | H | H | EP-A 88 299 |
| 12.26 | $C_3H_7$ | $C_2H_5$ | 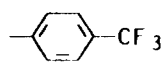 | H | H | K | EP-A 137 174 |
| 12.27 | $C_2H_5$ | $CH_2CH=CHCl$ | 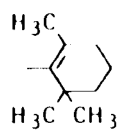 | H | H | H | EP-A 46 860 |

Tabelle 12 (Fortsetzung)

| Nr. | $R^d$ | $R^e$ | $R^f$ | $R^g$ | $R^h$ | $R^i$ | Literatur |
|---|---|---|---|---|---|---|---|
| 12.28 | $C_3H_7$ | $CH_2CH=CHCH_3$ | 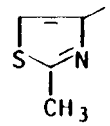 | H | H | H | EP-A 125 094 |
| 12.29 | $C_3H_7$ | $CH_2CH=CHCl$ | 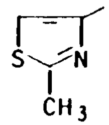 | H | H | H | EP-A 125 094 |
| 12.30 | $C_3H_7$ | $C_2H_5$ | 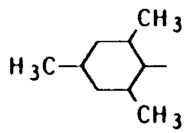 | H | H | H | EP-A 88 299 |
| 12.31 | $C_3H_7$ | $CH_2CH=CH_2$ | 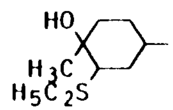 | H | H | H | EP-A 228 598 |
| 12.32 | $C_2H_5$ | $C_2H_5$ | 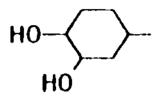 | H | H | H | EP-A 228 598 |
| 12.33 | $C_3H_7$ | $C_2H_5$ | 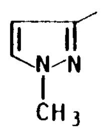 | H | H | H | EP-A 66 195 |
| 12.34 | $C_3H_7$ | $CH_2CH=CHCl$ | 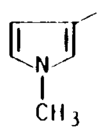 | H | H | H | EP-A 66195 |

Tabelle 12 (Fortsetzung

| Nr. | $R^d$ | $R^e$ | $R^f$ | $R^g$ | $R^h$ | $R^i$ | Literatur |
|---|---|---|---|---|---|---|---|
| 12.35 | $C_3H_7$ | $CH_2CH=CH_2$ | 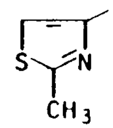 | H | H | H | EP-A 125 094 |
| 12.36 | $C_3H_7$ | $C_3H_7$ | $CH(SCH_2CH_3)_2$ | H | H | H | EP-A 230 260 |
| 12.37 | $C_3H_7$ | $C_2H_5$ | 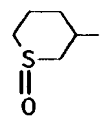 | H | H | H | EP-A 115 808 |
| 12.38 | $C_3H_7$ | $C_2H_5$ | 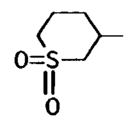 | H | H | H | EP-A 115 808 |
| 12.39 | $C_3H_7$ | $C_2H_5$ | $CH_3$ | $CH_3$ | $C(CH_3)=NOCH_3$ | H | EP-A 172 551 |
| 12.40 | $C_3H_7$ | $CH_2CH=CH_2$ | 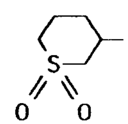 | OH | H | H | Proceedings Brit. Crop-Protection Conference - weeds 1985 Vol.1 S. 93-98 |
| 12.41 | $C_2H_5$ | $CH_2CH=CH-CH_2$- 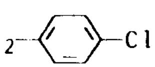 | 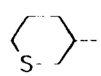 | H | H | H | EP-A 38 38 309 |
| 12.42 | $C_2H_5$ | $CH_2CH_2-CH=CH$- 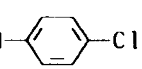 | 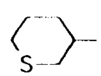 | H | H | H | EP-A 38 38 309 |

Tabelle 12 (Fortsetzung)

| Nr. | $R^d$ | $R^e$ | $R^f$ | $R^g$ | $R^h$ | $R^i$ | Literatur |
|---|---|---|---|---|---|---|---|
| 12.43 | $C_2H_5$ | $CH_2CH_2$-CH=CH- 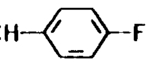 | 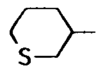 | H | H | H | EP-A 38 38 309 |
| 12.44 | n-$C_3H_7$ | $CH_2CH_2$-CH=CH- 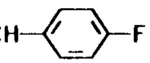 | 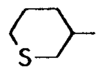 | H | H | H | EP-A 38 38 309 |
| 12.45 | $C_2H_5$ | $CH_2$CH=CH-$CH_2$- 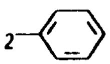 | 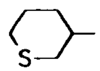 | H | H | H | EP-A 38 38 309 |
| 12.46 | n-$C_3H_7$ | $CH_2$ 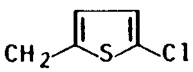 | 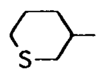 | H | H | H | EP-A 177 913 |
| 12.47 | $C_2H_5$ | $CH_2$ 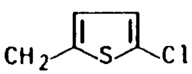 | 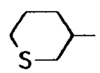 | H | H | H | EP-A 177 913 |
| 12.48 | $C_2H_5$ | $CH_2$ 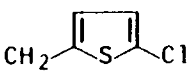 | 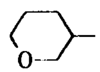 | H | H | H | EP-A 177 913 |
| 12.49 | n-$C_3H_7$ | $CH_2$ 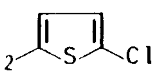 | 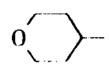 | H | H | H | EP-A 177 913 |

Tabelle 12 (Fortsetzung)

| Nr. | $R^d$ | $R^e$ | $R^f$ | $R^g$ | $R^h$ | $R^i$ | Literatur |
|---|---|---|---|---|---|---|---|
| 12.50 | $n-C_3H_7$ | $CH_2$ 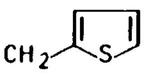 | 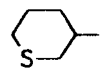 | H | H | H | EP-A 177 913 |
| 12.51 | $CH_3$ | $CH_2$ 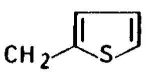 | 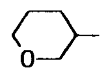 | H | H | H | EP-A 177 913 |
| 12.52 | $C_2H_5$ | $CH_2$ 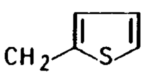 | 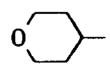 | H | H | H | EP-A 177 913 |

Tabelle 13

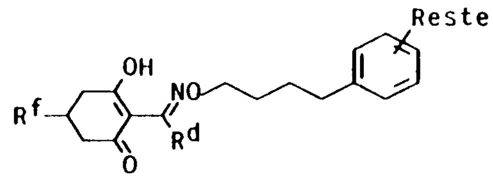

VI ($R^e$ = $-CH_2(CH_2)_2CH_2-$ 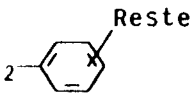 ; $R^g$, $R^h$, $R^i$ = H)

| Nr. | $R^d$ | $R^f$ | Reste am Phenylring | physik. Daten [NMR* (δ in ppm)] |
|---|---|---|---|---|
| 13.01 | $C_2H_5$ | 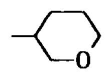 | 4-F | 2.9 (breit, 2H); 4.1 (breit, 2H) |
| 13.02 | n-$C_3H_7$ | 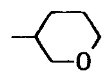 | 4-F | 2.9 (t, 2H); 4.05 (breit, 2H) |
| 13.03 | $C_2H_5$ | 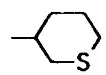 | 4-F | 2.9 (t, 2H); 4.05 (breit, 2H) |
| 13.04 | n-$C_3H_7$ | 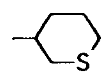 | 4-F | 2.9 (t, 2H); 4.05 (breit, 2H) |
| 13.05 | $C_2H_5$ | 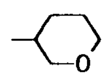 | 4-F | 4.05 (breit, 2H) |
| 13.06 | n-$C_3H_7$ | 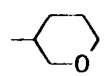 | 4-F | 4.05 (breit, 2H) |
| 13.07 | $C_2H_5$ | 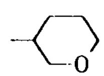 | 4-Cl | 2.9 (t, 2H); 4.05 (breit, 2H) |

*) ausgewählte Signale

Tabelle 13 (Fortsetzung)

| Nr. | $R^d$ | $R^f$ | Reste am Phenylring | physik. Daten [NMR* (δ in ppm)] |
|---|---|---|---|---|
| 13.08 | $n-C_3H_7$ | 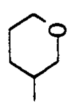 | 4-Cl | 2.9 (t, 2H); 4.05 (breit, 2H) |
| 13.09 | $C_2H_5$ | 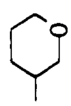 | 4-Cl | 2.9 (t, 2H); 4.05 (breit, 2H) |
| 13.10 | $n-C_3H_7$ | 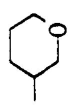 | 4-Cl | 2.9 (breit, 2H); 4.05 (breit, 2H) |
| 13.11 | $C_2H_5$ | 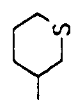 | 4-Cl | 4.05 (breit, 2H) |
| 13.12 | $n-C_3H_7$ | 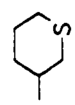 | 4-Cl | 4.05 (breit, 2H) |

*) ausgewählte Signale

Herbizide Wirkstoffe und antidotisch wirkende Verbindungen können gemeinsam oder getrennt nach dem Auflaufen auf die Blätter und Sprosse der Kulturpflanzen und der unerwünschten Gräser ausgebracht werden. Bevorzugt wird das antidotisch wirkende Mittel gleichzeitig mit dem herbiziden Wirkstoff ausgebracht. Auch eine getrennte Ausbringung, wobei das Antidot zuerst und anschließend der herbizide Wirkstoff auf das Feld gebracht werden, ist möglich. Herbizider Wirkstoff und Antidot können hierbei als Spritzmittel in suspendierbarer, emulgierbarer oder löslicher Form gemeinsam oder getrennt formuliert

vorliegen.

Antidotische Effekte werden auch durch Behandlung der Kulturpflanzensamen oder der Stecklinge mit dem Antidot vor der Aussaat bzw. vor dem Auspflanzen erzielt. Der herbizide Wirkstoff wird dann allein in der üblichen Weise appliziert.

Bei der Saatgutbehandlung werden im allgemeinen Wirkstoffmengen von 0,1 bis 10 g, vorzugsweise 1 bis 2 g, je Kilogramm Saatgut benötigt.

Bei der Applikation des Antidot durch Samenquellung oder bei der Stecklingsbehandlung werden bevorzugt Lösungen eingesetzt, die den antagonistischen Wirkstoff in einer Konzentration von 1 bis 10.000 ppm, insbesondere 100 bis 10.000 ppm, enthalten.

Für herbizide 2-(4-Heteroaryloxy)- bzw. 2-(4-Aryloxy)phenoxyessigsäurederivate V werden unterschiedliche Mengen einer antidotisch wirkenden Verbindung benötigt, wenn das Herbizid in verschiedenen Kulturen eingesetzt wird. Die Mengenverhältnisse sind in breiten Bereichen variabel. Sie sind ebenfalls abhängig von der Struktur der 2-(4-Heteroaryloxy)- bzw. 2-(4-Aryloxy)phenoxyessigsäurederivate V und der jeweiligen Zielkultur. Geeignete Anteilverhältnisse herbizider Wirkstoffe:antidotisch wirkender Verbindung liegen zwischen 1 : 10 bis 1 : 0,01; vorzugsweise zwischen 1 : 4 bis 1 : 0,1 Gew.-Teile.

Für das gleiche Cyclohexenonderivat VI werden unterschiedliche Mengen einer antidotisch wirkenden Verbindung benötigt, wenn das Cyclohexenonderivat VI in verschiedenen Kulturen eingesetzt wird. Die Mengenverhältnisse, in denen ein Cyclohexenonderivat VI und ein pyrido-anellliertes 4-Oxo-4H-benzopyran I oder I' eingesetzt werden, sind in breiten Bereichen variabel. Sie sind abhängig von der Struktur des Cyclohexenonderivats VI, des 4-Oxo-4H-benzopyrans I oder I' und der jeweiligen Kultur. Geeignete Anteilverhältnisse herbizider Wirkstoff: antidotisch wirkender Verbindung liegen zwischen 1 : 10 bis 1 : 0,01; vorzugsweise 1 : 4 bis 1 : 0,25 Gew.-Teile sowohl bei gemeinsamer als auch bei getrennter Ausbringung.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentige wäßrige, ölige oder sonstige Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Ölsdispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon und Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin-Sulfitablaugen und Methylcellulose in Betracht.

Pulver, Streu- und Stäubmittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,01 und 95 Gew.-% an herbizidem Wirkstoff und Antidot, vorzugsweise zwischen 0,5 und 90 Gew.-%. Die Aufwandmengen an herbizidem Wirkstoff betragen 0,1 bis 5 kg/ha aktive Substanz (a.S.).

Die neuen herbiziden Mittel können neben dem pyrido-anellierten 4-Oxo-4H-benzopyran I oder I' als Antidot und dem Herbizid aus der Gruppe der 2-(4-Heteroaryloxy)- bzw. 2-(4-Aryloxy)phenoxyessigsäuren V und der Cyclohexenone VI weitere herbizide oder wachstumsregulierende Wirkstoffe anderer chemischer Struktur enthalten, wobei der antagonistische Effekt erhalten bleibt.

Herstellungsbeispiele

Beispiel 1

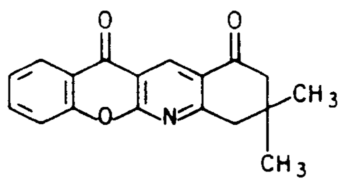

Wirkstoffbeispiel 2.012

Eine Mischung aus 9,5 g (0,050 mol) 2-Amino-4-oxo-4H-chromen-3-carbaldehyd, 7,7 g (0,055 mol) Dimedon, 300 ml Ethanol und 5 ml Piperidin wurde 5 h unter Rückfluß zum Sieden erhitzt. Der beim Abkühlen ausgefallene Niederschlag wurde abgesaugt, mit Ethanol gewaschen und getrocknet. Ausbeute: 72 %; Fp. 250 °C (Zers.).

Beispiel 2

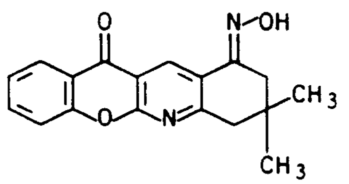

Wirkstoffbeispiel 3.001

Eine Mischung aus 7,3 g (0,025 mol) des Produktes aus Beispiel 1, 3,5 g (0,050 mol) Hydroxylammoniumchlorid, 100 ml Ethanol, 20 ml Wasser und 2 ml konz. Salzsäure wurden 5 h unter Rückfluß zum Sieden erhitzt. Der beim Abkühlen erhaltene Niederschlag wurde abgesaugt, mit Ethanol gewaschen und getrocknet. Ausbeute: 91 %; Fp. > 250 °C.

Beispiel 3

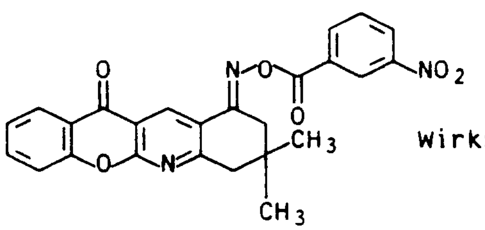

Wirkstoffbeispiel 3.013

Eine Mischung aus 9,27 g (0,030 mol) des Produktes aus Beispiel 2, 3,93 g (0,035 mol) Kalium-tert.-butylat und 70 ml Pyridin wurde bei Raumtemperatur mit 6,50 g (0,035 mol) 3-Nitrobenzoylchlorid versetzt.

Nach zweistündigem Rühren wurde die Suspension auf 200 ml 5proz. Salzsäure gegeben, der Niederschlag abgesaugt, mit Wasser gewaschen und getrocknet. Ausbeute: 90 %; Fp. 188 - 190 °C (Zers.).

Herstellung der Ausgangsstoffe gemäß J. Med. Chem. 28 (1985) 559.

Beispiel 4

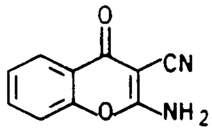

9,5 g (0,050 mol) 2-Amino-4-oxo-4H-chromen-3-carbaldehyd wurden mit 3,8 g (0,055 mol) Hydroxylammoniumchlorid in 50 ml Ameisensäure 45 Minuten zum Sieden erhitzt. Die abgekühlte Lösung wurde mit 200 ml Wasser versetzt, der Niederschlag abgesaugt, mit Wasser gewaschen und getrocknet. Ausbeute: 90 %; Fp. >250 °C.

Die in den nachstehenden Tabellen 14 und 15 aufgeführten Vorprodukte bzw. Wirkstoffe können analog diesen Beispielen hergestellt werden.

Tabelle 14: Ausgangsverbindungen

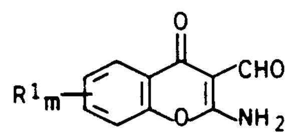

| Beispiel Nr. | R1 | Fp. (°C) | Lit. |
|---|---|---|---|
| 14.001 | H | 250(Z) | 1) |
| 14.002 | 6-$CH_3$ | 282-4 | 1) |
| 14.003 | 6-$C_2H_5$ | 246-9(Z) | 1) |
| 14.004 | 7-$OCH_3$ | 251-4(Z) | 1) |

Tabelle 15: Wirkstoffe I

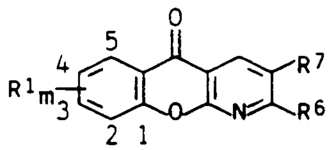

| Bsp. Nr. | $R^1$ | $R^6$ | $R^7$ | Fp. (°C) | Lit. |
|---|---|---|---|---|---|
| 15.001 | H | $C_6H_5$ | $C_6H_5$ | | |
| 15.002 | $5-C_2H_5$ | OH | CN | > 300 | 1 |
| 15.003 | H | $NH_2$ | CN | > 320 | 3 |
| 15.004 | H | $NH_2$ | $COOC_2H_5$ | 235 - 236 | 3 |
| 15.005 | H | OH | $COOC_2H_5$ | 239 - 242 | 3 |
| 15.006 | H | $NH_2$ | $C_6H_5$ | 248 - 250 | |
| 15.007 | H | $CH_3$ | $CO-CH_3$ | > 250 | |
| 15.008 | H | $CH_3$ | $C(CH_3)=N-OH$ | | |
| 15.009 | H | 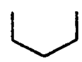 | | > 250 | |
| 15.010 | H | 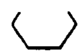 | | 265 | |
| 15.011 | H | 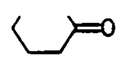 | | | |
| 15.012 | H | 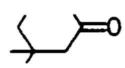 | | 250 (Zers.) | |
| 15.013 | H | 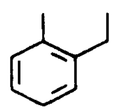 | | | |
| 15.014 | H | 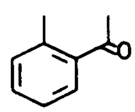 | | > 250 | |
| 15.015 | H | $COOCH_3$ | $COOCH_3$ | | |
| 15.016 | $3-OCH_3$ | $COOCH_3$ | $COOCH_3$ | | |
| 15.017 | $3-OCH_3$ | $COOCH_3$ | $COOCH_3$ | | |
| 15.018 | $4-i-C_3H_7$ | $COOCH_3$ | $COOCH_3$ | | |
| 15.019 | $3-OCH_3$ | COOH | COOH | | |

Tabelle 15: Wirkstoffe I
(Fortsetzung)

| Bsp. Nr. | $R^1$ | $R^6$ | $R^7$ | Fp. (°C) | Lit. |
|---|---|---|---|---|---|
| 15.020 | H | $CO-NH-C(CH_3)-CONH_2$<br>$i-C_3H_7$ | COOH | | |
| 15.021 | $4-i-C_3H_7$ | $CO-NH-C(CH_3)-CONH_2$<br>$i-C_3H_7$ | COOH | | |
| 15.022 | H | 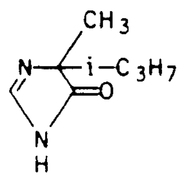 | COOH | | |
| 15.023 | $3-OCH_3$ | 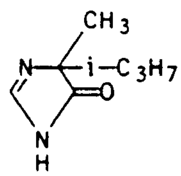 | COOH | | |
| 15.024 | $4-i-C_3H_7$ | 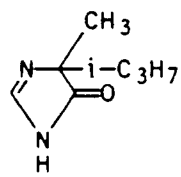 | COOH | | |

Tabelle 16

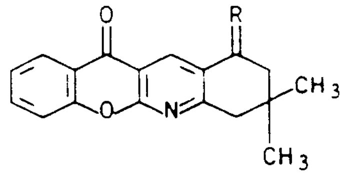

| Beispiel-Nr. | R | Fp. (°C) |
|---|---|---|
| 16.001 | =N–OH | > 250 |
| 16.002 | $=N-O-CO-CH_3$ | 200 (Zers.) |
| 16.003 | $=N-O-CO-CH_2-CH_3$ | 166 - 172 |
| 16.004 | $=N-O-CO-(CH_2)_2-CH_3$ | 200 |
| 16.005 | $=N-O-CO-(CH_2)_4-CH_3$ | 182 - 186 |
| 16.006 | $=N-O-CO-(CH_2)_{14}-CH_3$ | 92 - 96 |
| 16.007 | $=N-O-CO-C(CH_3)_3$ | 210 (Zers.) |
| 16.008 | $=N-O-CO-CH_2-C(CH_3)_3$ | 186 - 190 |
| 16.009 | $=N-O-CO-C_6H_5$ | |
| 16.010 | $=N-O-CO-(2-Cl-C_6H_4)$ | 180 - 190 |
| 16.011 | $=N-O-CO-(2,4-Cl_2-C_6H_3)$ | > 250 |
| 16.012 | $=N-O-CO-(2-NO_2-C_6H_4)$ | |
| 16.013 | $=N-O-CO-(3-NO_2-C_6H_4)$ | 188 - 190 |
| 16.014 | $=N-O-CO-(4-NO_2-C_6H_4)$ | > 250 |

Literatur

1) A.Nohara et al., J. Med. Chem. 28 (1985) 559.
2) G. P. Ellis et al., J. Chem. Soc. Perkin Trans I 1986, 1643.
3) U. Petersen et al., Liebigs Ann. Chem. 1976, 1659.

Beispiele zur biologischen Wirkung

Der Einfluß verschiedener Vertreter der erfindungsgemäßen herbiziden Mittel bzw. Mittelkombinationen, bestehend aus Herbizid und antidotisch wirkender Verbindung, auf das Wachstum von erwünschten und unerwünschten Pflanzen im Vergleich zum herbiziden Wirkstoff allein wird durch die folgenden biologischen Beispiele aus Gewächshausversuchen belegt:
Als Kulturgefäße dienten Plastikblumentöpfe mit rund 300 $cm^3$ Inhalt und lehmiger Sand mit etwa 3,0 % Humus als Substrat. Die Samen der Testpflanzen werden nach Arten getrennt, flach eingesät und befeuchtet. Danach wurden die Gefäße mit durchsichtigen Plastikhauben abgedeckt, bis die Samen gleichmäßig gekeimt und die Pflanzen angewachsen waren.

Für die Nachauflaufbehandlung wurden die Testpflanzen je nach Wuchsform erst bis zu einer Wuchshöhe von 3 bis 20 cm angezüchtet und erst dann behandelt. Die herbiziden Mittel wurden hierbei in Wasser

als Verteilungsmittel suspendiert oder emulgiert und mittels fein verteilender Düsen gespritzt.

Die Versuchsgefäße wurden im Gewächshaus aufgestellt, wobei für wärmeliebende Arten 18 bis 30 °C und für solche gemäßigtere Klimate 10 bis 25 °C bevorzugt wurden.

Die Versuchsperiode erstreckte sich über 3 bis 5 Wochen. Während dieser Zeit wurden die Pflanzen gepflegt, und ihre Reaktion auf die einzelnen Behandlungen wurde erfaßt.

Bewertet wurde die Schädigung der Testpflanzen anhand einer Skala von 0 bis 100 % im Vergleich zu den unbehandelten Kontrollpflanzen. Dabei bedeutet 0 keine Schädigung und 100 eine völlige Zerstörung der Pflanzen.

Die in den Gewächshausversuchen verwendeten Pflanzen setzten sich aus folgenden Arten zusammen:

| Lat.Name | Deutscher Name | Englischer Name |
|---|---|---|
| Brachiaria platiphylla | Signalgras | broadleaf signalgrass |
| Setaria viridis | Grüne Borstenhirse | green foxtail |
| Triticum aestivum | Weichweizen | winter wheat |
| Zea mays | Mais | Indian corn |

Als Beispielsherbizid der Cyclohexenonderivate der Formel VI diente

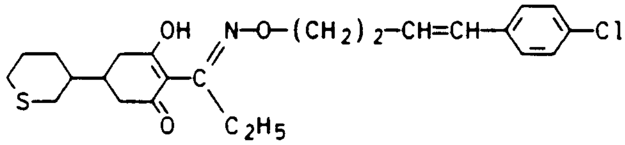

Nr. 12.42

Der Wirkstoff 12.42 wurde als Emulsionskonzentrat mit 200 g Wirkstoff/l allein unter Zugabe der für die Antidots nötigen Lösungsmittelmengen, 80 Gew.-% Cyclohexenon und 20 Gew.-% Tensid (Emulphor EL*)) mit 10 Gew.-% Wirkstoff, appliziert.

Sämtliche antidotisch wirkenden Verbindungen wurden für die Nachauflaufbehandlung in einem Gemisch, bestehend aus 80 Gew.-% Cyclohexenon und 20 Gew.-% Tensid (Emulphor EL*)) mit 10 Gew.-% Wirkstoff aufbereitet.

Die anschließenden Tabellen 17 bis 19 dokumentieren, daß die antidotisch wirksamen erfindungsgemäßen Verbindungen die Verträglichkeit des Wirkstoffs 12.42 für Kulturpflanzen, die zu der Familie der Gramineen (Gräser) zählen, deutlich verbessern.

Tabelle 17

**Verbesserung der Verträglichkeit des herbiziden Wirkstoffs 12.42 für Kulturpflanzen durch Kombination mit der Verbindung 15.004 bei Nachauflaufanwendung im Gewächshaus**

*) ethoxyliertes Rizinusöl (caster oil)

| Aufwandmenge [kg/ha a.S.] | | Testpflanzen und Schädigung [%] | | |
|---|---|---|---|---|
| | | Kulturpflanzen | | unerw.Pflanze |
| Herbizid 12.42 | Antidot 15.004 | Triticum aestivum[1] | Zea mays | Setaria viridis |
| 0.25 | - | 98 | 90 | 100 |
| 0.25 | 0.5 | 15 | 30 | 98 |

[1] Sorte "Star"

Tabelle 18

Verbesserung der Verträglichkeit des Herbizids 12.42 für Mais durch Zumischen der antidotisch wirksamen Verbindung 15.018 oder 15.021 bei Nachauflaufanwendung im Gewächshaus

| Aufwandmenge [kg/ha a.S.] | | | Testpflanzen und Schädigung [%] | | |
|---|---|---|---|---|---|
| | | | Kulturpflanze | unerw.Pflanzen | |
| Herbizid | Antidot | | Zea [1] | Brachiaria | Setaria |
| 12.42 | 15.015 | 15.021 | mays | platiphylla | viridis |
| 0,125 | - | - | 65 | 100 | 98 |
| 0,125 | 0,125 | - | 20 | 95 | 98 |
| 0,125 | - | 0,125 | 25 | 98 | 95 |

[1]) Sorte "Lixis"

Tabelle 19

Verbesserung der Verträglichkeit des Herbizids 12.42 für Mais und Weizen durch Zumischen der antidotisch wirksamen Verbindung 15.015 bei Nachauflaufanwendung im Gewächshaus

| Aufwandmenge [kg/ha a.S.] | | Testpflanzen und Schädigung [%] | | |
|---|---|---|---|---|
| | | Kulturpflanzen | | unerw.Pflanze |
| Herbizid 12.42 | Antidot 15.015 | Triticum aestivum2) | Zea mays1) | Setaria viridis |
| 0,125 | - | 90 | 98 | 98 |
| 0,125 | 0,125 | 10 | 10 | 95 |

1) Sorte "Lixis"
2) Sorte "Star"

## Patentansprüche

**1.** Pyrido-anellierte 4-Oxo-4H-benzopyrane der allgemeinen Formel I

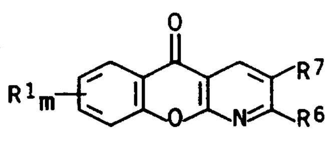

I,

in der die Substituenten und der Index folgende Bedeutung haben:

m 0, 1 oder 2, wobei die Reste $R^1$ verschieden sein können, wenn m 2 bedeutet;

$R^1$ Wasserstoff; Hydroxy; Halogen; Nitro; $C_1$-$C_6$-Alkyl; $C_1$-$C_6$-Alkoxy; $C_1$-$C_6$-Halogenalkyl; $C_1$-$C_4$-Halogenalkoxy; $C_1$-$C_4$-Alkylthio oder eine Gruppe $NR^2R^3$, wobei

$R^2$ Wasserstoff oder $C_1$-$C_4$-Alkyl und

$R^3$ Wasserstoff, $C_1$-$C_4$-Alkyl, eine Gruppe -CO-$R^4$, -CS-$R^4$ oder -$SO_2$-$R^5$ bedeuten;

$R^4$ $C_1$-$C_{20}$-Alkyl; $C_1$-$C_6$-Halogenalkyl; $C_3$-$C_8$-Cycloalkyl; $C_1$-$C_3$-Aralkyl; Amino; $C_1$-$C_4$-Alkylamino; Di-$C_1$-$C_4$-alkylamino; eine Phenyl- oder Phenylaminogruppe, wobei der aromatische Ring eine bis drei der für $R^1$ genannten Gruppen tragen kann;

$R^5$ $C_1$-$C_4$-Alkyl; Phenyl, das eine bis drei der bei $R^1$ genannten Gruppen tragen kann;

$R^6$ Hydroxy; $C_1$-$C_6$-Alkoxy; $C_1$-$C_6$-Alkyl; eine Gruppe -NH-$R^8$ oder -N($C_1$-$C_4$-Alkyl)-$R^8$, wobei $R^8$ für eine der Bedeutungen von $R^3$ steht; ein Phenylrest, der eine bis drei der für $R^1$ genannten Gruppen tragen kann, eine $C_1$-$C_4$-alkylsubstituierte 4-Methyl-5-oxo-imidazolin-2-yl-Gruppe oder eine Gruppe -CO-$R^9$ oder -CS-$R^9$;

$R^7$ Cyano, $C_1$-$C_6$-Alkyl; ein Phenylrest, der eine bis drei der für $R^1$ genannten Gruppen tragen kann; eine Gruppe -CH=N-$R^{10}$, -C($C_1$-$C_4$-Alkyl)=N-$R^{10}$, -CO-$R^{11}$ oder -CS-$R^{11}$;

$R^9$, $R^{11}$ $C_1$-$C_6$-Alkyl; $C_1$-$C_6$-Alkoxy; Hydroxy; Amino; $C_1$-$C_4$-Alkylamino; durch eine Amidgruppe $CONH_2$ substituiertes, verzweigtes $C_2$-$C_6$-Alkyl; Di-$C_1$-$C_4$-alkylamino; ein Aminophenylrest, wobei der aromatische Ring ein bis drei der für $R^1$ genannten Gruppen tragen kann;

$R^{10}$ $C_1$-$C_4$-Alkyl; $C_3$-$C_8$-Cycloalkyl; Phenyl, welches eine bis drei der für $R^1$ genannten Gruppen tragen kann; Hydroxy, $C_1$-$C_8$-Alkoxy; eine Gruppe -O-CO-$R^{12}$ oder -O-CS-$R^{12}$, wobei $R^{12}$ für $C_1$-$C_{20}$-Alkyl; $C_1$-$C_6$-Halogenalkyl; $C_3$-$C_8$-Cycloalkyl; Phenyl-$C_1$-$C_3$-alkyl oder Phenyl, wobei die Phenylringe gewünschtenfalls noch einen bis drei der bei $R^1$ genannten Reste tragen können, steht;

oder für den Fall, daß $R^6$ und $R^7$ Alkyl oder $R^6$ Alkyl und $R^7$ eine Gruppe -CO-H, -CO-($C_1$-$C_4$-Alkyl), -CH=N-$R^{10}$ oder -C($C_1$-$C_4$-Alkyl)=N-$R^{10}$ bedeuten:

$R^6$ und $R^7$ gemeinsam einen 5 - 7-gliedrigen Ring, der mit einer bis vier $C_1$-$C_4$-Alkylgruppen substituiert oder mit einem Phenylring, der eine bis drei der für $R^1$ genannten Gruppen tragen kann, anelliert ist;

sowie die landwirtschaftlich brauchbaren Salze der Verbindungen I, sofern diese Verbindungen einen basischen Stickstoffsubstituenten oder einen sauren Hydroxysubstituenten enthalten,

ausgenommen 2,3-Diphenyl-[1]benzopyran[2,3-b]pyridin-5-on, 2,3-Dimethyl-[1]benzopyran[2,3-b]pyridin-5-on, 3-Ethoxycarbonyl-2-methyl-[1]benzopyran [2, 3-b]pyridin-5-on, 3-Ethoxycarbonyl-7-ethyl-2-methoxy-[1]benzopyran[2, 3-b]pyridin-5-on, 3-Ethoxycarbonyl-7-ethyl-2-methyl-[1]benzopyran [2, 3-b]pyridin-5-on, 2-Hydroxy-3-methyl-[1]benzopyran[2, 3-b]pyridin-5-on, 2,3-Diethoxycarbonyl-7-ethyl-[1]benzopyran[2,3-b]pyridin-5-on, sowie diejenigen Verbindungen I, bei denen entweder $R^6$ für $C_1$-$C_6$-Alkyl, Phenyl, Carboxyl, Hydroxyl, $C_1$-$C_6$-Alkoxy, Amino oder $C_1$-$C_4$-Alkylamino und $R^7$ für Cyano, Carboxyl oder Ethoxycarbonyl stehen, oder $R^6$ für Hydroxy und $R^7$ für Ethoxycarbonyl stehen oder $R^6$ für Methyl und $R^7$ für Methylcarbonyl stehen.

**2.** Verfahren zur Herstellung der Verbindung I gemäß Anspruch 1, dadurch gekennzeichnet, daß man den o-Aminoaldehyd II

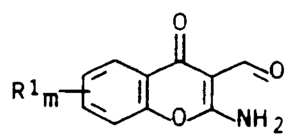

II

in Gegenwart einer Base in einem inerten Lösungsmittel mit einem Keton der Formel III

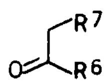

III

kondensiert.

**3.** Verfahren zur Herstellung der Verbindung I gemäß Anspruch 1, in der $R^6$ für Hydroxy oder Amino steht, dadurch gekennzeichnet, daß man den 2-Amino-4-oxo-4H-chromen-3-carbaldehyd II in an sich bekannter Weise in einem inerten Lösungsmittel in Gegenwart einer Base mit einem Essigsäurederivat der Formel IVa oder IVb anelliert, wobei R in der Formel IVa für eine $C_1$-$C_4$-Alkylgruppe steht.

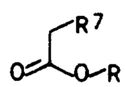

IVa

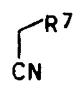

IVb

**4.** Herbizides Mittel, enthaltend mindestens ein pyridoanelliertes 4-Oxo-4H-benzopyran der allgemeinen Formel I'

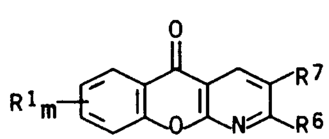

I',

| | |
|---|---|
| m | 0, 1 oder 2, wobei die Reste $R^1$ verschieden sein können, wenn m 2 bedeutet; |
| $R^1$ | Wasserstoff; Hydroxy; Halogen; Nitro; $C_1$-$C_6$-Alkyl; $C_1$-$C_6$-Alkoxy; $C_1$-$C_6$-Halogenalkyl; $C_1$-$C_4$-Halogenalkoxy; $C_1$-$C_4$-Alkylthio oder eine Gruppe $NR^2R^3$, wobei |
| $R^2$ | Wasserstoff oder $C_1$-$C_4$-Alkyl und |

$R^3$ Wasserstoff, $C_1$-$C_4$-Alkyl, eine Gruppe -CO-$R^4$, -CS-$R^4$ oder -$SO_2$-$R^5$ bedeuten;

$R^4$ $C_1$-$C_{20}$-Alkyl; $C_1$-$C_6$-Halogenalkyl; $C_3$-$C_8$-Cycloalkyl; $C_1$-$C_3$-Aralkyl; Amino; $C_1$-$C_4$-Alkylamino; Di-$C_1$-$C_4$-alkylamino; eine Phenyl- oder Phenylaminogruppe, wobei der aromatische Ring eine bis drei der für $R^1$ genannten Gruppen tragen kann;

$R^5$ $C_1$-$C_4$-Alkyl; Phenyl, das eine bis drei der bei $R^1$ genannten Gruppen tragen kann;

$R^6$ Hydroxy; $C_1$-$C_6$-Alkoxy; $C_1$-$C_6$-Alkyl; eine Gruppe -NH-$R^8$ oder -N($C_1$-$C_4$-Alkyl)-$R^8$, wobei $R^8$ für eine der Bedeutungen von $R^3$ steht; ein Phenylrest, der eine bis drei der für $R^1$ genannten Gruppen tragen kann, eine $C_1$-$C_4$-alkylsubstituierte 4-Methyl-5-oxo-imidazolin-2-yl-Gruppe oder eine Gruppe -CO-$R^9$ oder -CS-$R^9$;

$R^7$ Cyano, $C_1$-$C_6$-Alkyl; ein Phenylrest, der eine bis drei der für $R^1$ genannten Gruppen tragen kann; eine Gruppe -CH = N-$R^{10}$, -C($C_1$-$C_4$-Alkyl) = N-$R^{10}$, -CO-$R^{11}$ oder -CS-$R^{11}$;

$R^9$, $R^{11}$ $C_1$-$C_6$-Alkyl; $C_1$-$C_6$-Alkoxy; Hydroxy; Amino; $C_1$-$C_4$-Alkylamino; durch eine Amidgruppe $CONH_2$ substituiertes, verzweigtes $C_2$-$C_6$-Alkyl; Di-$C_1$-$C_4$-alkylamino; ein Aminophenylrest, wobei der aromatische Ring ein bis drei der für $R^1$ genannten Gruppen tragen kann;

$R^{10}$ $C_1$-$C_4$-Alkyl; $C_3$-$C_8$-Cycloalkyl; Phenyl, welches eine bis drei der für $R^1$ genannten Gruppen tragen kann; Hydroxy,
$C_1$-$C_8$-Alkoxy; eine Gruppe -O-CO-$R^{12}$ oder -O-CS-$R^{12}$,
wobei $R^{12}$ für
$C_1$-$C_{20}$-Alkyl; $C_1$-$C_6$-Halogenalkyl; $C_3$-$C_8$-Cycloalkyl;
Phenyl-$C_1$-$C_3$-alkyl oder Phenyl, wobei die Phenylringe gewünschtenfalls noch einen bis drei der für $R^1$ genannten Reste tragen können, steht;

oder für den Fall, daß $R^6$ und $R^7$ Alkyl oder $R^6$ Alkyl und $R^7$ eine Gruppe -CO-H, -CO-($C_1$-$C_4$-Alkyl), -CH = N-$R^{10}$ oder -C($C_1$-$C_4$-Alkyl) = N-$R^{10}$ bedeuten:

$R^6$ und $R^7$ gemeinsam einen 5 - 7-gliedrigen Ring, der mit einer bis vier $C_1$-$C_4$-Alkylgruppen substituiert oder mit einem Phenylring, der eine bis drei der für $R^1$ genannten Gruppen tragen kann, anelliert ist;

sowie die landwirtschaftlich brauchbaren Salze der Verbindungen I', sofern diese Verbindungen einen basischen Stickstoffsubstituenten oder einen sauren Hydroxysubstituenten enthalten,
und mindestens einen herbiziden Wirkstoff aus der Gruppe

A) der 2-(4-Heteroaryloxy)- oder 2-(4-Aryloxy)-phenoxyessigsäurederivate der Formel V

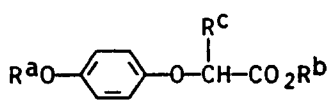

V

$R^a$ ein Phenylring, ein Pyridylring, ein Benzoxazylrest, ein Benzthiazylrest oder ein Benzpyrazinylrest, wobei diese aromatischen Ringsysteme bis zu zwei der folgenden Reste tragen können:
Halogen, Nitro, $C_1$-$C_4$-Alkyl, $C_1$-$C_4$-Halogenalkyl und/oder $C_1$-$C_4$-Halogenalkoxy,

$R^b$ Wasserstoff oder eine $C_1$-$C_4$-Alkylgruppe und

$R^c$ Wasserstoff oder eine Methylgruppe und

B) der Cyclohexenonoximether der Formel VI

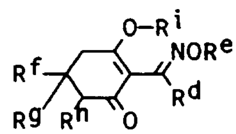

VI

in welcher die Substituenten folgenden Bedeutung haben:

$R^d$ eine $C_1$-$C_4$-Alkylgruppe;

$R^e$ eine $C_1$-$C_4$-Alkylgruppe, eine $C_3$-$C_4$-Alkenylgruppe, eine $C_3$-$C_4$-Alkinylgruppe oder eine partiell oder vollständig halogenierte $C_3$-$C_4$-Alkenylgruppe;
eine $C_1$-$C_4$-Alkylen- oder $C_2$-$C_4$-Alkenylkette, die beide noch ein bis 3 $C_1$-$C_3$-Alkylreste und/oder Halogenatome tragen können, oder eine gewünschtenfalls durch $C_1$-$C_3$-Alkyl substituierte 3- bis 6-gliedrige Alkylen- oder 4- bis 6-gliedrige Alkenylenkette, die jeweils

ein dem Oximetherteil nicht direkt benachbartes Sauerstoff- oder Schwefelatom als Kettenglied enthält, wobei alle vorstehend genannten Ketten endständig den Phenylring tragen, der seinerseits durch ein bis drei Reste substituiert sein kann, ausgewählt aus einer Gruppe bestehend aus einem Benzyloxycarbonyl- oder Phenylrest und jeweils einem bis drei der folgenden Reste: Nitro, Cyano, Halogen, $C_1$-$C_4$-Alkyl, $C_1$-$C_4$-Alkoxy, $C_1$-$C_4$-Alkylthio, partiell oder vollständig halogeniertes $C_1$-$C_4$-Alkyl, partiell oder vollständig halogeniertes $C_1$-$C_4$-Alkoxy, Carboxyl und $C_1$-$C_4$-Alkoxycarbonyl, und wobei der Phenylring noch zusätzlich so viele Halogenatome tragen kann, daß die Gesamtzahl der Reste 4 oder 5 beträgt;

eine Thienylmethylgruppe, die noch ein Halogenatom tragen kann;

$R^f$ eine $C_1$-$C_4$-Alkylgruppe, welche einfach durch $C_1$-$C_4$-Alkylthio oder $C_1$-$C_4$-Alkoxy substituiert sein kann;

ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, wobei dieser Ring bis zu drei der folgenden Reste tragen kann: Hydroxy, Halogen, $C_1$-$C_4$-Alkyl, partiell oder vollständig halogeniertes $C_1$-$C_4$-Alkyl, $C_1$-$C_4$-Alkoxy und/oder $C_1$-$C_4$-Alkylthio;

einen 10-gliedrigen gesättigten oder einfach ungesättigten Heterocyclus, welcher zwei Sauerstoffatome oder Schwefelatome enthält und durch bis zu drei $C_1$-$C_4$-Alkylgruppen und/oder Methoxygruppen substituiert sein kann;

die Phenylgruppe, die Pyridylgruppe, die Thiazolylgruppe, die Pyrazolylgruppe, die Pyrrolylgruppe oder die Isoxazolylgruppe, wobei diese Gruppen jeweils ein bis drei Reste tragen können, ausgewählt aus einer Gruppe bestehend aus $C_1$-$C_4$-Alkyl, partiell oder vollständig halogeniertes $C_1$-$C_4$-Alkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-Alkyl, $C_1$-$C_4$-Alkylthio-$C_1$-$C_3$-Alkyl, $C_1$-$C_4$-Dialkoxy-$C_1$-$C_3$-alkyl, Formyl, Halogen und Benzoylamino;

$R^g$ Wasserstoff, Hydroxy oder, wenn $R^f$ die Bedeutung $C_1$-$C_6$-Alkylgruppe hat, eine $C_1$-$C_6$-Alkylgruppe;

$R^h$ Wasserstoff, eine Cyanogruppe, ein Halogenatom, eine $C_1$-$C_4$-Alkoxycarbonylgruppe oder eine $C_1$-$C_4$-Alkylketoximgruppe und

$R^i$ Wasserstoff.

**5.** Herbizides Mittel nach Anspruch 4, dadurch gekennzeichnet, daß das Gewichtsverhältnis pyrido-anelliertes 4-Oxo-4H-benzopyran I' zu herbizidem Wirkstoff A) oder B) 10:1 bis 0,01:1 Gew.-Teile beträgt.

**6.** Verfahren zur selektiven Bekämpfung von unerwünschtem Pflanzenwuchs, dadurch gekennzeichnet, daß man ein pyrido-anelliertes 4-Oxo-4H-benzopyran der Formel I' gemäß Anspruch 4 und

A) ein 2-(4-Heteroaryloxy)- oder 2-(4-Aryloxy)-phenoxyessigsäurederivat der Formel V oder

B) ein Cyclohexenonderivat der Formel VI

gemäß Anspruch 4 vor, bei oder nach der Aussaat der Kulturpflanzen, vor oder während des Auflaufens der Kulturpflanzen gleichzeitig oder nacheinander ausbringt.

**7.** Verfahren zur Verhinderung von Schädigungen von Kulturpflanzen durch

A) herbizide 2-(4-Heteroaryloxy)- oder 2-(4-Aryloxy)-phenoxyessigsäurederivate der Formel V oder

B) Cyclohexenonderivate der Formel VI gemäß Anspruch 4,

dadurch gekennzeichnet, daß man das Saatgut der Kulturpflanzen mit einer antagonistisch wirksamen Menge eines pyrido-anellierten 4-Oxo-4H-benzopyrans der Formel I' gemäß Anspruch 4 behandelt.

**8.** Verfahren zur selektiven Bekämpfung von unerwünschtem Pflanzenwuchs, dadurch gekennzeichnet, daß man die Blätter der Kulturpflanzen und der unerwünschten Pflanzen im Nachauflaufverfahren mit einem pyrido-anellierten 4-Oxo-4H-benzopyran der Formel I' gemäß Anspruch 4 und mit einem 2-(4-Heteroaryloxy) oder 2-(4-Aryloxy)-phenoxyessigsäurederivat der Formel V oder einem Cyclohexenonderivat der Formel VI gemäß Anspruch 4 gleichzeitig oder nacheinander behandelt.

**9.** Verfahren nach Anspruch 6, dadurch gekennzeichnet, daß die Kulturpflanzen Gerste, Weizen, Mais, Kultursorghum und Reis sind.

10. Verfahren nach Anspruch 7, dadurch gekennzeichnet, daß die Kulturpflanzen Gerste, Weizen, Mais, Kultursorghum und Reis sind.

11. Verfahren nach Anspruch 8, dadurch gekennzeichnet, daß die Kulturpflanzen Gerste, Weizen, Mais, Kultursorghum und Reis sind.

## Claims

1. A pyrido-fused 4-oxo-4H-benzopyran of the formula I

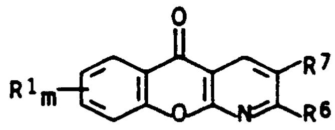

I

where
m is 0, 1 or 2, and the radicals $R^1$ may be different when m is 2,
$R^1$ is hydrogen, hydroxyl, halogen, nitro, $C_1$-$C_6$-alkyl, $C_1$-$C_6$-alkoxy, $C_1$-$C_6$-haloalkyl, $C_1$-$C_4$-haloalkoxy, $C_1$-$C_4$-alkylthio or a group $NR^2R^3$,
$R^2$ is hydrogen or $C_1$-$C_4$-alkyl,
$R^3$ is hydrogen, $C_1$-$C_4$-alkyl or a group -CO-$R^4$, -CS-$R^4$ or -$SO_2$-$R^5$,
$R^4$ is $C_1$-$C_{20}$-alkyl, $C_1$-$C_6$-haloalkyl, $C_3$-$C_8$-cycloalkyl, $C_1$-$C_3$-aralkyl, amino, $C_1$-$C_4$-alkylamino, di-$C_1$-$C_4$-alkylamino, or phenyl or phenylamino where the aromatic ring may carry from one to three of the groups stated for $R^1$,
$R^5$ is $C_1$-$C_4$-alkyl or is phenyl which may carry from one to three of the groups stated for $R^1$,
$R^6$ is hydroxyl, $C_1$-$C_6$-alkoxy, $C_1$-$C_6$-alkyl or a group -NH-$R^8$ or -N($C_1$-$C_4$-alkyl)-$R^8$ where $R^8$ has one of the meanings of $R^3$, or $R^6$ is phenyl which may carry from one to three of the groups stated for $R^1$, or is $C_1$-$C_4$-alkyl-substituted 4-methyl-5-oxoimidazolin-2-yl or a group -CO-$R^9$ or -CS-$R^9$,
$R^7$ is cyano or $C_1$-$C_6$-alkyl or is phenyl which may carry from one to three of the groups stated for $R^1$, or is a group -CH=N-$R^{10}$, -C($C_1$-$C_4$-alkyl)=N-$R^{10}$, -CO-$R^{11}$ or -CS-$R^{11}$,
$R^9$ and $R^{11}$ are each $C_1$-$C_6$-alkyl, $C_1$-$C_6$-alkoxy, hydroxyl, amino or $C_1$-$C_4$-alkylamino or is branched $C_2$-$C_6$-alkyl which is substituted by an amido group $CONH_2$, or is di-$C_1$-$C_4$-alkylamino or aminophenyl where the aromatic ring may carry from one to three of the groups stated for $R^1$,
$R^{10}$ is $C_1$-$C_4$-alkyl or $C_3$-$C_8$-cycloalkyl or is phenyl which may carry from one to three of the groups stated for $R^1$, or is hydroxyl, $C_1$-$C_8$-alkoxy or a group -O-CO-$R^{12}$ or -O-CS-$R^{12}$ where $R^{12}$ is $C_1$-$C_{20}$-alkyl, or $R^{10}$ is $C_1$-$C_6$-haloalkyl, $C_3$-$C_8$-cycloalkyl, phenyl-$C_1$-$C_3$-alkyl or phenyl where the phenyl rings may, if desired, furthermore carry from one to three of the radicals stated for $R^1$,
or, if $R^6$ and $R^7$ are each alkyl or $R^6$ is alkyl and $R^7$ is a group -CO-H, -CO-($C_1$-$C_4$-alkyl), -CH=N-$R^{10}$ or -C($C_1$-$C_4$-alkyl)=N-$R^{10}$,
$R^6$ and $R^7$ together form a 5-membered to 7-membered ring which is substituted by from one to four $C_1$-$C_4$-alkyl groups or is fused with a phenyl ring which may carry from one to three of the groups stated for $R^1$,
and the agriculturally useful salts of the compounds I, where these compounds contain a basic nitrogen substituent or an acidic hydroxyl substituent,
with the exception of 2,3-diphenyl-[1]benzopyran[2,3-b]pyridin-5-one,2,3-dimethyl-[1]benzopyran[2,3-b]-pyridin-5-one, 3-ethoxycarbonyl-2-methyl-[1]benzopyran[2,3-b]pyridin-5-one, 3-ethoxycarbonyl-7-ethyl-2-methoxy-[1]benzopyran[2,3-b]pyridin-5-one, 3-ethoxycarbonyl-7-ethyl-2-methyl-[1]benzopyran[2,3-b]-pyridin-5-one, 2-hydroxy-3-methyl-[1]benzopyran[2,3-b]pyridin-5-one, 2,3-diethoxycarbonyl-7-ethyl-[1]-benzopyran[2,3-b]pyridin-5-one and those compounds I in which either $R^6$ is $C_1$-$C_6$-alkyl, phenyl, carboxyl, hydroxyl, $C_1$-$C_6$-alkoxy, amino or $C_1$-$C_4$-alkylamino and $R^7$ is cyano, carboxyl or ethoxycarbonyl or $R^6$ is hydroxyl and $R^7$ is ethoxycarbonyl or $R^6$ is methyl and $R^7$ is methylcarbonyl.

2. A process for the preparation of a compound I as claimed in claim 1, wherein an o-aminoaldehyde II

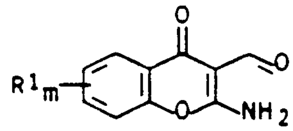

II

is condensed with a ketone of the formula III

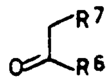

III

in the presence of a base in an inert solvent.

3. A process for the preparation of a compound I as claimed in claim 1, in which $R^6$ is hydroxyl or amino, wherein a 2-amino-4-oxo-4H-chromene-3-carbaldehyde II is fused in a conventional manner, in an inert solvent in the presence of a base, with an acetic acid derivative of the formula IVa or IVb where R in the formula IVa is $C_1$-$C_4$-alkyl.

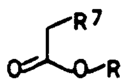

IVa

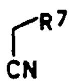

IVb

4. A herbicide containing at least one pyrido-fused 4-oxo-4H-benzopyran of the formula I'

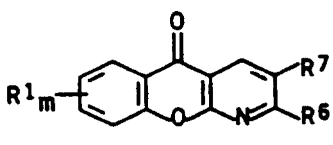

I'

where
m is 0, 1 or 2, and the radicals $R^1$ may be different when m is 2,
$R^1$ is hydrogen, hydroxyl, halogen, nitro, $C_1$-$C_6$-alkyl, $C_1$-$C_6$-alkoxy, $C_1$-$C_6$-haloalkyl, $C_1$-$C_4$-haloalkoxy, $C_1$-$C_4$-alkylthio or a group $NR^2R^3$,
$R^2$ is hydrogen or $C_1$-$C_4$-alkyl,
$R^3$ is hydrogen, $C_1$-$C_4$-alkyl or a group -CO-$R^4$, -CS-$R^4$ or -$SO_2$-$R^5$,
$R^4$ is $C_1$-$C_{20}$-alkyl, $C_1$-$C_6$-haloalkyl, $C_3$-$C_8$-cycloalkyl, $C_1$-$C_3$-aralkyl, amino, $C_1$-$C_4$-alkylamino, di-$C_1$-$C_4$-alkylamino, or phenyl or phenylamino where the aromatic ring may carry from one to three of the groups stated for $R^1$,
$R^5$ is $C_1$-$C_4$-alkyl or is phenyl which may carry from one to three of the groups stated for $R^1$,
$R^6$ is hydroxyl, $C_1$-$C_6$-alkoxy, $C_1$-$C_6$-alkyl or a group -NH-$R^8$ or -N($C_1$-$C_4$-alkyl)-$R^8$ where $R^8$ has one of the meanings of $R^3$, or $R^6$ is phenyl which may carry from one to three of the groups stated for $R^1$, or is $C_1$-$C_4$-alkyl-substituted 4-methyl-5-oxoimidazolin-2-yl or a group -CO-$R^9$ or -CS-$R^9$,
$R^7$ is cyano or $C_1$-$C_6$-alkyl or is phenyl which may carry from one to three of the groups stated for $R^1$, or is a group -CH=N-$R^{10}$, -C($C_1$-$C_4$-alkyl)=N-$R^{10}$, -CO-$R^{11}$ or -CS-$R^{11}$,
$R^9$ and $R^{11}$ are each $C_1$-$C_6$-alkyl, $C_1$-$C_6$-alkoxy, hydroxyl, amino or $C_1$-$C_4$-alkylamino or is branched $C_2$-$C_6$-alkyl which is substituted by an amido group $CONH_2$, or is di-$C_1$-$C_4$-alkylamino or aminophenyl where the aromatic ring may carry from one to three of the groups stated for $R^1$,
$R^{10}$ is $C_1$-$C_4$-alkyl or $C_3$-$C_8$-cycloalkyl or is phenyl which may carry from one to three of the groups stated for $R^1$, or is hydroxyl, $C_1$-$C_8$-alkoxy or a group -O-CO-$R^{12}$ or -O-CS-$R^{12}$ where $R^{12}$ is $C_1$-$C_{20}$-

alkyl, or $R^{10}$ is $C_1$-$C_6$-haloalkyl, $C_3$-$C_8$-cycloalkyl, phenyl-$C_1$-$C_3$-alkyl or phenyl where the phenyl rings may, if desired, furthermore carry from one to three of the radicals stated for $R^1$,

or, if $R^6$ and $R^7$ are each alkyl or $R^6$ is alkyl and $R^7$ is a group -CO-H, -CO-($C_1$-$C_4$-alkyl), -CH = N-$R^{10}$ or -C($C_1$-$C_4$-alkyl) = N-$R^{10}$,

$R^6$ and $R^7$ together form a 5-membered to 7-membered ring which is substituted by from one to four $C_1$-$C_4$-alkyl groups or is fused with a phenyl ring which may carry from one to three of the groups stated for $R^1$,

and the agriculturally useful salts of the compounds I', where these compounds contain a basic nitrogen substituent or an acidic hydroxyl substituent,

and at least one herbicidal active ingredient from the group consisting of

A) the 2-(4-hetaryloxy)- or 2-(4-aryloxy)-phenoxyacetic acid derivatives of the formula V

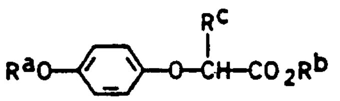

V

where

$R^a$ is a phenyl ring, a pyridyl ring, benzoxazyl, benzothiazyl or benzopyrazinyl, where these aromatic ring systems may carry up to two of the following radicals: halogen, nitro, $C_1$-$C_4$-alkyl, $C_1$-$C_4$-haloalkyl and/or $C_1$-$C_4$-haloalkoxy,

$R^b$ is hydrogen or $C_1$-$C_4$-alkyl and

$R^c$ is hydrogen or methyl, and

B) a cyclohexenone oxime ether of the formula VI

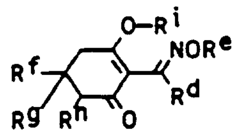

VI

where $R^d$ is $C_1$-$C_4$-alkyl,

$R^e$ is $C_1$-$C_4$-alkyl, $C_3$- or $C_4$-alkenyl, $C_3$- or $C_4$-alkynyl or partially or completely halogenated $C_3$- or $C_4$-alkenyl,

a $C_1$-$C_4$-alkylene or $C_2$-$C_4$-alkenyl chain, both of which may furthermore carry from one to 3 $C_1$-$C_3$-alkyl radicals and/or halogen atoms, or a 3-membered to 6-membered alkylene chain or a 4-membered to 6-membered alkenylene chain which, if desired, is substituted by $C_1$-$C_3$-alkyl and each of which contains, as a chain member, an oxygen or sulfur atom which is not directly adjacent to the oxime ether moiety, all abovementioned chains carrying a terminal phenyl ring which in turn may be substituted by from one to three radicals selected from the group consisting of a benzyloxycarbonyl or phenyl radical and in each case from one to three of the following radicals: nitro, cyano, halogen, $C_1$-$C_4$-alkyl, $C_1$-$C_4$-alkoxy, $C_1$-$C_4$-alkylthio, partially or completely halogenated $C_1$-$C_4$-alkyl, partially or completely halogenated $C_1$-$C_4$-alkoxy, carboxyl and $C_1$-$C_4$-alkoxycarbonyl, and it being possible for the phenyl ring additionally to carry a number of halogen atoms such that the total number of radicals is 4 or 5, or

thienylmethyl which may furthermore carry a halogen atom,

$R^f$ is $C_1$-$C_4$-alkyl which may be monosubstituted by $C_1$-$C_4$-alkylthio or $C_1$-$C_4$-alkoxy,

a 5-membered or 6-membered saturated or monounsaturated ring system which, in addition to carbon members, may contain an oxygen or a sulfur atom or a sulfoxyl or sulfonyl group, where this ring may carry up to three of the following radicals: hydroxyl, halogen, $C_1$-$C_4$-alkyl, partially or completely halogenated $C_1$-$C_4$-alkyl, $C_1$-$C_4$-alkoxy and/or $C_1$-$C_4$-alkylthio,

a 10-membered saturated or monounsaturated heterocyclic structure which contains two oxygen atoms or sulfur atoms and may be substituted by up to three $C_1$-$C_4$-alkyl groups and/or methoxy groups,

phenyl, pyridyl, thiazolyl, pyrazolyl, pyrrolyl or isoxazolyl, where these groups may each carry from one to three radicals selected from the group consisting of $C_1$-$C_4$-alkyl, partially or completely halogenated $C_1$-$C_4$-alkyl, $C_1$-$C_4$-alkoxy, $C_1$-$C_4$-alkylthio, $C_3$-$C_6$-alkenyloxy, $C_3$-$C_6$-alkynyloxy, $C_1$-$C_4$-alkoxy-$C_1$-$C_3$-alkyl, $C_1$-$C_4$-alkylthio-$C_1$-$C_3$-alkyl, $C_1$-$C_4$-dialkoxy-$C_1$-$C_3$-alkyl, formyl, halogen and ben-

zoylamino,
$R^g$ is hydrogen or hydroxyl or, if $R^f$ is $C_1$-$C_6$-alkyl, $R^g$ is $C_1$-$C_6$-alkyl,
$R^h$ is hydrogen, cyano, halogen, $C_1$-$C_4$-alkoxycarbonyl or $C_1$-$C_4$-alkylketoxime and
$R^i$ is hydrogen.

**5.** A herbicide as claimed in claim 4, wherein the weight ratio of pyrido-fused 4-oxo-4H-benzopyran I' to herbicidal active ingredient A) or B) is from 10 : 1 to 0.01 : 1.

**6.** A method for selectively controlling undesirable plant growth, wherein a pyrido-fused 4-oxo-4H-benzopyran of the formula I' as claimed in claim 4 and
A) a 2-(4-hetaryloxy)- or 2-(4-aryloxy)-phenoxyacetic acid derivative of the formula V or
B) a cyclohexenone derivative of the formula VI
as claimed in claim 4 are applied, simultaneously or in succession, before, during or after sowing of the crops or before or during emergence of the crops.

**7.** A method for preventing damage to crops by
A) herbicidal 2-(4-hetaryloxy)- or 2-(4-aryloxy)-phenoxyacetic acid derivatives of the formula V or
B) cyclohexenone derivatives of the formula VI as claimed in claim 4,
wherein the seed of the crops is treated with an antagonistic amount of a pyrido-fused 4-oxo-4H-benzopyran of the formula I' as claimed in claim 4.

**8.** A method for selectively controlling undesirable plant growth, wherein the leaves of the crops and of the undesirable plants are treated by the postemergence method, simultaneously or in succession, with a pyrido-fused 4-oxo-4H-benzopyran of the formula I' as claimed in claim 4 and with a 2-(4-hetaryloxy)- or 2-(4-aryloxy)-phenoxyacetic acid derivative of the formula V or a cyclohexenone derivative of the formula VI as claimed in claim 4.

**9.** A method as claimed in claim 6, wherein the crops are barley, wheat, corn, millet or rice.

**10.** A method as claimed in claim 7, wherein the crops are barley, wheat, corn, millet or rice.

**11.** A method as claimed in claim 8, wherein the crops are barley, wheat, corn, millet or rice.

**Revendications**

**1.** 4-oxo-4H-benzopyrannes pyrido-condensés de formule générale

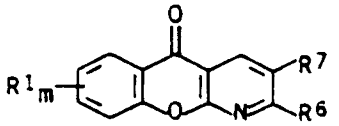

I,

dans lesquels les substituants et l'indice ont la signification suivante:

m — est 0, 1 ou 2, tandis que les restes $R^1$ peuvent être différents lorsque m vaut 2;

$R^1$ — représente l'hydrogène ou un radical hydroxy, halogéno, nitro, alkyle en $C_1$-$C_6$, alcoxy en $C_1$-$C_6$, halogénoalkyle en $C_1$-$C_6$, halogénoalcoxy en $C_1$-$C_4$, alkyl($C_1$-$C_4$)thio ou un groupe $NR^2R^3$, tandis que

$R^2$ — désigne l'hydrogène ou un radical alkyle en $C_1$-$C_4$ et

$R^3$ — représente l'hydrogène ou un radical alkyle en $C_1$-$C_4$, un groupe -CO-$R^4$, -CS-$R^4$ ou -$SO_2$-$R^5$;

$R^4$ — représente un radical alkyle en $C_1$-$C_{20}$; halogénoalkyle en $C_1$-$C_6$; cycloalkyle en $C_3$-$C_8$; aralkyle en $C_1$-$C_3$; amino; alkyl($C_1$-$C_4$)amino; dialkyl($C_1$-$C_4$)-amino; un groupe phényle ou phénylamino, tandis que le cycle aromatique peut porter de un à trois des groupes indiqués pour $R^1$;

$R^5$ — désigne un radical alkyle en $C_1$-$C_4$; phényle, qui peut porter de un à trois des groupes indiqués pour $R^1$;

$R^6$ — représente un radical hydroxy; alcoxy en $C_1$-$C_6$; alkyle en $C_1$-$C_6$; un groupe -NH-$R^8$ ou

-N(alkyl en $C_1$-$C_4$)-$R^8$, tandis que $R^8$ a l'une des significations indiquées pour $R^3$; un reste phényle, qui peut porter de un à trois des groupes indiqués pour $R^1$, un groupe 4-méthyl-5-oxo-imidazoline-2-yle substitué par un groupe alkyle en $C_1$-$C_4$ ou un groupe -CO-$R^9$ ou -CS-$R^9$;

$R^7$ représente un radical cyano, alkyle en $C_1$-$C_6$; un reste phényle qui peut porter de un à trois des groupes mentionnés pour $R^1$; un groupe -CH=N-$R^{10}$, -C(alkyl en $C_1$-$C_4$)=N-$R^{10}$, -CO-$R^{11}$ ou -CS-$R^{11}$;

$R^9$, $R^{11}$ désignent un radical alkyle en $C_1$-$C_6$; alcoxy en $C_1$-$C_6$; hydroxy; amino; alkyl($C_1$-$C_4$)-amino; alkyle en $C_2$-$C_6$ ramifié, substitué par un groupe amido $CONH_2$; dialkyl($C_1$-$C_4$)-amino; un reste aminophényle, tandis que le cycle aromatique peut porter de un à trois des groupes indiqués pour $R^1$;

$R^{10}$ représente un radical alkyle en $C_1$-$C_4$; cycloalkyle en $C_3$-$C_8$; phényle, qui peut porter de un à trois des groupes indiqués pour $R^1$; hydroxy, alcoxy en $C_1$-$C_8$; un groupe -O-CO-$R^{12}$ ou -O-CS-$R^{12}$, tandis que $R^{12}$ désigne un radical alkyle en $C_1$-$C_{20}$; halogénoalkyle en $C_1$-$C_6$; cycloalkyle en $C_3$-$C_8$; phényl-(alkyle en $C_1$-$C_3$) ou phényle, tandis que les cycles phényle peuvent, si on le souhaite, porter encore de un à trois des restes susdits pour $R^1$;

ou, dans le cas où $R^6$ et $R^7$ désignent un groupe alkyle ou $R^6$ un groupe alkyle et $R^7$ un groupe -CO-H, -CO-(alkyle en $C_1$-$C_4$), -CH=N-$R^{10}$ ou -C(alkyl en $C_1$-$C_4$)=N-$R^{10}$:

$R^6$ et $R^7$ représentent ensemble un cycle à 5-7 chaînons, qui est substitué par un à quatre radicaux alkyle en $C_1$-$C_4$ ou est condensé avec un cycle qui peut porter de un à trois des groupes indiqués plus haut;

ainsi que les sels utilisables en agriculture des composés I, dans la mesure où ces composés contiennent un substituant azoté basique ou un substituant hydroxylé acide,

à l'exception des composés: 2,3-diphényl-[1]benzopyranne-[2,3-b]pyridine-5-one, 2,3-diméthyl-[1]-benzopyranne-[2,3-b]pyridine-5-one, 3-éthoxycarbonyl-2-méthyl-[1]benzopyranne-[2,3-b]pyridine-5-one, 3-éthoxycarbonyl-7-éthyl-2-méthoxy-[1]benzopyranne-[2,3-b]pyridine-5-one, 3-éthoxycarbonyl-7-éthyl-2-méthyl-[1]benzopyranne-[2,3-b]pyridine-5-one, 2-hydroxy-3-méthyl-[1]benzopyranne-[2,3-b]pyridine-5-one, 2,3-diéthoxycarbonyl-7-éthyl-[1]benzopyranne-[2,3-b]pyridine-5-one, ainsi que ceux des composés I dans lesquels soit $R^6$ désigne un radical alkyle en $C_1$-$C_6$, phényle, carboxyle, hydroxyle, alcoxy en $C_1$-$C_6$, amino ou alkyl($C_1$-$C_4$)amino et $R^7$ désigne un radical cyano, carboxyle ou éthoxycarbonyle, soit $R^6$ représente un radical hydroxy et $R^7$ représente un radical éthoxycarbonyle, soit $R^6$ désigne un radical méthyle et $R^7$ désigne un radical méthylcarbonyle.

**2.** Procédé pour la préparation du composé I selon la revendication 1, caractérisé en ce qu'on condense l'o-aminoaldéhyde II

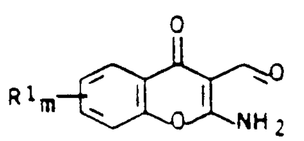

II

en présence d'une base dans un solvant inerte avec une cétone de formule III

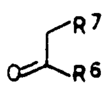

III

**3.** Procédé pour la préparation du composé I selon la revendication 1, dans lequel $R^6$ représente un radical hydroxy ou amino, caractérisé en ce qu'on cyclise le 2-amino-4-oxo-4H-chromène-3-carbaldéhyde II d'une manière connue en soi dans un solvant inerte en présence d'une base avec un dérivé d'acide acétique de formule IVa ou IVb, tandis que R dans la formule IVa désigne un groupe alkyle en $C_1$-$C_4$

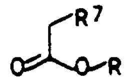

IVa

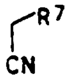

IVb

4. Agent herbicide, contenant au moins un 4-oxo-4H-benzopyranne pyrido-condensé de formule générale I'

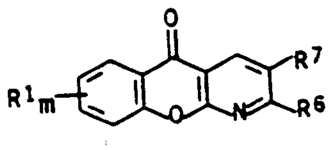

I'

où

- m est 0, 1 ou 2, tandis que les restes $R^1$ peuvent être différents lorsque m vaut 2;
- $R^1$ représente l'hydrogène ou un radical hydroxy, halogéno, nitro, alkyle en $C_1$-$C_6$, alcoxy en $C_1$-$C_6$, halogéno- alkyle en $C_1$-$C_6$, halogénoalcoxy en $C_1$-$C_4$, alkyl($C_1$-$C_4$)thio ou un groupe $NR^2R^3$, tandis que
- $R^2$ désigne l'hydrogène ou un radical alkyle en $C_1$-$C_4$ et
- $R^3$ représente l'hydrogène ou un radical alkyle en $C_1$-$C_4$, un groupe -CO-$R^4$, -CS-$R^4$ ou -$SO_2$-$R^5$;
- $R^4$ représente un radical alkyle en $C_1$-$C_{20}$; halogénoalkyle en $C_1$-$C_6$; cycloalkyle en $C_3$-$C_8$; aralkyle en $C_1$-$C_3$; amino; alkyl($C_1$-$C_4$)amino; dialkyl($C_1$-$C_4$)-amino; un groupe phényle ou phénylamino, tandis que le cycle aromatique peut porter de un à trois des groupes indiqués pour $R^1$;
- $R^5$ désigne un radical alkyle en $C_1$-$C_4$; phényle, qui peut porter de un à trois des groupes indiqués pour $R^1$;
- $R^6$ représente un radical hydroxy; alcoxy en $C_1$-$C_6$; alkyle en $C_1$-$C_6$; un groupe -NH-$R^8$ ou -N(alkyl en $C_1$-$C_4$)-$R^8$, tandis que $R^8$ a l'une des significations indiquées pour $R^3$; un reste phényle, qui peut porter de un à trois des groupes indiqués pour $R^1$, un groupe 4-méthyl-5-oxo-imidazoline-2-yle substitué par un groupe alkyle en $C_1$-$C_4$ ou un groupe -CO-$R^9$ ou -CS-$R^9$;
- $R^7$ représente un radical cyano, alkyle en $C_1$-$C_6$; un reste phényle qui peut porter de un à trois des groupes mentionnés pour $R^1$; un groupe -CH=N-$R^{10}$, -C(alkyl en $C_1$-$C_4$)=N-$R^{10}$, -CO-$R^{11}$ ou -CS-$R^{11}$;
- $R^9$, $R^{11}$ désignent un radical alkyle en $C_1$-$C_6$; alcoxy en $C_1$-$C_6$; hydroxy; amino; alkyl($C_1$-$C_4$)-amino; alkyle en $C_2$-$C_6$ ramifié, substitué par un groupe amido $CONH_2$; dialkyl($C_1$-$C_4$)-amino; un reste aminophényle, tandis que le cycle aromatique peut porter de un à trois des groupes indiqués pour $R^1$;
- $R^{10}$ représente un radical alkyle en $C_1$-$C_4$; cycloalkyle en $C_3$-$C_8$; phényle, qui peut porter de un à trois des groupes indiqués pour $R^1$; hydroxy, alcoxy en $C_1$-$C_8$; un groupe -O-CO-$R^{12}$ ou -O-CS-$R^{12}$, tandis que $R^{12}$ désigne un radical alkyle en $C_1$-$C_{20}$; halogénoalkyle en $C_1$-$C_6$; cycloalkyle en $C_3$-$C_8$; phényl-(alkyle en $C_1$-$C_3$) ou phényle, tandis que les cycles phényle peuvent, si on le souhaite, porter encore de un à trois des restes susdits pour $R^1$;

ou, dans le cas où $R^6$ et $R^7$ désignent un groupe alkyle ou $R^6$ un groupe alkyle et $R^7$ un groupe -CO-H, -CO-(alkyle en $C_1$-$C_4$), -CH=N-$R^{10}$ ou -C(alkyl en $C_1$-$C_4$)=N-$R^{10}$:

- $R^6$ et $R^7$ représentent ensemble un cycle à 5-7 chaînons, qui est substitué par un à quatre radicaux alkyle en $C_1$-$C_4$ ou est condensé avec un cycle qui peut porter de un à trois des groupes indiquée plus haut;

ainsi que les sels utilisables en agriculture des composés I, dans la mesure où ces composés contiennent un substituant azoté basique ou un substituant hydroxylé acide,
et au moins un agent à activité herbicide du groupe

A) des dérivés d'acide 2-(4-hétéroaryloxy)- ou 2-(4-aryloxy)-phénoxyacétique de formule V

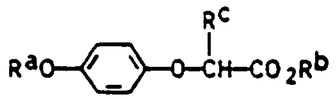

V

où

$R^a$ représente un cycle phényle, un cycle pyridyle, un reste benzoxazyle, un reste benzothioazyle ou un reste benzopyrazinyle, tandis que ces systèmes cycliques aromatiques peuvent porter jusqu'à deux des restes suivants: halogéno, nitro, alkyle en $C_1$-$C_4$, halogénoalkyle en $C_1$-$C_4$ et/ou halogénoalcoxy en $C_1$-$C_4$,

$R^b$ représente l'hydrogène ou un groupe alkyle en $C_1$-$C_4$ et

$R^c$ représente l'hydrogène ou un groupe méthyle et

B) des éthers de cyclohexénonoxime de formule VI

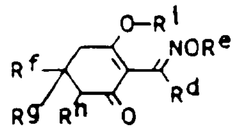

VI

dans laquelle les substituants ont les significations suivantes:

$R^d$ représente un groupe alkyle en $C_1$-$C_4$;

$R^e$ désigne un groupe alkyle en $C_1$-$C_4$, un groupe alcényle en $C_3$-$C_4$, un groupe alcynyle en $C_3$-$C_4$ ou un groupe alcényle en $C_3$-$C_4$ partiellement ou totalement halogéné;

une chaîne alkylène en $C_1$-$C_4$ ou alcényle en $C_2$-$C_4$, qui peuvent toutes deux porter en outre de un à trois restes alkyle en $C_1$-$C_3$ et/ou atomes d'halogène, ou une chaîne alkylène à 3 à 6 chaînons ou alcénylène à 4 à 6 chaînons éventuellement substituée par un radical alkyle en $C_1$-$C_3$, et qui contient en tant que maillon de la chaîne suivant les cas un atome d'oxygène ou de soufre non directement voisin de la partie éther d'oxime, tandis que toutes les chaînes mentionnées plus haut portent le cycle phényle en position terminale, ce cycle pouvant quant à lui être substitué par un à trois restes,

choisis dans un groupe comprenant un reste benzyloxy-carbonyle ou phényle, et suivant les cas de un à trois des restes suivants: nitro, cyano, halogéno, alkyle en $C_1$-$C_4$, alcoxy en $C_1$-$C_4$, alkyl($C_1$-$C_4$)thio, alkyle en $C_1$-$C_4$ partiellement ou totalement halogéné, alcoxy en $C_1$-$C_4$ partiellement ou totalement halogéné, carboxyle et alcoxy($C_1$-$C_4$)carbonyle, et où le cycle phényle peut encore porter en outre assez d'atomes d'halogène pour que le nombre total des restes soit de 4 ou 5;

un groupe thiénylméthyle, qui peut encore porter un atome d'halogène;

$R^f$ désigne un groupe alkyle en $C_1$-$C_4$, qui peut être substitué une fois par un groupe alkyl-($C_1$-$C_4$)thio ou alcoxy en $C_1$-$C_4$;

un système cyclique à 5 ou 6 chaînons, saturé ou insaturé une seule fois, qui peut contenir, outre les chaînons carbonés, un atome d'oxygène, un atome de soufre, un groupe sulfoxyde ou un groupe sulfone, tandis que ce cycle peut porter jusqu'à trois des restes suivants: hydroxy, halogéno, alkyle en $C_1$-$C_4$, alkyle en $C_1$-$C_4$ partiellement ou totalement halogéné, alcoxy en $C_1$-$C_4$ et/ou alkyl($C_1$-$C_4$)thio;

un hétérocycle à 10 chaînons, saturé ou insaturé une seule fois, qui contient deux atomes d'oxygène ou de soufre et peut être substitué par jusqu'à trois groupes alkyle en $C_1$-$C_4$ et/ou groupes méthoxy;

le groupe phényle, le groupe pyridyle, le groupe thiazolyle, le groupe pyrazolyle, le groupe pyrrolyle ou le groupe isoxazolyle, tandis que ces groupes peuvent porter suivant les cas de un à trois restes, choisis dans le groupe consistant en les radicaux alkyle en $C_1$-$C_4$, alkyle en $C_1$-$C_4$ partiellement ou totalement halogéné, alcoxy en $C_1$-$C_4$, alkyl($C_1$-$C_4$)thio, alcényloxy en $C_3$-$C_6$, alcynyloxy en $C_3$-$C_6$, alcoxy($C_1$-$C_4$)alkyle en $C_1$-$C_3$, alkyl($C_1$-$C_4$)thio-alkyle en $C_1$-$C_3$, dialkyl($C_1$-$C_4$)oxy-alkyle en $C_1$-$C_3$, formyle, halogéno et benzoylamino;

$R^g$ représente l'hydrogène ou un radical hydroxy ou, lorsque $R^f$ désigne un groupe alkyle en $C_1$-$C_6$, un groupe alkyle en $C_1$-$C_6$;

$R^h$ représente l'hydrogène ou un groupe cyano, un atome d'halogène, un groupe alcoxy($C_1$-$C_4$)carbonyle ou un groupe alkyl($C_1$-$C_4$)cétoxime et

$R^i$ représente l'hydrogène.

**5.** Agent herbicide selon la revendication 4, caractérisé en ce que le rapport en poids du 4-oxo-4H-benzopyranne pyrido-condensé I' à l'agent herbicide A) ou B) est de 10:1 à 0,01:1 parties en poids.

**6.** Procédé pour la lutte sélective contre la croissance des plantes parasites, caractérisé en ce qu'on répand, simultanément ou l'un après l'autre,avant, pendant ou après le semis des plantes cultivées, avant ou pendant la pousse des plantes cultivées, un 4-oxo-4H-benzopyranne pyrido-condensé de formule I' selon la revendication 4 et

A) un dérivé d'acide 2-(4-hétéroaryloxy)- ou 2-(4-aryloxy)-phénoxyacétique de formule V ou

B) un dérivé de cyclohexénone de formule VI selon la revendication 4.

**7.** Procédé pour empêcher les dommages aux plantes cultivées par

A) des dérivés d'acide 2-(4-hétéroaryloxy)- ou 2-(4-aryloxy)-phénoxyacétique de formule V herbicides de formule V ou

B) des dérivés de cyclohexénone de formule VI selon la revendication 4, caractérisé en ce qu'on traite les semences des plantes cultivées avec une quantité à activité antagoniste d'un 4-oxo-4H-benzopyranne pyrido-condensé de formule I' selon la revendication 4.

**8.** Procédé pour la lutte sélective contre la croissance de plantes parasites, caractérisé en ce qu'on traite les feuilles des plantes cultivées et des plantes parasites en post-levée avec un 4-oxo-4H-benzopyranne pyrido-condensé de formule I' selon la revendication 4 et avec un dérivé d'acide 2-(4-hétéroaryloxy)- ou 2-(4-aryloxy)-phénoxyacétique de formule V ou un dérivé de cyclohexénone de formule VI selon la revendication 4, simultanément ou l'un après l'autre.

**9.** Procédé selon la revendication 6, caractérisé en ce que les plantes cultivées sont l'orge, le blé, le maïs, le sorgho de culture et le riz.

**10.** Procédé selon la revendication 7, caractérisé en ce que les plantes cultivées sont l'orge, le blé, le maïs, le sorgho de culture et le riz.

**11.** Procédé selon la revendication 8, caractérisé en ce que les plantes cultivées sont l'orge, le blé, le maïs, le sorgho de culture et le riz.